(19) Europäisches Patentamt / European Patent Office / Office européen des brevets

(11) EP 4 272 741 A1

(12) # EUROPEAN PATENT APPLICATION
published in accordance with Art. 153(4) EPC

(43) Date of publication:
**08.11.2023 Bulletin 2023/45**

(21) Application number: **21914663.6**

(22) Date of filing: **30.12.2021**

(51) International Patent Classification (IPC):
***A61K 31/5377*** *(2006.01)* ***A61K 31/52*** *(2006.01)*
***A61P 35/00*** *(2006.01)*

(52) Cooperative Patent Classification (CPC):
**A61K 31/52; A61K 31/5377; A61P 35/00**

(86) International application number:
**PCT/CN2021/143219**

(87) International publication number:
**WO 2022/143933 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.12.2020 CN 202011642452**
**22.07.2021 CN 202110829596**

(71) Applicant: **CSPC Zhongqi Pharmaceutical Technology (Shijiazhuang) Co., Ltd.**
**Shijiazhuang, Hebei 050035 (CN)**

(72) Inventors:
- **LIU, Cuiyan**
**Shijiazhuang, Hebei 050035 (CN)**
- **BAI, Jing**
**Shijiazhuang, Hebei 050035 (CN)**
- **WEN, Shilong**
**Shijiazhuang, Hebei 050035 (CN)**
- **LIU, Na**
**Shijiazhuang, Hebei 050035 (CN)**
- **GU, Cong**
**Shijiazhuang, Hebei 050035 (CN)**
- **JI, Dehua**
**Shijiazhuang, Hebei 050035 (CN)**

(74) Representative: **Grünecker Patent- und Rechtsanwälte**
**PartG mbB**
**Leopoldstraße 4**
**80802 München (DE)**

(54) **PHARMACEUTICAL COMPOSITION OF MULTI-TARGET PROTEIN KINASE INHIBITORS, AND USE THEREOF**

(57) A pharmaceutical composition containing multi-target protein kinase inhibitor compounds, and the use thereof. The pharmaceutical composition contains compounds shown in formula II and formula A or formula B as active ingredients, and an excipient. The pharmaceutical composition has the characteristics of a simple preparation method, a smooth preparation process and suitability for industrial production. Moreover, an oral preparation prepared from the pharmaceutical composition, especially an oral solid preparation, has advantageous preparation properties such as dissolution rate and content uniformity, and excellent stability; and same is suitable for use and storage as a medicine.

EP 4 272 741 A1

## Description

## CROSS-REFERENCE TO RELATED APPLICATION

The present disclosure claims the priorities of Chinese Patent Application No. 202011642452.1 entitled "PHARMACEUTICAL COMPOSITION OF MULTI-TARGET PROTEIN KINASE INHIBITOR, AND USE THEREOF" and filed in China on December 31, 2020, and Chinese Patent Application No. 202110829596.6 entitled "PHARMACEUTICAL COMPOSITION OF MULTI-TARGET PROTEIN KINASE INHIBITOR, AND USE THEREOF" and filed in China on July 22, 2021, and the disclosures of these two patent applications are incorporated herein by reference in their entirety.

## TECHNICAL FIELD

The present application belongs to the technical field of pharmaceutical formulations, in particular, relates to a pharmaceutical composition of a multi-target protein kinase inhibitor and use thereof.

## BACKGROUND

Malignant tumor is one of the most severe diseases threatening the human’s life and health, and has become the second leading cause of human death, right behind the cardiovascular disease. Chemotherapy is the most important therapeutic means for malignant tumors besides surgery and radiotherapy. Traditional chemotherapeutic drugs act mainly on DNA, RNA, microtubule protein and the like, which are the common components associated with the survival and death of all cells, and therefore have low selectivity and high toxicity. The targeted therapeutic drugs act on the key molecules and the signaling pathways thereof in tumor cells, which regulate the cell growth and proliferation and are quite different from normal cells. The targeted drugs have the advantages such as a high selectivity on tumor cells and low toxicity to normal tissues, and therefore have become a hotspot in the study of anti-tumor drugs.

Among many of the molecules regulating the signaling pathways of cells, the family of protein kinases is the most important signaling molecule. Studies have shown that the occurrence and development of many tumors are relevant to the gene abnormality or excess activation of protein kinases. Therefore, protein kinases have become the most important anti-tumor therapeutic targets. Tyrosine and serine/threonine protein kinases such as EGFR (Epidermal Growth Factor Receptor), VEGFR (Vascular Endothelial Growth Factor Receptor), PDGFR (Platelet Derived Growth Factor Receptor), c-Kit, c-SRC, MET, BTK, ALK, Abl, and FLT3 are most important among the family members of the protein kinases, and have been listed as oncogenes or oncoproteins.

At present, a plurality of small molecule inhibitors targeting these tyrosine and serine/threonine protein kinases have been applied to clinical tumor treatment. Typical examples include: two EGFR inhibitors, i.e., Gefitinib and Erlotinib, mainly useful for the treatment of non-small cell lung cancer; two angiogenesis inhibitors (their main targets are VEGFR, PDGFR and the like), i.e., Sunitinib and Sorafenib, useful for the anti-angiogenesis treatment in solid tumors; and Bcr-Abl kinase inhibitor Imatinib, mainly useful for the treatment of chronic myeloid leukemia (CML) with positive Philadelphia chromosome.

With more and more targeted anti-tumor drugs applied in clinical practice, some issues have gradually attracted people’s attention. On the one hand, an anti-tumor drug targeting a single or a few kinase oncogenes or oncoproteins faces the disadvantages such as low efficiency and susceptibility to drug resistance. Typical examples are EGFR kinase inhibitors Gefitinib and Erlotinib. These two drugs are effective in only 10% to 20% of patients with non-small cell lung cancer. Studies on their mechanism of action have revealed that Gefitinib and Erlotinib belong to selective EGFR kinase inhibitors, and in principle, patients with EGFR exon 19 deletion mutation and patients with exon 21 L858R point mutation (the EGFR exon 19 deletion mutation and exon 21 L858R point mutation are collectively referred to as EGFR-sensitive mutations) are most sensitive to them. Even for these patients with EGFR-sensitive mutations, most of them have developed the drug resistance after 6 to 9 months of treatment with Gefitinib and Erlotinib. Studies have shown that there are various reasons for the occurrence of drug resistance, which mainly include (1) the occurrence of a secondary mutation based on the EGFR-sensitive mutation, i.e., T790M mutation in EGFR; and (2) the amplification of MET gene (MET is a tyrosine kinase). On the other hand, currently available protein kinase inhibitors still focus on a small number of kinase targets such as EGFR, FGFR, VEGFR, JAK, PI3K, and CDK, and the exploration on other kinase members is not adequate enough, and there is still much room for exploration in terms of the skeleton structures and mechanisms of action of inhibitors. In addition, although targeted drugs are featured in low toxicity and strong specificity compared with conventional anti-tumor drugs, different degrees of toxic and side effects have been still observed in the clinical trials of small molecule kinase inhibitors. For example, it is reported that common adverse reactions of Midostaurin in the treatment course of acute myeloid leukemia (AML) mainly include leukopenia, nausea and vomiting, mucositis, headache, skin petechiae, musculoskeletal pain, epistaxis, hyperglycemia, upper respiratory tract infection, and the like.

Developing novel anti-tumor drugs to address the problems of low effective rate, drug resistance and safety of

small molecule kinase inhibitors is the focus of current efforts in research and development of targeted anti-tumor drugs. Currently, effective solutions are being explored, in which the most promising solution mainly includes: (1) a multi-kinase inhibitor that simultaneously targets multiple kinases associated with tumor genesis and progression (for example, EGFR is overexpressed or abnormally and excessively activated in a variety of human tumor tissues; if a certain small molecule kinase inhibitor could not only inhibit the activity of EGFR, but also inhibit neovascularization or the activities of oncogenes or oncoproteins of other key kinases regulating cell growth and proliferation, e.g., kinases such as c-Kit, c-SRC, MET, BTK, ALK, Abl and FLT3, this inhibitor would be capable of improving the effective rate of tumor treatment and reducing the incidence of drug resistance); and (2) directly targeting the kinase that has developed a drug-resistant mutation (for example, as stated hereinbefore, after EGFR inhibitors Gefitinib and Erlotinib are administered, EGFR itself is prone to experiencing a secondary mutation on the basis of the original mutation (*i.e.,* the EGFR-sensitive mutation), namely, the T790M mutation in EGFR; the occurrence of this mutation is one of the main reasons causing EGFR inhibitors Gefitinib and Erlotinib to be ineffective; therefore, the research and development of an EGFR kinase inhibitor that directly targets the drug-resistant mutation (*i.e.,* the T790M mutation) is a direct approach of overcoming such kind of drug resistance of tumors).

Patent No. WO2011147066A1 discloses Compound I and a preparation method and use thereof. Compound I is a novel multi-target protein kinase inhibitor acting mainly on targets including FLT3, EGFR, Abl, Fyn, Hck, Lck, Lyn, Ret, Yes, etc., and is useful in the treatment of tumor diseases:

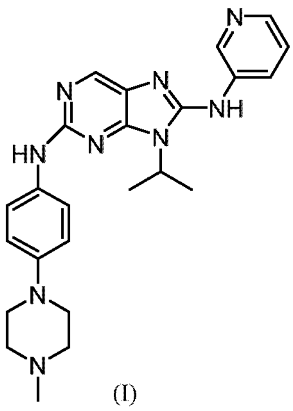

(I)

**SUMMARY**

Technical Problem

On the basis of Compound I, hydrochloride of Compound I (*e.g.*, a compound of Formula II or Formula A) with better druggability than that of Compound I has been further discovered in the present application. The hydrochloride is not only superior to Compound I in terms of the properties such as solubility and stability, but also is generally more suitable for drug preparation than other salt forms of Compound I in consideration of multiple aspects such as solubility, hygroscopicity, stability and the like. The corresponding studies and screening process have already been documented in the Patent Application No. PCT/CN2021/073285, and the content of this application is incorporated herein by reference in its entirety. In view of the promising application prospect of Compound I in the treatment of tumor diseases, seeking for a pharmaceutical composition with excellent formulation properties that comprises Compound I or a pharmaceutically acceptable salt thereof (*e.g.*, a compound of Formula II or Formula A) becomes a pressing problem to be solved by a person skilled in the art.

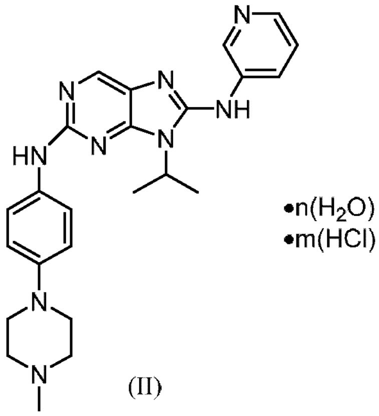

(II)

,

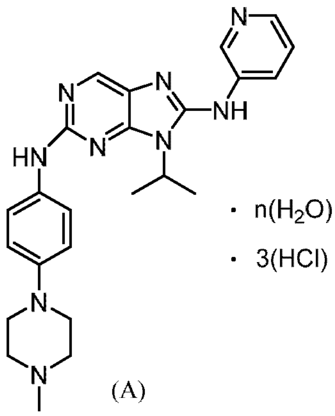

(A)

.

One of the technical problems to be solved by the present application is to provide a pharmaceutical composition comprising a compound represented by Formula II, in particular a compound represented by Formula A, as an active ingredient that satisfies the requirements for oral administration.

Solutions to Problem

In the course of ongoing researches to explore a desirable pharmaceutical composition and/or oral formulation comprising a compound represented by Formula II, in particular a compound represented by Formula A, the present inventors have conducted meticulous screening experiments on the ingredients of the pharmaceutical composition and have found that specific formulation compositions of the pharmaceutical composition are capable of solving the above-mentioned technical problem, thereby completing the present application.

In one aspect, the present application provides a pharmaceutical composition, comprising a compound represented by Formula II as an active ingredient and an excipient:

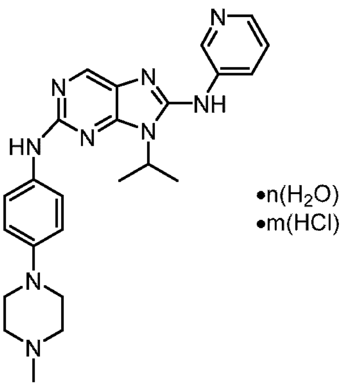

(II),

wherein m is 1 to 5, preferably 1 to 3; and n is 0 to 10, preferably 0 to 5, further preferably 3 to 5, further preferably 3, 3.5, 4, 4.5 or 5, still further preferably 5.

In the second aspect, the present application provides a pharmaceutical composition, comprising a compound represented by Formula A as an active ingredient and an excipient:

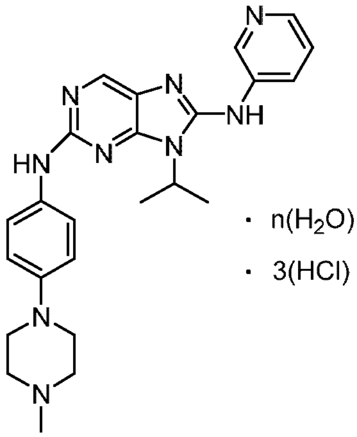

(A),

wherein n is 0 to 10, preferably 0 to 5, further preferably 3 to 5, further preferably 3, 3.5, 4, 4.5 or 5, still further preferably 5.

In the third aspect, the present application provides a pharmaceutical composition, comprising a compound represented by Formula B as an active ingredient and an excipient:

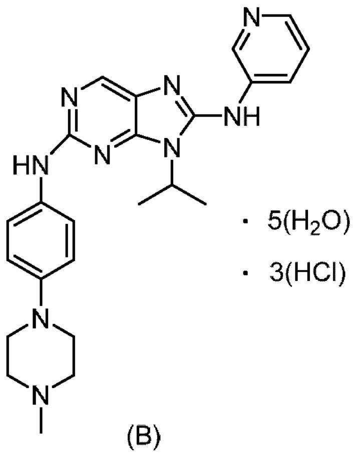

(B)

.

In some embodiments of the present application, the compound represented by Formula B has characteristic peaks at 2θ angles of 8.5±0.2°, 11.8±0.2°, 19.6±0.2°, 25.2±0.2°, and 27.2±0.2° in an X-ray powder diffraction pattern obtained by using Cu-Kα radiation; preferably, the compound represented by Formula B has characteristic peaks at 2θ angles of 8.5±0.2°, 11.8±0.2°, 12.6±0.2°, 19.6±0.2°, 20.0±0.2°, 23.7±0.2°, 25.2±0.2°, and 27.2±0.2° in an X-ray powder diffraction pattern obtained by using Cu-Kα radiation; further preferably, the compound represented by Formula B has characteristic peaks at 2θ angles of 7.3±0.2°, 8.5±0.2°, 9.0±0.2°, 11.8±0.2°, 12.6±0.2°, 14.3±0.2°, 18.1±0.2°, 19.6±0.2°, 20.0±0.2°, 21.1±0.2°, 21.9±0.2°, 23.7±0.2°, 25.2±0.2°, 26.1±0.2°, and 27.2±0.2° or has characteristic peaks at 2θ angles of 7.3±0.2°, 8.5±0.2°, 9.1±0.2°, 11.8±0.2°, 12.6±0.2°, 14.3±0.2°, 18.1±0.2°, 19.6±0.2°, 20.0±0.2°, 21.1±0.2°, 21.9±0.2°, 23.7±0.2°, 25.2±0.2°, 26.1±0.2°, and 27.2±0.2° in an X-ray powder diffraction pattern obtained by using Cu-Kα radiation; still further preferably, the compound represented by Formula B has an X-ray powder diffraction pattern obtained by using Cu-Kα radiation, which is substantially as shown in Figure 1.

In some embodiments of the present application, the single crystal of the compound represented by Formula B is a triclinic system, has a space group $P\bar{1}$, and has the following unit cell parameters: {a=7.04142(7)Å, b=12.15291(7)Å, c=18.13188(10)Å, α=93.2215(5)°, β=95.3039(6)°, γ=91.9554(6)°, V=1541.32(2)Å$^3$}, as shown by using Cu-Kα radiation.

In some embodiments of the present application, the components in the pharmaceutical composition described above (in the first, second, and third aspects) and the weight percentages thereof are as follows:

the active ingredient having a weight percentage of 0.5% to 90%, or 1% to 90%, or 0.5% to 85%, or 0.5% to 80%, or 1% to 80%, or 5% to 80%, or 5% to 75%, or 5% to 70%, or 5% to 65%, or 5% to 60%, or 5% to 55%, or 5% to 50%, or 5% to 45%, or 5% to 40%, or 10% to 70%, or 10% to 65%, or 10% to 60%, or 15% to 60%, or 20% to 60%, or 25% to 60%, or 25% to 50%, or 25% to 45%, or 25% to 40%; and the remainder being an excipient.

In some embodiments of the present application, the components of the pharmaceutical composition described

above (in the first, second, and third aspects) and the weight percentages thereof are as follows:

the active ingredient having a weight percentage of 1% to 90%, preferably 5% to 80%, 5% to 65%, 5% to 55%, 5 % to 45% or 5% to 40%;

an excipient having a weight percentage of 1% to 99%, preferably 5% to 95%, 10% to 90%, 20% to 80% or 30 % to 70%; and the sum of the weight percentage of each component above is 100%.

In some embodiments of the present application, the components in the pharmaceutical composition described above (in the first, second, and third aspects) and the weight percentages thereof are as follows: the active ingredient having a weight percentage of 5% to 90%, or 10% to 90%, or 10% to 80%, or 15% to 80%, or 20% to 70%, or 25% to 60%, or 25% to 50%, or 25% to 45%, or 25% to 40%; and the remainder being an excipient.

The "excipient" according to the present application, also referred to as "pharmaceutically acceptable adjuvant", "adjuvant" or "additive", refers to a generic term for all additional materials, other than the active ingredient, used for prescription formulation and manufacturing of drugs, which are generally pharmaceutically acceptable inert ingredients that have been reasonably evaluated in terms of safety. Examples of the excipient include, without limitation, binders, disintegrants, lubricating adjuvants (lubricants, glidants, anti-adhesive agents), stabilizers, fillers (or referred to as diluents), and substances such as flavoring agents, thickeners, dispersing agents, colouring agents, bacteriostats, antioxidants, pH modifiers, surfactants, perfumes, and coating materials (plasticizers, opacifying agents, pigments). For example, excipients are capable of enhancing the operation characteristics of pharmaceutical formulations, *e.g.*, making the formulation preparation in line with the process requirements by increasing flowability and/or adhesiveness. Further, the "excipient" should have good compatibility with the active ingredient, that is, the excipient *per se* or impurities contained therein do not chemically react with the structural group(s) in the active ingredient or cause degradation of the active ingredient, which would result in a decrease in the content of the active ingredient.

In some embodiments of the present application, the excipient comprises one or more selected from the group consisting of a filler, a binder, a disintegrant, a flavoring agent, a lubricating adjuvant, a bacteriostat, an antioxidant, a pH modifier, a surfactant, a perfume, a solvent, and a coating material.

In some embodiments of the present application, the excipient comprises a filler, and optionally further comprises one or more selected from the group consisting of a binder, a disintegrant, a flavoring agent, a lubricating adjuvant, a bacteriostat, an antioxidant, a surfactant, a perfume, and a coating material.

In some embodiments of the present application, the excipient comprises a filler and optionally further comprises one or more selected from the group consisting of a disintegrant, a binder, and a lubricating adjuvant. In one embodiment, the excipient comprises a filler and a lubricating adjuvant and optionally further comprises a disintegrant and/or a binder. In one embodiment, the excipient is composed of a filler and a lubricating adjuvant. In another embodiment, the excipient is composed of a filler, a lubricating adjuvant, and a disintegrant. In another embodiment, the excipient is composed of a filler, a lubricating adjuvant, a disintegrant, and a binder.

The pharmaceutical composition of the present application may further comprise an additional excipient in addition to a filler, a lubricating adjuvant, and a disintegrant. Specific examples of the additional excipient may be exemplified by a binder, a flavoring agent, a bacteriostat, a pH modifier, a pigment, a thickener, a dispersing agent, a colouring agent, an antioxidant, a stabilizer, a coating material, and a perfume. Preferably, the weight percentage of the additional excipient in the pharmaceutical composition is 0% to 25%, preferably 0% to 20%, 0% to 15%, 0% to 10% or 0% to 5%.

The "filler" or "diluent" according to the present application refers to an excipient which is used to increase the weight and volume of the pharmaceutical composition, so as to facilitate molding and dosing. The filler according to the present application may be a single filler or a mixture of two or more fillers. In some embodiments of the present application, the filler is one or more selected from the group consisting of starch, pregelatinized starch, Confectioner's sugar, magnesium hydroxide, lactose, microcrystalline cellulose, sugar alcohols, and inorganic calcium salts; preferably, the sugar alcohol is one or more selected from the group consisting of xylitol, sorbitol, and mannitol; preferably, the inorganic calcium salt is one or more selected from the group consisting of calcium phosphate, dibasic calcium phosphate, calcium carbonate, and calcium sulfate. In one embodiment, the filler is one of pregelatinized starch, anhydrous dibasic calcium phosphate, calcium carbonate, microcrystalline cellulose, and mannitol, or is a combination of pregelatinized starch and mannitol, or a combination of microcrystalline cellulose and lactose, or a combination of pregelatinized starch and lactose, or a combination of pregelatinized starch and anhydrous dibasic calcium phosphate, or a combination of pregelatinized starch and calcium carbonate. In one embodiment, the filler is a combination of pregelatinized starch and mannitol, a combination of microcrystalline cellulose and lactose, a combination of pregelatinized starch and anhydrous dibasic calcium phosphate, or a combination of pregelatinized starch and calcium carbonate. In a combination of two fillers, the weight ratio of the two fillers is 1: 10 to 10:1, or 1:7 to 7: 1, or 1:6 to 6:1, or 1:5 to 5:1, or 1:4 to 4:1, or 1:3 to 3:1, or 1:2 to 2:1, or 1:1.

The "binder" or "adhesive" according to the present application refers to a viscous excipient that enables non-viscous or less viscous materials or adjuvants to be aggregated and bonded into granules or molded by compression, which may be solid powder or viscous liquid. In some embodiments of the present application, the binder is one or more selected from the group consisting of starch slurry, copovidone, cellulose derivatives, Confectioner's sugar, syrup, polyvinyl pyrrolidone, mucilage, polyethylene glycol 4000, and dextrin; the cellulose derivatives include methyl cellulose, hydroxypropyl methyl cellulose, hydroxypropyl cellulose, ethyl cellulose, and carboxymethyl cellulose sodium; the dextrin includes maltodextrin; the polyvinyl pyrrolidone, also referred to as povidone, includes povidone K30, povidone K25, povidone K90, etc.; the copovidone includes copovidone VA64, copovidone VA64Fine, etc.; the mucilage includes acacia mucilage, gelatin mucilage, etc.

The "disintegrant" according to the present application refers to an excipient used to promote the disintegration of the pharmaceutical composition in gastrointestinal tract and increase the dissolution rate of the active ingredient. In some embodiments of the present application, the pharmaceutical composition comprises no disintegrant. In some embodiments of the present application, the pharmaceutical composition comprises a disintegrant, and the disintegrant is one or more selected from the group consisting of dry starch, carboxymethyl cellulose sodium, microcrystalline cellulose, powdered cellulose, methyl cellulose, polacrilin potassium, sodium alginate, sodium starch glycolate, polyvinylpyrrolidone, maltodextrin, magnesium aluminum silicate, corn starch, pregelatinized starch, crospovidone, low-substituted hydroxypropyl cellulose, carboxymethyl cellulose calcium, effervescent disintegrant, sodium starch glycolate, and croscarmellose sodium. Preferably, the disintegrant is one or more selected from the group consisting of dry starch, pregelatinized starch, crospovidone, low-substituted hydroxypropyl cellulose, sodium starch glycolate, and croscarmellose sodium. In one embodiment, the disintegrant is preferably one or more selected from the group consisting of pregelatinized starch, crospovidone, low-substituted hydroxypropyl cellulose, sodium starch glycolate, and croscarmellose sodium; further preferably crospovidone, sodium starch glycolate or croscarmellose sodium; more preferably sodium starch glycolate. The crospovidone includes crospovidone CL-M, crospovidone CL-SF, crospovidone CL-F, crospovidone CL, etc.

The "flavoring agent" or "flavouring agent" according to the present application refers to a pharmaceutical adjuvant which is used to improve or shield undesirable smell and taste of a drug to make the smell or taste of the drug imperceptible to users, and may be further classified into sweetening agents (or sweeteners), fragrances, mucilages, effervescent agents, and the like. In some embodiments of the present application, the flavoring agent comprises one or more selected from the group consisting of a sweetening agent, a fragrance, a mucilage, and an effervescent agent.

In some embodiments of the present application, the sweetening agent is one or more selected from the group consisting of sorbose, xylose, xylitol, glycerol, disodium glycyrrhizinate, mannose, galactose, maltose, lactose, fructose, sucrol, saccharin sodium, stevioside, glucose, sucrose, sucralose, aspartame, and neotame.

In some embodiments of the present application, the fragrance is selected from the group consisting of a natural volatile aromatic oil and an artificially synthesized essence, etc. For example, the fragrance is one or more selected from the group consisting of fennel oil, rose oil, peppermint oil, orange-peel oil, lemon oil, rose essence, lemon essence, vanilla essence, vanillin, banana essence, pineapple essence, and apple essence.

In some embodiments of the present application, the mucilage is one or more selected from the group consisting of starch, gum arabic, tragacanth, carboxymethyl cellulose, methyl cellulose, sodium alginate, pectin, and agar.

The "lubricating adjuvant" according to the present application is a lubricant in a broad sense, and refers to an excipient which is used to reduce the friction between the granules of the pharmaceutical composition and the friction between the granules and the die hole and to improve the transmission and distribution of force. The lubricating adjuvant is also classified into a lubricant, a glidant, and an anti-adhesive agent according to its functions in terms of reducing friction, increasing the fluidity of granules, and resisting the adhesion between die hole and drug granules.

In some embodiments of the present application, the lubricating adjuvant comprises a lubricant and/or a glidant.

In some embodiments of the present application, the lubricant is one or more selected from the group consisting of stearic acid, magnesium stearate, calcium stearate, palmitic acid, glyceryl palmitostearate, sodium benzoate, sodium lauryl sulfate, talc, silicon dioxide, zinc stearate, sodium stearyl fumarate, magnesium stearyl fumarate, magnesium lauryl sulfate, hydrogenated vegetable oil, sodium dodecyl sulfate, magnesium dodecyl sulfate, and polyethylene glycols, preferably one or more selected from the group consisting of talc, magnesium stearate, calcium stearate, and sodium stearyl fumarate; further preferably magnesium stearate.

In some embodiments of the present application, the glidant is one or more selected from the group consisting of colloidal silicon dioxide and aluminum hydroxide, preferably colloidal silicon dioxide.

In some embodiments of the present application, the pH modifier is one or more selected from the group consisting of sodium hydroxide, sodium carbonate, sodium bicarbonate, and potassium hydroxide.

In some embodiments of the present application, the bacteriostat is one or more selected from the group consisting of sodium benzoate, potassium sorbate, urotropine, ethanol, benzyl alcohol, parabens, benzoic acid, sorbic acid, benzyl alcohol, and phenethyl alcohol.

In some embodiments of the present application, the antioxidant is one or more selected from the group consisting of sodium bisulfite, sodium pyrosulfite, sodium sulfite, anhydrous sodium sulfite, sodium thiosulfate, ascorbic

acid, methionine, thiourea, disodium ethylenediamine tetraacetate, phosphoric acid, citric acid, butylated hydroxyanisole, tert-butyl p-cresol, tocopherol, nordihydroguaiaretic acid, tocopherol, and gallate.

The "coating material", "coating powder", "coating agent" or "coating premix" according to the present application is a mixture of multiple pharmaceutical adjuvants, which is mainly used for colouring, taste masking, protection from light, shelf-life extension, appearance improvement, etc. The "film-coated tablet" or "film coating tablet" according to the present application refers to a tablet coated with a film coating on the tablet core (made from the pharmaceutical composition of the present application by tableting). The film coating may be prepared using the coating materials and methods commonly used in the art. For example, the film coating material typically comprises one or more selected from the group consisting of a film-forming agent (or referred to as a polymer material), a plasticizer, a pore-forming agent, a colouring agent, an opacifying agent, and some solid materials; further, the coating material may be dissolved in a solvent and prepared into a coating solution. The polymer material may be one or more selected from the group consisting of hydroxypropyl methyl cellulose, hydroxypropyl cellulose, methyl cellulose, hydroxyethyl cellulose, acrylic resin, ethyl cellulose, cellulose acetate, cellulose acetate phthalate, polyvinyl acetate phthalate, cellulose acetate trimellitate, hydroxypropyl methyl cellulose phthalate, polyvinyl alcohol and the like. The plasticizer may be selected from the group consisting of glycerol, propylene glycol, polyethylene glycol, glycerol monoacetate, glycerol triacetate, dibutyl sebacate, dibutyl phthalate, diethyl phthalate, castor oil, silicone oil, corn oil, liquid paraffin, etc. The pore-forming agent (also referred to as release rate regulator) may be selected from the group consisting of sucrose, sodium chloride, surfactants, etc. The solid material may be selected from the group consisting of talc, magnesium stearate, colloidal silicon dioxide, etc. The opacifying agent includes titanium dioxide. Conventional colouring agents in the art may also be used as appropriate. For example, the colouring agent is one or more selected from the group consisting of amaranth, carmine, tartrazine, soluble indigo, orange G, eosin, magenta, methylene blue, Sudan yellow, and mercurochrome. The coating material may also be selected directly from commercially available premixed coating powder, such as Opadry® series coating powder, Acryl-EZE® series coating powder, Sureteric® series coating powder, Surelease® series coating powder, and Ethocel® series coating powder. The coating material may be a gastric-soluble coating material, and may also be an enteric-soluble coating material. The weight gain of the film coating accounts for 1% to 5%, preferably 1.5% to 4%, more preferably 1.5% to 3% of the weight of the tablet core. The solvent for coating material is selected from the group consisting of water and ethanol, preferably water, which may be removed during drying without being retained in the final product. For example, the Opadry® series coating powder includes the following models: Opadry 85G64788, 85F18422, 0366507, 8568918, 85F12252, 03F58908, etc.

In some embodiments of the present application, the solvent is one or more selected from the group consisting of water, ethanol, and vegetable oil.

In some embodiments of the present application, the surfactant is one or more selected from the group consisting of polyethylene glycols, polysorbates, sorbitan monolaurates, polyoxyethylene lauryl ethers, emulsifier OP, Milky A, Cetomacrogol-1000, Pluronic, glycerol monooleate, glyceryl monostearate, soft soap, hard soap, aluminium monostearate, calcium stearate, triethanolamine oleate, sodium lauryl sulfate, sodium cetearyl sulfate, sulfated castor oil, sodium dioctyl sulfosuccinate, geramine, bromo-geramine, benzalkonium chloride, Chloramphenicol, and hexadecyl trimethyl ammonium bromide.

In some embodiments of the present application, the pharmaceutical composition described above (in the first, second, and third aspects) is prepared into an oral formulation; preferably, the oral formulation comprises an oral solid formulation and/or an oral liquid formulation; further preferably, the oral solid formulation is one or more selected from the group consisting of a capsule, a tablet, a granule, a fine granule, and a powder; the oral liquid formulation is an oral solution.

In the fourth aspect, the present application provides a pharmaceutical composition, comprising a compound represented by Formula II, Formula A or Formula B as an active ingredient and an excipient, the excipient comprises a filler and optionally further comprises a lubricating adjuvant, a disintegrant and/or a binder.

In some embodiments of the present application, the excipient comprises a filler and a lubricating adjuvant, and optionally further comprises a disintegrant and/or a binder.

In some embodiments of the present application, the excipient comprises a filler, a lubricating adjuvant, and a disintegrant, and optionally further comprises a binder.

In some embodiments of the present application, the excipient comprises a filler, a lubricating adjuvant, a disintegrant, and a binder.

In some embodiments of the present application, the lubricating adjuvant is a lubricant, and optionally further comprises a glidant.

In some embodiments of the present application, the lubricating adjuvant is a combination of a lubricant and a glidant.

In some embodiments of the present application, the filler is one or more selected from the group consisting of starch, Confectioner's sugar, magnesium hydroxide, pregelatinized starch, lactose, microcrystalline cellulose, sugar alcohols, and inorganic calcium salts; preferably, the sugar alcohol-based filler is one or more selected from the group

consisting of mannitol, sorbitol, and xylitol, and the inorganic calcium salt-based filler is one or more selected from the group consisting of calcium phosphate, dibasic calcium phosphate, calcium sulfate, and calcium carbonate; preferably, the filler is one or more selected from the group consisting of pregelatinized starch, lactose, microcrystalline cellulose, mannitol, sorbitol, anhydrous dibasic calcium phosphate, calcium sulfate dihydrate, and calcium carbonate; further preferably, the filler is one or more selected from the group consisting of pregelatinized starch, lactose, microcrystalline cellulose, mannitol, anhydrous dibasic calcium phosphate, and calcium carbonate; further preferably, the filler is one or more selected from the group consisting of pregelatinized starch, lactose, microcrystalline cellulose, anhydrous dibasic calcium phosphate, and mannitol; further preferably, the filler is one or more selected from the group consisting of pregelatinized starch, lactose, microcrystalline cellulose, and mannitol.

In some embodiments of the present application, the filler is one of pregelatinized starch, anhydrous dibasic calcium phosphate, calcium carbonate, and microcrystalline cellulose, or is a combination of pregelatinized starch and mannitol, or a combination of microcrystalline cellulose and lactose, or a combination of pregelatinized starch and anhydrous dibasic calcium phosphate, or a combination of pregelatinized starch and calcium carbonate, or a combination of pregelatinized starch and lactose; preferably, the filler is one of pregelatinized starch, anhydrous dibasic calcium phosphate, and microcrystalline cellulose, or is a combination of pregelatinized starch and mannitol, or a combination of microcrystalline cellulose and lactose, or a combination of pregelatinized starch and anhydrous dibasic calcium phosphate, or a combination of pregelatinized starch and calcium carbonate; further preferably, the filler is one of pregelatinized starch and microcrystalline cellulose, or is a combination of pregelatinized starch and mannitol, or a combination of microcrystalline cellulose and lactose, or a combination of pregelatinized starch and anhydrous dibasic calcium phosphate, or a combination of pregelatinized starch and calcium carbonate; further preferably, the filler is one of pregelatinized starch and microcrystalline cellulose, or a combination of pregelatinized starch and mannitol, or a combination of microcrystalline cellulose and lactose.

In some embodiments of the present application, in the combination of pregelatinized starch and mannitol, the combination of microcrystalline cellulose and lactose, the combination of pregelatinized starch and anhydrous dibasic calcium phosphate, the combination of pregelatinized starch and lactose, or the combination of pregelatinized starch and calcium carbonate, the weight ratio of the two fillers is 1:10 to 10:1, or 1:7 to 7:1, or 1:6 to 6:1, or 1:5 to 5:1, or 1:4 to 4:1, or 1:3 to 3:1, or 1:2 to 2:1, or 1:1.

In some embodiments of the present application, the lubricant is one or more selected from the group consisting of stearic acid, magnesium stearate, calcium stearate, palmitic acid, glyceryl palmitostearate, sodium benzoate, sodium lauryl sulfate, hydrogenated vegetable oil, talc, silicon dioxide, zinc stearate, sodium stearyl fumarate, magnesium stearyl fumarate, magnesium lauryl sulfate, sodium dodecyl sulfate, magnesium dodecyl sulfate, and polyethylene glycols; preferably one or more selected from the group consisting of stearic acid, magnesium stearate, calcium stearate, palmitic acid, glyceryl palmitostearate, sodium benzoate, sodium lauryl sulfate, talc, silicon dioxide, zinc stearate, sodium stearyl fumarate, magnesium stearyl fumarate, magnesium lauryl sulfate, polyethylene glycol 4000, polyethylene glycol 6000, sodium dodecyl sulfate, and magnesium dodecyl sulfate; further preferably one or more selected from the group consisting of stearic acid, magnesium stearate, calcium stearate, palmitic acid, glyceryl palmitostearate, sodium benzoate, sodium lauryl sulfate, talc, silicon dioxide, zinc stearate, sodium stearyl fumarate, magnesium stearyl fumarate, and magnesium lauryl sulfate; further preferably one or more selected from the group consisting of talc, magnesium stearate, calcium stearate, and sodium stearyl fumarate; still further preferably magnesium stearate.

In some embodiments of the present application, the glidant is one or more selected from the group consisting of colloidal silicon dioxide and aluminum hydroxide; preferably colloidal silicon dioxide.

In some embodiments of the present application, the lubricating adjuvant is a combination of a lubricant and a glidant, and the weight ratio of the glidant to the lubricant is 1:6 to 6:1; or 1:5 to 5:1; or 1:4 to 4:1; or 1:3 to 3:1; or 1:2 to 2:1; or 1:1, 1:2, 1:1.5, 2:1, 2:3, 3:1, 3:2, 4:3 or 5:3.

In some embodiments of the present application, the pharmaceutical composition comprises a disintegrant, and the disintegrant is one or more selected from the group consisting of dry starch, carboxymethyl cellulose, microcrystalline cellulose, powdered cellulose, methyl cellulose, polacrilin potassium, sodium alginate, sodium starch glycolate, polyvinylpyrrolidone, maltodextrin, magnesium aluminum silicate, corn starch, pregelatinized starch, crospovidone, low-substituted hydroxypropyl cellulose, carboxymethyl cellulose calcium, effervescent disintegrant, sodium starch glycolate, and croscarmellose sodium; preferably one or more selected from the group consisting of dry starch, pregelatinized starch, sodium starch glycolate, low-substituted hydroxypropyl cellulose, croscarmellose sodium, and crospovidone; preferably one or more selected from the group consisting of crospovidone, low-substituted hydroxypropyl cellulose, sodium starch glycolate, and croscarmellose sodium; further preferably crospovidone, sodium starch glycolate, or croscarmellose sodium; more preferably sodium starch glycolate or croscarmellose sodium.

In some embodiments of the present application, the pharmaceutical composition comprises a binder, and the binder is one or more selected from the group consisting of starch slurry, copovidone, Confectioner's sugar, syrup, polyvinyl pyrrolidone, cellulose derivative, mucilage, polyethylene glycol 4000, and dextrin; preferably, the cellulose derivative is selected from the group consisting of methyl cellulose, hydroxypropyl methyl cellulose, hydroxypropyl

cellulose, ethyl cellulose, and carboxymethyl cellulose sodium; further preferably copovidone, Confectioner's sugar, polyvinyl pyrrolidone, hydroxypropyl methyl cellulose, and carboxymethyl cellulose sodium; further preferably copovidone VA64 and polyvinyl pyrrolidone.

In some embodiments of the present application, the filler is one of pregelatinized starch, microcrystalline cellulose, and anhydrous dibasic calcium phosphate, or is a combination of pregelatinized starch and mannitol, or a combination of microcrystalline cellulose and lactose, or a combination of pregelatinized starch and anhydrous dibasic calcium phosphate, or a combination of pregelatinized starch and calcium carbonate; the lubricating adjuvant is a lubricant and/or a glidant, wherein the lubricant is magnesium stearate, and the glidant is colloidal silicon dioxide; optionally, the disintegrant is selected from the group consisting of sodium starch glycolate and croscarmellose sodium; optionally, the binder is copovidone.

In some embodiments of the present application, the pharmaceutical composition comprises an active ingredient, a filler, a lubricant, and a glidant, wherein the filler is pregelatinized starch or microcrystalline cellulose; preferably, the glidant is colloidal silicon dioxide; further preferably, the lubricant is magnesium stearate; further preferably, the pharmaceutical composition further comprises a disintegrant, and the disintegrant is sodium starch glycolate or croscarmellose sodium.

In some embodiments of the present application, the pharmaceutical composition comprises an active ingredient, a filler, a lubricant, and a glidant, wherein the filler is a combination of pregelatinized starch and mannitol; preferably, the glidant is colloidal silicon dioxide; further preferably, the lubricant is magnesium stearate.

In some embodiments of the present application, the pharmaceutical composition comprises an active ingredient, a filler, and a lubricant, wherein the filler is microcrystalline cellulose; preferably, the lubricant is magnesium stearate.

In some embodiments of the present application, the pharmaceutical composition comprises an active ingredient, a filler, a lubricant, a glidant, and a disintegrant, wherein the filler is a combination of microcrystalline cellulose and lactose; preferably, the glidant is colloidal silicon dioxide; further preferably, the disintegrant is sodium starch glycolate or croscarmellose sodium; still further preferably, the lubricant is magnesium stearate or sodium stearyl fumarate.

In some embodiments of the present application, the pharmaceutical composition comprises an active ingredient, a filler, a lubricant, and a glidant, wherein the filler is anhydrous dibasic calcium phosphate or a combination of anhydrous dibasic calcium phosphate and pregelatinized starch; preferably, the glidant is colloidal silicon dioxide; further preferably, the lubricant is magnesium stearate; still further preferably, the pharmaceutical composition further comprises a disintegrant, and the disintegrant is sodium starch glycolate or croscarmellose sodium.

In some embodiments of the present application, the pharmaceutical composition comprises an active ingredient, a filler, a lubricant, and a glidant, wherein the filler is calcium carbonate or a combination of calcium carbonate and pregelatinized starch; preferably, the glidant is colloidal silicon dioxide; further preferably, the lubricant is magnesium stearate; still further preferably, the pharmaceutical composition further comprises a disintegrant, and the disintegrant is sodium starch glycolate or croscarmellose sodium; still further preferably, the pharmaceutical composition further comprises a binder, and the binder is copovidone.

In some embodiments of the present application, the components of the pharmaceutical composition and the weight percentages thereof are as follows:

the active ingredient having a weight percentage of 0.5% to 90%, or 1% to 90%, or 0.5% to 85%, or 5% to 80%, or 5% to 75%, or 5% to 70%, or 10% to 70%, or 5% to 65%, or 5% to 60%, or 5% to 55%, or 5% to 50%, or 5% to 45%, or 5% to 40%, or 10% to 65%, or 10% to 60%, or 15% to 60%, or 20% to 60%, or 25% to 60%, or 25% to 50%, or 25% to 45%, or 25% to 40%;
a filler having a weight percentage of 5% to 99%, or 10% to 95%, or 15% to 95%, or 15% to 90%, or 15% to 85%, or 15% to 80%, or 20% to 80%, or 25% to 90%, or 25% to 75%, or 30% to 70%, or 35% to 90%, or 35% to 70%, or 40% to 90%, or 40% to 70%, or 45% to 70%, or 50% to 90%, or 50% to 70%, or 55% to 70%;
a disintegrant having a weight percentage of 0% to 20%, or 0% to 15%, or 0% to 10%, or 0% to 8%, or 0% to 6%, or 0% to 5%, or 0% to 4%, or 0% to 3%, or 0.5% to 3%, or 1% to 3%, or 1.5% to 3%, or 2% to 3%;
a binder having a weight percentage of 0% to 10%, or 0% to 6%, or 0% to 5%, or 0% to 4%, or 0% to 3%, or 0% to 2%;
a lubricating adjuvant having a weight percentage of 0% to 18%, or 0.1% to 18%, or 0% to 15%, or 0.1% to 15%, or 0% to 10%, or 0.1% to 10%, or 0% to 8%, or 0% to 7%, or 0% to 6%, or 1% to 6%, or 0.5% to 6%, or 0% to 5%, or 1% to 5%, or 1.5% to 4.5%, or 2% to 4%, or 2% to 3.5%, or 2% to 3%, or 2.5% to 5%, or 3.5% to 5%, or 4% to 5%;
an additional excipient having a weight percentage of 0% to 25%, or 0% to 20%, or 0% to 15%, or 0% to 10%, or 0% to 5%; and
the sum of the weight percentage of each component above is 100%.

In some embodiments of the present application, the components of the pharmaceutical composition and the weight percentages thereof are as follows:

the active ingredient having a weight percentage of 1% to 90%, preferably 5% to 80%, 5 % to 65%, 5% to 55%, 5% to 45% or 5 % to 40%;
a filler having a weight percentage of 10% to 95%, preferably 25% to 90%, 35% to 90%, 40% to 90% or 50% to 90%;
a disintegrant having a weight percentage of 0% to 15%, preferably 0% to 10%, 0% to 6%, 0 % to 5% or 0 % to 3%;
a lubricating adjuvant having a weight percentage of 0% to 18%, preferably 0.1% to 18%, 0% to 15%, 0% to 10%, 0 % to 6%, 0 % to 5%, 0.1% to 15%, 0.1% to 10%, 0.5% to 0%, 1% to 5%, 2.5% to 5%, 3.5% to 5% or 4% to 5%;
an additional excipient having a weight percentage of 0% to 25%, preferably 0 % to 20%, 0% to 15%, 0% to 10% or 0% to 5%; and the sum of the weight percentage of each component above is 100%.

In some embodiments of the present application, the components of the pharmaceutical composition and the weight percentages thereof are as follows:

the active ingredient having a weight percentage of 5% to 90%, or 10% to 90%, or 10% to 80%, or 15% to 80%, or 20% to 70%, or 25% to 60%, or 25% to 50%, or 25% to 45%, or 25% to 40%;
a filler having a weight percentage of 5% to 90%, or 15% to 90%, or 15% to 85%, or 25% to 85%, or 25% to 80%, or 35% to 75%, or 35% to 70%, or 50% to 70%, or 55% to 70%;
a disintegrant having a weight percentage of 0% to 5%, or 0% to 4%, or 0.5% to 4%, or 1% to 3%;
a binder having a weight percentage of 0% to 5%, or 0% to 4%, or 0% to 3%;
a lubricating adjuvant having a weight percentage of 2% to 7%, or 2% to 6%, or 2.5% to 6%, or 3% to 5%, or 3% to 6%, or 3.5% to 6%, or 4% to 6%, or 4% to 5%, or 5% to 6%;
an additional excipient having a weight percentage of 0% to 10%, or 0% to 5%; and the sum of the weight percentage of each component above is 100%.

In some embodiments of the present application, the lubricating adjuvant is a lubricant.
In some embodiments of the present application, the lubricating adjuvant is a combination of a lubricant and a glidant, and the weight ratio of the glidant to the lubricant is 1:6 to 6:1; or 1:5 to 5:1; or 1:4 to 4:1; or 1:3 to 3:1; or 1:2 to 2:1; or 1:1, 1:2, 1:1.5, 2:1, 2:3, 3:1, 3:2, 4:3 or 5:3.
In some embodiments of the present application, the components of the pharmaceutical composition and the weight percentages thereof are as follows:

the active ingredient having a weight percentage of 0.5% to 90%, or 1% to 90%, or 0.5% to 85%, or 5% to 80%, or 5% to 75%, or 5% to 70%, or 10% to 70%, or 5% to 65%, or 5% to 60%, or 5% to 55%, or 5% to 50%, or 5% to 45%, or 5% to 40%, or 10% to 65%, or 10% to 60%, or 15% to 60%, or 20% to 60%, or 25% to 60%, or 25% to 50%, or 25% to 45%, or 25% to 40%;
a filler having a weight percentage of 5% to 99%, or 10% to 95%, or 15% to 95%, or 15% to 90%, or 15% to 85%, or 15% to 80%, or 20% to 80%, or 25% to 90%, or 25% to 75%, or 30% to 70%, or 35% to 90%, or 35% to 70%, or 40% to 90%, or 40% to 70%, or 45% to 70%, or 50% to 90%, or 50% to 70%, or 55% to 70%;
a disintegrant having a weight percentage of 0% to 20%, or 0% to 15%, or 0% to 10%, or 0% to 8%, or 0% to 6%, or 0% to 5%, or 0% to 4%, or 0% to 3%, or 0.5% to .00%, or 1% to 3%, or 1.5% to 3%, or 2% to 3%;
a binder having a weight percentage of 0% to 10%, or 0% to 6%, or 0% to 5%, or 0% to 4%, or 0% to 3%, or 0% to 2%;
a lubricant having a weight percentage of 0% to 10%, or 0% to 8%, or 0.1% to 8%, or 0% to 6%, or 0% to 5%, or 0.1% to 5%, or 0% to 4%, or 0.1% to 4%, or 0% to 3%, or 1% to 5%, or 2% to 4%, or 2% to 3%, or 1% to 4%, or 1% to 3.5%, or 0.5% to 3%, or 1% to 3%, or 1% to 2.5%, or 1% to 2%, or 2% to 2.5%;
a glidant having a weight percentage of 0% to 10%, or 0.5% to 10%, or 0% to 8%, or 0.5% to 8 %, or 0% to 6%, or 0% to 5%, or 0.5% to 5%, or 1% to 5%, or 0% to 4%, or 1% to 4%, or 0% to 3.5%, or 0% to 3%, or 1% to 3%, or 1% to 2.5%, or 1% to 2%, or 1.5% to 3%, or 2% to 3%, or 3%;
an additional excipient having a weight percentage of 0% to 25%, or 0% to 20%, or 0% to 15%, or 0% to 10%, or 0% to 5%; and
the sum of the weight percentage of each component above is 100%.

In some embodiments of the present application, the components of the pharmaceutical composition and the weight percentages thereof are as follows:

the active ingredient having a weight percentage of 1% to 90%, preferably 5% to 80%, 5% to 65 %, 5% to 55%, 5% to 45% or 5% to 40%;
a filler having a weight percentage of 10% to 95%, preferably 25% to 90%, 35% to 90%, 40% to 90% or 50% to 90%;
a disintegrant having a weight percentage of 0 % to 15%, preferably 0% to 10%, 0% to 6%, 0% to 5% or 0 % to 3%;
a lubricant having a weight percentage of 0.1% to 8%, preferably 0.1% to 5%, 0.1% to 4%, 0.5% to 3% or 1% to 3%;

a glidant having a weight percentage of 0% to 10%, preferably 0% to 8%, 0% to 5%, 0% to 4%, or 0% to 3%;
an additional excipient having a weight percentage of 0% to 25%, preferably 0% to 20%, 0% to 15%, 0% to 10% or 0% to 5%; and the sum of the weight percentage of each component above is 100%.

In some embodiments of the present application, the components of the pharmaceutical composition and the weight percentages thereof are as follows:

the active ingredient having a weight percentage of 1% to 90%, preferably 5% to 80%, 5% to 65%, 5% to 55%, 5% to 45% or 5% to 40%;
a filler having a weight percentage of 10% to 95%, preferably 25% to 90%, 35% to 90%, 40% to 90% or 50% to 90%;
a disintegrant having a weight percentage of 0% to 15%, preferably 0% to 10%, .00% to 6%, 0% to 5% or 0% to 3%;
a lubricant having a weight percentage of 0.1% to 8%, preferably 0.1% to 5%, 0.1% to 4%, 0.5% to 3% or 1% to 3%;
a glidant having a weight percentage of 0.5% to 10%, preferably 0.5% to 8%, 0.5% to 5%, 1% to 5%, 1% to 4%, or 1% to 3%;
an additional excipient having a weight percentage of 0% to 25%, preferably 0% to 20%, 0% to 15%, 0% to 10% or 0% to 5%; and the sum of the weight percentage of each component above is 100%.

In some embodiments of the present application, the components of the pharmaceutical composition and the weight percentages thereof are as follows:

the active ingredient having a weight percentage of 1% to 90%, preferably 5% to 80%, 5% to 65%, 5% to 55%, 5% to 45% or 5% to 40%;
a filler having a weight percentage of 10% to 95%, preferably 25% to 90%, 35% to 90%, 40% to 90% or 50% to 90%;
a disintegrant having a weight percentage of 0% to 15%, preferably 0% to 10%, 0% to 6%, 0% to 5% or 0% to 3%;
a lubricant having a weight percentage of 1% to 5%, preferably 2% to 4%, 2% to 3% or 2% to 2.5%;
a glidant having a weight percentage of 1% to 5%, preferably 1% to 4%, 1% to 3%, 1.5% to 3%, 2% to 3% or 3%;
an additional excipient having a weight percentage of 0% to 25%, preferably 0% to 20%, 0% to 15%, 0% to 10% or 0% to 5%; and the sum of the weight percentage of each component above is 100%.

In some embodiments of the present disclosure, the components of the pharmaceutical composition and the weight percentages thereof are as follows:

the active ingredient having a weight percentage of 1% to 90%, preferably 5% to 80%, 5% to 65%, 5% to 55%, 5% to 45% or 5% to 40%;
microcrystalline cellulose having a weight percentage of 10% to 95%, preferably 25% to 90%, 35% to 90%, 40% to 90% or 50% to 90%;
a disintegrant having a weight percentage of 0% to 15%, preferably 0% to 10%, 0% to 6%, 0% to 5% or 0% to 3%;
a lubricant having a weight percentage of 0.1% to 8%, preferably 0.1% to 5%, 0.1% to 4%, 0.5% to 3% or 1% to 2%;
an additional excipient having a weight percentage of 0% to 25%, preferably 0% to 20%, 0% to 15%, 0% to 10% or 0% to 5%; and
the sum of the weight percentage of each component above is 100%.

In some embodiments of the present application, the components of the pharmaceutical composition and the weight percentages thereof are as follows:

the active ingredient having a weight percentage of 5% to 90%, or 10% to 90%, or 10% to 80%, or 15% to 80%, or 20% to 70%, or 25% to 60%, or 25% to 50%, or 25% to 45%, or 25% to 40%;
a filler having a weight percentage of 5% to 90%, or 15% to 90%, or 15% to 85%, or 25% to 85%, or 25% to 80%, or 35% to 75%, or 35% to 70%, or 50% to 70%, or 55% to 70%;
a disintegrant having a weight percentage of 0% to 5%, or 0% to 4%, or 0.5% to 4%, or 1% to 3%;
a binder having a weight percentage of 0% to 5%, or 0% to 4%, or 0% to 3%;
a lubricant having a weight percentage of 1% to 5%, or 1% to 4%, or 2% to 4%, or 2% to 3%;
a glidant having a weight percentage of 0% to 5%, or 0% to 4%, or 1% to 4%, or 1% to 3%, or 0% to 3%, or 2% to 4%, or 3% to 4%, or 2% to 3%;
an additional excipient having a weight percentage of 0% to 10%, or 0% to 5%; and the sum of the weight percentage of each component above is 100%.

In some embodiments of the present application, the components of the pharmaceutical composition and the

weight percentages thereof are as follows:

0.5% to 90% of the active ingredient, 5% to 99% of filler, 0% to 20% of disintegrant, 0% to 10% of binder, 0.1% to 15% of lubricating adjuvant, and 0% to 25% of additional excipient; or
5% to 80% of the active ingredient, 10% to 95% of filler, 0% to 15% of disintegrant, 0% to 6% of binder, 0.50% to 10% of lubricating adjuvant, and 0% to 15% of additional excipient; or
5% to 80% of the active ingredient, 15% to 85% of filler, 0% to 5% of disintegrant, 0% to 4% of binder, 0% to 7% of lubricating adjuvant, and 0% to 10% of additional excipient; or
5% to 80% of the active ingredient, 15% to 80% of filler, 0% to 4% of disintegrant, 0% to 3% of binder, 1% to 6% of lubricating adjuvant, and 0% to 5% of additional excipient; and the sum of the weight percentage of each component above is 100%.

In some embodiments of the present application, the components of the pharmaceutical composition described above and the weight percentages thereof are as follows:

5% to 90% of the active ingredient, 5% to 90% of filler, 0% to 5% of disintegrant, 0% to 5% of binder, 1% to 7% of lubricating adjuvant, and 0% to 5% of additional excipient; or
10% to 90% of the active ingredient, 15% to 90% of filler, 0% to 5% of disintegrant, 0% to 4% of binder, 1% to 6% of lubricating adjuvant, and 0% to 5% of additional excipient; or
10% to 80% of the active ingredient, 15% to 85% of filler, 0% to 5% of disintegrant, 0% to 4% of binder, 3% to 5% of lubricating adjuvant, and 0% to 5% of additional excipient; or
10% to 80% of the active ingredient, 15% to 85% of filler, 0% to 5% of disintegrant, 0% to 4% of binder, 3.5% to 6% of lubricating adjuvant, and 0% to 5% of additional excipient; or
10% to 80% of the active ingredient, 15% to 85% of filler, 0% to 5% of disintegrant, 0% to 4% of binder, 4% to 6% of lubricating adjuvant, and 0% to 5% of additional excipient; or
25% to 60% of the active ingredient, 35% to 70% of filler, 0% to 4% of disintegrant, 0% to 3% of binder, 5% to 6% of lubricating adjuvant, and 0% to 5% of additional excipient; and
the sum of the weight percentage of each component above is 100%.

In some embodiments of the present application, the filler is one of pregelatinized starch, microcrystalline cellulose, anhydrous dibasic calcium phosphate, and calcium carbonate, or is a combination of pregelatinized starch and mannitol, or a combination of microcrystalline cellulose and lactose, or a combination of pregelatinized starch and anhydrous dibasic calcium phosphate, or a combination of pregelatinized starch and calcium carbonate; the lubricating adjuvant is a lubricant and/or a glidant, wherein the lubricant is magnesium stearate, and the glidant is colloidal silicon dioxide; the disintegrant is selected from the group consisting of sodium starch glycolate and croscarmellose sodium; the binder is copovidone, preferably copovidone VA64 and/or copovidone VA64Fine.
In some embodiments of the present application, the filler is a combination of pregelatinized starch and mannitol, a combination of microcrystalline cellulose and lactose, a combination of pregelatinized starch and anhydrous dibasic calcium phosphate, a combination of pregelatinized starch and lactose, or a combination of pregelatinized starch and calcium carbonate. At this time, the weight ratio of the two fillers is 1:10 to 10:1, preferably 1:7 to 7:1, further preferably 1:6 to 6:1, further preferably 1:5 to 5:1, further preferably 1:4 to 4:1, further preferably 1:3 to 3:1, still further preferably 1:2 to 2:1, still further preferably 1:1.
In some embodiments of the present application, the lubricating adjuvant is a lubricant.
In some embodiments of the present application, the lubricating adjuvant is a combination of a lubricant and a glidant, and the weight ratio of the glidant to the lubricant is 1:6 to 6:1; or 1:5 to 5:1; or 1:4 to 4:1; or 1:3 to 3:1; or 1:2 to 2:1; or 1:1, 1:2, 1:1.5, 2:1, 2:3, 3:1, 3:2, 4:3 or 5:3.
In some embodiments of the present application, the components of the pharmaceutical composition and the weight percentages thereof are as follows:

0.50% to 90% of the active ingredient, 5% to 99% of filler, 0% to 20% of disintegrant, 0% to 10% of binder, 0% to 10 % of lubricant, 0% to 10% of glidant, and 0% to 25% of additional excipient; or
5% to 80% of the active ingredient, 10% to 95% of filler, 0% to 10% of disintegrant, 0% to 6% of binder, 0% to 5% of lubricant, 0% to 5% of glidant, and 0% to 25% of additional excipient; or
5% to 80% of the active ingredient, 15% to 85% of filler, 0% to 6% of disintegrant, 0% to 5% of binder, 1% to 4% of lubricant, 0% to 4% of glidant, and 0% to 15% of additional excipient; or
5% to 80% of the active ingredient, 15% to 80% of filler, 0% to 5% of disintegrant, 0% to 4% of binder, 1% to 3.50% of lubricant, 0% to 3.50% of glidant, and 0% to 10% of additional excipient; or
5% to 80% of the active ingredient, 15% to 80% of filler, 0% to 4% of disintegrant, 0% to 3% of binder, 1% to 3% of

lubricant, 0% to 3% of glidant, and 0% to 5% of additional excipient; and the sum of the weight percentage of each component above is 100%.

In some embodiments of the present application, the components of the pharmaceutical composition and the weight percentages thereof are as follows:

5% to 90% of the active ingredient, 5% to 90% of filler, 0% to 5% of disintegrant, 0% to 5% of binder, 1% to 5% of lubricant, 0% to 5% of glidant, and 0% to 5% of additional excipient; or
5% to 90% of the active ingredient, 5% to 90% of filler, 0% to 5% of disintegrant, 0% to 5% of binder, 1% to 4% of lubricant, 0% to 4% of glidant, and 0% to 5% of additional excipient; or
10% to 90% of the active ingredient, 15% to 90% of filler, 0% to 5% of disintegrant, 0% to 4% of binder, 1% to 3% of lubricant, 0% to 3% of glidant, and 0% to 5% of additional excipient; or
10% to 90% of the active ingredient, 15% to 90% of filler, 0% to 4% of disintegrant, 0% to 3% of binder, 1% to 3% of lubricant, 0% to 3% of glidant, and 0% to 5% of additional excipient; and
the sum of the weight percentage of each component above is 100%.

In some embodiments of the present application, the filler is one of pregelatinized starch, microcrystalline cellulose, anhydrous dibasic calcium phosphate, and calcium carbonate, or is a combination of pregelatinized starch and mannitol, or a combination of microcrystalline cellulose and lactose, or a combination of pregelatinized starch and anhydrous dibasic calcium phosphate, or a combination of pregelatinized starch and calcium carbonate; the lubricant is one or more selected from the group consisting of magnesium stearate and sodium stearyl fumarate; the glidant is colloidal silicon dioxide; the disintegrant is selected from the group consisting of sodium starch glycolate and croscarmellose sodium; and the binder is copovidone, preferably copovidone VA64.

In some embodiments of the present application, the filler is selected from the group consisting of a combination of pregelatinized starch and mannitol, a combination of microcrystalline cellulose and lactose, a combination of pregelatinized starch and anhydrous dibasic calcium phosphate, a combination of pregelatinized starch and lactose, and a combination of pregelatinized starch and calcium carbonate, preferably the weight ratio of the two fillers is 1:10 to 10:1, preferably 1:7 to 7:1, further preferably 1:6 to 6:1, further preferably 1:5 to 5:1, further preferably 1:4 to 4:1, further preferably 1:3 to 3:1, still further preferably 1:2 to 2:1, still further preferably 1:1.

In some embodiments of the present application, the components of the pharmaceutical composition and the weight percentages thereof are as follows:

5% to 45% of the active ingredient, 50% to 90% of filler, 0% to 5% of disintegrant, 0% to 5% of binder, 1% to 5% of lubricant, 0% to 5% of glidant, and 0% to 5% of additional excipient; or
5% to 40% of the active ingredient, 50% to 90% of filler, 0% to 5% of disintegrant, 0% to 3% of binder, 2% to 4% of lubricant, 1% to 4% of glidant, and 0% to 5% of additional excipient; or
5% to 40% of the active ingredient, 50% to 90% of filler, 0% to 5% of disintegrant, 0% to 3% of binder, 2% to 3% of lubricant, 2% to 4% of glidant, and 0% to 5% of additional excipient; or
5% to 40% of the active ingredient, 50% to 90% of filler, 0% to 5% of disintegrant, 0% to 3% of binder, 2% to 3% of lubricant, 3% to 4% of glidant, and 0% to 5% of additional excipient; or
20% to 45% of the active ingredient, 50% to 75% of filler, 0% to 5% of disintegrant, 0% to 3% of binder, 2% to 3% of lubricant, 3% to 4% of glidant, and 0% to 5% of additional excipient; or
25% to 40% of the active ingredient, 50% to 70% of filler, 0% to 4% of disintegrant, 0% to 3% of binder, 2% to 3% of lubricant, 3% to 4% of glidant, and 0% to 5% of additional excipient; or
25% to 40% of the active ingredient, 50% to 70% of filler, 2% to 3% of lubricant, and 3% to 4% of glidant; and
the sum of the weight percentage of each component above is 100%.

In some embodiments of the present application, the filler is pregelatinized starch or microcrystalline cellulose, or a combination of pregelatinized starch and mannitol, or a combination of microcrystalline cellulose and lactose; the lubricant is one or more selected from the group consisting of magnesium stearate and sodium stearyl fumarate; the glidant is colloidal silicon dioxide; the disintegrant is selected from the group consisting of sodium starch glycolate and croscarmellose sodium; the binder is copovidone, preferably copovidone VA64.

In some embodiments of the present application, the filler is selected from the group consisting of a combination of pregelatinized starch and mannitol and a combination of microcrystalline cellulose and lactose, preferably the weight ratio of the two fillers is 1:10 to 10:1, preferably 1:7 to 7:1, further preferably 1:6 to 6:1, further preferably 1:5 to 5:1, further preferably 1:4 to 4:1, further preferably 1:3 to 3:1, still further preferably 1:2 to 2:1, still further preferably 1:1.

In some embodiments of the present application, the components of the pharmaceutical composition and the weight percentages thereof are as follows:

5% to 45% of the active ingredient, 50% to 90% of filler, 0% to 5% of disintegrant, 0% to 5% of binder, 1% to 5% of lubricant, 0% to 5% of glidant, and 0% to 5% of additional excipient; or
15% to 45% of the active ingredient, 50% to 70% of filler, 1% to 5% of disintegrant, 0% to 3% of binder, 2% to 4% of lubricant, 3% to 4% of glidant, and 0% to 5% of additional excipient; or
25% to 40% of the active ingredient, 50% to 70% of filler, 1% to 3% of disintegrant, 0% to 3% of binder, 2% to 3% of lubricant, 2% to 4% of glidant, and 0% to 5% of additional excipient; or
25% to 40% of the active ingredient, 50% to 70% of filler, 1% to 3% of disintegrant, 2% to 3% of lubricant, and 2% to 4% of glidant; and
the sum of the weight percentage of each component above is 100%.

In some embodiments of the present application, the filler is a combination of microcrystalline cellulose and lactose; preferably the weight ratio of the two fillers is 1:10 to 10:1, preferably 1:7 to 7:1, further preferably 1:6 to 6:1, further preferably 1:5 to 5:1, further preferably 1:4 to 4:1, further preferably 1:3 to 3:1, still further preferably 1:2 to 2:1, still further preferably 1:1; the lubricant is one or more selected from the group consisting of magnesium stearate and sodium stearyl fumarate; the glidant is colloidal silicon dioxide; the disintegrant is selected from the group consisting of sodium starch glycolate and croscarmellose sodium; the binder is copovidone, preferably copovidone VA64.
In some embodiments of the present application, the components of the pharmaceutical composition and the weight percentages thereof are as follows:

10% to 90% of the active ingredient, 5% to 90% of filler, 0% to 5% of disintegrant, 0% to 5% of binder, 1% to 4% of lubricant, 1% to 4% of glidant, and 0% to 5% of additional excipient; or
10% to 90% of the active ingredient, 5% to 90% of filler, 0% to 5% of disintegrant, 0% to 4% of binder, 1% to 3% of lubricant, 1% to 3% of glidant, and 0% to 5% of additional excipient; or
15% to 70% of the active ingredient, 20% to 80% of filler, 0% to 5% of disintegrant, 0% to 4% of binder, 1% to 3% of lubricant, 1% to 3% of glidant, and 0% to 5% of additional excipient; or
15% to 70% of the active ingredient, 20% to 80% of filler, 0% to 4% of disintegrant, 0% to 3% of binder, 2% to 3% of lubricant, 2% to 3% of glidant, and 0% to 5% of additional excipient; or
15% to 70% of the active ingredient, 20% to 80% of filler, 0% to 4% of disintegrant, 0% to 3% of binder, 2% to 3% of lubricant, and 2% to 3% of glidant; and
the sum of the weight percentage of each component above is 100%.

In some embodiments of the present application, the filler is anhydrous dibasic calcium phosphate or calcium carbonate, or a combination of pregelatinized starch and anhydrous dibasic calcium phosphate, or a combination of pregelatinized starch and calcium carbonate; the lubricant is one or more selected from the group consisting of magnesium stearate and sodium stearyl fumarate; the glidant is colloidal silicon dioxide; the disintegrant is selected from the group consisting of sodium starch glycolate and croscarmellose sodium; the binder is copovidone, preferably copovidone VA64.
In some embodiments of the present application, the filler is a combination of pregelatinized starch and anhydrous dibasic calcium phosphate, or a combination of pregelatinized starch and calcium carbonate, preferably the weight ratio of the two fillers is 1:10 to 10:1, preferably 1:7 to 7:1, further preferably 1:6 to 6:1, further preferably 1:5 to 5:1, further preferably 1:4 to 4:1, further preferably 1:3 to 3:1, still further preferably 1:2 to 2:1, still further preferably 1:1.
In some embodiments of the present application, the components of the pharmaceutical composition and the weight percentages thereof are as follows:

0.50% to 90% of the active ingredient, 5% to 99% of filler, 0% to 20% of disintegrant, 0% to 10% of binder, 0% to 10% of lubricant, 0% to 10% of glidant, and 0% to 25% of additional excipient; or
5% to 80% of the active ingredient, 10% to 95% of filler, 0% to 10% of disintegrant, 0% to 6% of binder, 0% to 5% of lubricant, 0% to 5% of glidant, and 0% to 25% of additional excipient; orand
5% to 80% of the active ingredient, 15% to 85% of filler, 0% to 6% of disintegrant, 0% to 5% of binder, 1% to 4% of lubricant, 0% to 4% of glidant, and 0% to 15% of additional excipient; or
5% to 80% of the active ingredient, 15% to 80% of filler, 0% to 5% of disintegrant, 0% to 4% of binder, 1% to 3.50% of lubricant, 0% to 3.50% of glidant, and 0% to 10% of additional excipient; or
5% to 80% of the active ingredient, 15% to 80% of filler, 0% to 4% of disintegrant, 0% to 3% of binder, 1% to 3% of lubricant, 0% to 3% of glidant, and 0% to 5% of additional excipient; or
5% to 80% of the active ingredient, 15% to 80% of filler, 0% to 4% of disintegrant, 0% to 3% of binder, 1% to 3% of lubricant, 1% to 5% of glidant, and 0% to 5% of additional excipient; or
5% to 80% of the active ingredient, 15% to 80% of filler, 0% to 4% of disintegrant, 0% to 3% of binder, 1% to 3% of lubricant, 1% to 3% of glidant, and 0% to 5% of additional excipient; and
the sum of the weight percentage of each component above is 100%, wherein the filler is pregelatinized starch; the

lubricant is magnesium stearate; the glidant is colloidal silicon dioxide; the disintegrant is sodium starch glycolate and/or croscarmellose sodium; and the binder is copovidone.

In some embodiments of the present application, the components of the pharmaceutical composition and the weight percentages thereof are as follows:

20% to 45% of the active ingredient, 40% to 80% of filler, 0% to 5% of disintegrant, 0% to 5% of binder, 2% to 5% of lubricant, 1% to 5% of glidant, and 0% to 5% of additional excipient; or
20% to 45% of the active ingredient, 40% to 80% of filler, 0% to 3% of disintegrant, 0% to 3% of binder, 2% to 4% of lubricant, 1% to 4% of glidant, and 0% to 5% of additional excipient; or
20% to 45% of the active ingredient, 40% to 80% of filler, 0% to 3% of disintegrant, 0% to 3% of binder, 2% to 3% of lubricant, 2% to 4% of glidant, and 0% to 5% of additional excipient; or
20% to 45% of the active ingredient, 40% to 80% of filler, 0% to 3% of disintegrant, 0% to 3% of binder, 2% to 3% of lubricant, 3% to 4% of glidant, and 0% to 5% of additional excipient; or
25% to 40% of the active ingredient, 50% to 75% of filler, 0% to 3% of disintegrant, 0% to 3% of binder, 2% to 3% of lubricant, 3% to 4% of glidant, and 0% to 5% of additional excipient; or
25% to 40% of the active ingredient, 50% to 70% of filler, 0% to 3% of disintegrant, 0% to 3% of binder, 2% to 3% of lubricant, 3% to 4% of glidant, and 0% to 5% of additional excipient; or
the sum of the weight percentage of each component above is 100%, wherein the filler is pregelatinized starch; the lubricant is magnesium stearate; the glidant is colloidal silicon dioxide; the disintegrant is sodium starch glycolate and/or croscarmellose sodium; and the binder is copovidone.

In some embodiments of the present disclosure, the components of the pharmaceutical composition and the weight percentages thereof are as follows:

5% to 65% of the active ingredient, 35% to 90% of pregelatinized starch, 0% to 6% of disintegrant, 0.1% to 5% of lubricant, 1% to 5% of glidant, and 0% to 25% of additional excipient; or
5% to 55% of the active ingredient, 40% to 90% of pregelatinized starch, 0% to 5% of disintegrant, 0.1% to 4% of lubricant, 1% to 5% of glidant, and 0% to 20% of additional excipient; or
5% to 40% of the active ingredient, 50% to 90% of pregelatinized starch, 0% to 3% of disintegrant, 1% to 3% of lubricant, 1% to 5% of glidant, and 0% to 15% of additional excipient; or
5% to 40% of the active ingredient, 50% to 90% of pregelatinized starch, 0% to 3% of disintegrant, 1% to 3% of lubricant, 1% to 5% of colloidal silicon dioxide, and 0% to 10% of additional excipient; or
5% to 40% of the active ingredient, 50% to 90% of pregelatinized starch, 0% to 3% of disintegrant, 1% to 3% of magnesium stearate, 1% to 5% of colloidal silicon dioxide, and 0% to 5% of additional excipient; or
5% to 40% of the active ingredient, 50% to 90% of pregelatinized starch, 0% to 3% of sodium starch glycolate or croscarmellose sodium, 1% to 3% of magnesium stearate, and 1% to 5% of colloidal silicon dioxide; and
the sum of the weight percentage of each component above is 100%.

In some embodiments of the present application, the components of the pharmaceutical composition and the weight percentages thereof are as follows:

20% to 45% of the active ingredient, 40% to 80% of pregelatinized starch, 0% to 5% of disintegrant, 0% to 5% of binder, 2% to 5% of lubricant, 1% to 5% of glidant, and 0% to 5% of additional excipient; or
20% to 45% of the active ingredient, 40% to 80% of pregelatinized starch, 0% to 3% of disintegrant, 0% to 3% of binder, 2% to 4% of lubricant, 1% to 4% of glidant, and 0% to 5% of additional excipient; or
20% to 45% of the active ingredient, 40% to 80% of pregelatinized starch, 0% to 3% of disintegrant, 0% to 3% of binder, 2% to 3% of lubricant, 2% to 4% of colloidal silicon dioxide, and 0% to 5% of additional excipient; or
20% to 45% of the active ingredient, 40% to 80% of pregelatinized starch, 0% to 3% of disintegrant, 0% to 3% of binder, 2% to 3% of lubricant, 3% to 4% of colloidal silicon dioxide, and 0% to 5% of additional excipient; or
25% to 40% of the active ingredient, 50% to 75% of pregelatinized starch, 0% to 3% of disintegrant, 0% to 3% of binder, 2% to 3% of lubricant, 3% to 4% of colloidal silicon dioxide, and 0% to 5% of additional excipient; or
25% to 40% of the active ingredient, 50% to 70% of pregelatinized starch, 0% to 3% of disintegrant, 0% to 3% of binder, 2% to 3% of lubricant, and 3% to 4% of colloidal silicon dioxide; the sum of the weight percentage of each component above is 100%;
preferably, the lubricant is one or more selected from the group consisting of magnesium stearate and sodium stearyl fumarate; preferably, the glidant is colloidal silicon dioxide; preferably, the disintegrant is one or both of sodium starch glycolate and croscarmellose sodium; preferably, the binder is copovidone, more preferably copovidone VA64.

In some embodiments of the present disclosure, the components of the pharmaceutical composition and the weight percentages thereof are as follows:

5% to 65% of the active ingredient, 10% to 95% of filler (pregelatinized starch and mannitol), 0% to 6% of disintegrant, 0.1% to 5% of lubricant, 1% to 5% of glidant, and 0% to 25% of additional excipient; or
5% to 65% of the active ingredient, 5% to 30% of pregelatinized starch, 5% to 65% of mannitol, 0% to 6% of disintegrant, 0.1% to 5% of lubricant, 1% to 5% of glidant, and 0% to 20% of additional excipient; or
5% to 55% of the active ingredient, 5% to 30% of pregelatinized starch, 5% to 65% of mannitol, 0% to 5% of disintegrant, 0.1% to 4% of lubricant, 1% to 5% of glidant, and 0% to 15% of additional excipient; or
5% to 40% of the active ingredient, 5% to 30% of pregelatinized starch, 5% to 65% of mannitol, 0% to 3% of disintegrant, 1% to 3% of lubricant, 1% to 5% of glidant, and 0% to 10% of additional excipient; or
5% to 40% of the active ingredient, 5% to 30% of pregelatinized starch, 5% to 65% of mannitol, 0% to 3% of disintegrant, 1% to 3% of lubricant, 1% to 5% of colloidal silicon dioxide, and 0% to 10% of additional excipient; or
5% to 40% of the active ingredient, 5% to 30% of pregelatinized starch, 5% to 65% of mannitol, 0% to 3% of disintegrant, 1% to 3% of magnesium stearate, 1% to 5% of colloidal silicon dioxide, and 0% to 5% of additional excipient; or
5% to 40% of the active ingredient, 5% to 30% of pregelatinized starch, 5% to 65% of mannitol, 0% to 3% of sodium starch glycolate or croscarmellose sodium, 1% to 3% of magnesium stearate, and 1% to 5% of colloidal silicon dioxide; and
the sum of the weight percentage of each component above is 100%;
preferably, the weight ratio of pregelatinized starch to mannitol is 1:10 to 10:1, preferably 1:7 to 7:1, further preferably 1:6 to 6:1, further preferably 1:5 to 5:1, further preferably 1:4 to 4:1, further preferably 1:3 to 3:1, still further preferably 1:2 to 2:1.

In some embodiments of the present application, the components of the pharmaceutical composition and the weight percentages thereof are as follows:

5% to 45% of the active ingredient, 50% to 90% of filler (pregelatinized starch and mannitol), 0% to 3% of disintegrant, 0% to 4% of binder, 2% to 4% of lubricant, 2.5% to 5% of glidant, and 0% to 5% of additional excipient; or
5% to 45% of the active ingredient, 5% to 60% of pregelatinized starch, 5% to 65% of mannitol, 0% to 3% of disintegrant, 0% to 3% of binder, 2% to 4% of lubricant, 2.5% to 5% of glidant, and 0% to 5% of additional excipient; or
25% to 45% of the active ingredient, 5% to 55% of pregelatinized starch, 5% to 65% of mannitol, 0% to 3% of disintegrant, 0% to 3% of binder, 2% to 4% of lubricant, 3% to 4% of glidant, and 0% to 5% of additional excipient; and the sum of the weight percentage of each component above is 100%;
preferably, the weight ratio of pregelatinized starch to mannitol is 1:10 to 10:1, preferably 1:7 to 7:1, further preferably 1:6 to 6:1, further preferably 1:5 to 5:1, further preferably 1:4 to 4:1, further preferably 1:3 to 3:1, still further preferably 1:2 to 2:1; preferably, the lubricant is one or more selected from the group consisting of magnesium stearate and sodium stearyl fumarate; preferably, the glidant is colloidal silicon dioxide; preferably, the disintegrant is one or both of sodium starch glycolate and croscarmellose sodium; preferably, the binder is copovidone, preferably copovidone VA64.

In some embodiments of the present application, the components of the pharmaceutical composition and the weight percentages thereof are as follows:

5% to 65% of the active ingredient, 10% to 95% of filler (microcrystalline cellulose and lactose), 0% to 6% of disintegrant, 0.1% to 5% of lubricant, 0.5% to 5% of glidant, and 0% to 25% of additional excipient; or
5% to 65% of the active ingredient, 5% to 50% of microcrystalline cellulose, 5% to 45% of lactose, 0% to 6% of disintegrant, 0.1% to 5% of lubricant, 0.5% to 5% of glidant, and 0% to 20% of additional excipient; or
5% to 55% of the active ingredient, 15% to 45% of microcrystalline cellulose, 10% to 45% of lactose, 0% to 5% of disintegrant, 0.1% to 4% of lubricant, 0.5% to 5% of glidant, and 0% to 15% of additional excipient; or
5% to 40% of the active ingredient, 15% to 45% of microcrystalline cellulose, 10% to 45% of lactose, 0% to 3% of disintegrant, 1% to 2% of lubricant, 0.5% to 5% of glidant, and 0% to 10% of additional excipient; or
5% to 40% of the active ingredient, 15% to 45% of microcrystalline cellulose, 10% to 45% of lactose, 0% to 3% of disintegrant, 1% to 2% of lubricant, 0.5% to 5% of colloidal silicon dioxide, and 0% to 10% of additional excipient; or
5% to 40% of the active ingredient, 15% to 45% of microcrystalline cellulose, 10% to 45% of lactose, 0% to 3% of disintegrant, 1% to 2% of magnesium stearate, 0.5% to 5% of colloidal silicon dioxide, and 0% to 5% of additional excipient; or
5% to 40% of the active ingredient, 15% to 45% of microcrystalline cellulose, 10% to 45% of lactose, 0% to 3% of

sodium starch glycolate or croscarmellose sodium, 1% to 2% of magnesium stearate, and 0.5% to 5% of colloidal silicon dioxide; and

the sum of the weight percentage of each component above is 100%;

preferably, the weight ratio of microcrystalline cellulose to lactose is 1:10 to 10:1, preferably 1:7 to 7:1, further preferably 1:6 to 6:1, further preferably 1:5 to 5:1, further preferably 1:4 to 4:1, further preferably 1:3 to 3:1, still further preferably 1:2 to 2:1.

In some embodiments of the present application, the components of the pharmaceutical composition and the weight percentages thereof are as follows:

20% to 45% of the active ingredient, 50% to 75% of filler (microcrystalline cellulose and lactose), 0% to 5% of disintegrant, 0% to 5% of binder, 2% to 4% of lubricant, 2% to 4% of glidant, and 0% to 5% of additional excipient; or

20% to 45% of the active ingredient, 50% to 75% of filler (microcrystalline cellulose and lactose), 1% to 4% of disintegrant, 0% to 4% of binder, 2% to 3% of lubricant, 3% to 4% of glidant, and 0% to 5% of additional excipient; or

20% to 45% of the active ingredient, 15% to 50% of microcrystalline cellulose, 15% to 45% of lactose, 1% to 4% of disintegrant, 0% to 3% of binder, 2% to 3% of lubricant, 3% to 4% of glidant, and 0% to 5% of additional excipient; or

25% to 45% of the active ingredient, 15% to 50% of microcrystalline cellulose, 15% to 45% of lactose, 1% to 3% of disintegrant, 0% to 3% of binder, 2% to 3% of lubricant, 3% to 4% of glidant, and 0% to 5% of additional excipient; and the sum of the weight percentage of each component above is 100%;

preferably, the lubricant is one or more selected from the group consisting of magnesium stearate and sodium stearyl fumarate; preferably, the glidant is colloidal silicon dioxide; preferably, the disintegrant is one or both of sodium starch glycolate and croscarmellose sodium; preferably, the binder is copovidone, preferably copovidone VA64.

In some embodiments of the present application, the components of the pharmaceutical composition and the weight percentages thereof are as follows:

10% to 90% of the active ingredient, 5% to 85% of anhydrous dibasic calcium phosphate, 0% to 3% of disintegrant, 0% to 3% of binder, 2% to 3% of lubricant, 1% to 3% of glidant, and 0% to 5% of additional excipient; or

10% to 80% of the active ingredient, 15% to 85% of anhydrous dibasic calcium phosphate, 0% to 3% of disintegrant, 0% to 3% of binder, 2% to 3% of lubricant, 1% to 3% of glidant, and 0% to 5% of additional excipient; and the sum of the weight percentage of each component above is 100%;

preferably, the lubricant is one or more selected from the group consisting of magnesium stearate and sodium stearyl fumarate; preferably, the glidant is colloidal silicon dioxide; preferably, the disintegrant is one or both of sodium starch glycolate and croscarmellose sodium; preferably, the binder is copovidone, preferably copovidone VA64.

In some embodiments of the present application, the components of the pharmaceutical composition and the weight percentages thereof are as follows:

15% to 80% of the active ingredient, 5% to 20% of anhydrous dibasic calcium phosphate, 5% to 60% of pregelatinized starch, 0% to 3% of disintegrant, 0% to 3% of binder, 1.5% to 3% of lubricant, 2% to 3% of glidant, and 0% to 5% of additional excipient; and

the sum of the weight percentage of each component above is 100%;

preferably, the lubricant is one or more selected from the group consisting of magnesium stearate and sodium stearyl fumarate; preferably, the glidant is colloidal silicon dioxide; preferably, the disintegrant is one or both of sodium starch glycolate and croscarmellose sodium; preferably, the binder is copovidone, preferably copovidone VA64.

In some embodiments of the present application, the components of the pharmaceutical composition and the weight percentages thereof are as follows:

10% to 80% of the active ingredient, 15% to 75% of calcium carbonate, 0% to 5% of disintegrant, 0% to 3% of binder, 2% to 3% of lubricant, 2% to 3% of glidant, and 0% to 5% of additional excipient; or

10% to 80% of the active ingredient, 15% to 75% of calcium carbonate, 0.5% to 4% of disintegrant, 0% to 3% of binder, 2% to 3% of lubricant, 2% to 3% of glidant, and 0% to 5% of additional excipient; and

the sum of the weight percentage of each component above is 100%;

preferably, the lubricant is one or more selected from the group consisting of magnesium stearate and sodium stearyl fumarate; preferably, the glidant is colloidal silicon dioxide; preferably, the disintegrant is one or both of sodium starch glycolate and croscarmellose sodium; preferably, the binder is copovidone, preferably copovidone VA64.

In some embodiments of the present application, the components of the pharmaceutical composition and the weight percentages thereof are as follows:

10% to 80% of the active ingredient, 5% to 40% of calcium carbonate, 5% to 60% of pregelatinized starch, 0% to 2% of disintegrant, 0% to 3% of binder, 2% to 3% of lubricant, 2% to 3% of glidant, and 0% to 5% of additional excipient; or
10% to 80% of the active ingredient, 5% to 40% of calcium carbonate, 5% to 60% of pregelatinized starch, 0% to 2% of disintegrant, 0% to 2% of binder, 2% to 3% of lubricant, 2% to 3% of glidant, and 0% to 5% of additional excipient; and
the sum of the weight percentage of each component above is 100%;
preferably, the lubricant is one or more selected from the group consisting of magnesium stearate and sodium stearyl fumarate; preferably, the glidant is colloidal silicon dioxide; preferably, the disintegrant is one or both of sodium starch glycolate and croscarmellose sodium; preferably, the binder is copovidone, preferably copovidone VA64.

In some embodiments of the present disclosure, the components of the pharmaceutical composition and the weight percentages thereof are as follows:

5% to 65% of the active ingredient, 10% to 95% of microcrystalline cellulose, 0% to 6% of disintegrant, 0.1% to 5% of lubricant, 0% to 10% of glidant, and 0% to 25% of additional excipient; or
5% to 55% of the active ingredient, 20% to 85% of microcrystalline cellulose, 0% to 5% of disintegrant, 0.1% to 4% of lubricant, 0% to 8% of glidant, and 0% to 20% of additional excipient; or
5% to 45% of the active ingredient, 30% to 75% of microcrystalline cellulose, 0% to 4% of disintegrant, 1% to 3% of lubricant, 0% to 3% of glidant, and 0% to 10% of additional excipient; and
the sum of the weight percentage of each component above is 100%.

In some embodiments of the present application, the pharmaceutical composition described above (in the fourth aspect) may further comprise an additional excipient except for the excipients of aforesaid types. The additional excipient is selected from the group consisting of a binder, a flavoring agent, a bacteriostat, a pH modifier, a pigment, a thickener, a dispersing agent, a colouring agent, an antioxidant, a stabilizer, a fragrance, a coating material, etc. In the embodiments of the present application, the weight percentage of the additional excipient in the pharmaceutical composition is 0% to 25%, preferably 0% to 20%, 0% to 15%, 0% to 10% or 0% to 5%.

In some embodiments of the present application, the components of the pharmaceutical composition and the weight percentages thereof are as follows: 10% to 70% of the active ingredient, 30% to 80% of pregelatinized starch, 0% to 5% of colloidal silicon dioxide, 0% to 5% of magnesium stearate, and 0% to 5% of sodium starch glycolate and/or croscarmellose sodium, and the sum of the weight percentage of each component is 100%.

Preferably, the components of the pharmaceutical composition and the weight percentages thereof are as follows: 15% to 60% of the active ingredient, 40% to 80% of pregelatinized starch, 1% to 5% of colloidal silicon dioxide, 1% to 5% of magnesium stearate, and 0% to 5% of sodium starch glycolate and/or croscarmellose sodium, and the sum of the weight percentage of each component is 100%.

Preferably, the components of the pharmaceutical composition and the weight percentages thereof are as follows: 20% to 50% of the active ingredient, 45% to 75% of pregelatinized starch, 1% to 4% of colloidal silicon dioxide, 1% to 5% of magnesium stearate, and 0% to 5% of sodium starch glycolate and/or croscarmellose sodium, and the sum of the weight percentage of each component is 100%.

Preferably, the components of the pharmaceutical composition and the weight percentages thereof are as follows: 25% to 45% of the active ingredient, 50% to 70% of pregelatinized starch, 1% to 4% of colloidal silicon dioxide, 1% to 4% of magnesium stearate, and 0% to 4% of sodium starch glycolate and/or croscarmellose sodium, and the sum of the weight percentage of each component is 100%.

Preferably, the components of the pharmaceutical composition and the weight percentages thereof are as follows: 25% to 40% of the active ingredient, 56% to 68% of pregelatinized starch, 1% to 4% of colloidal silicon dioxide, 2% to 4% of magnesium stearate, and 0% to 4% of sodium starch glycolate and/or croscarmellose sodium, and the sum of the weight percentage of each component is 100%.

Preferably, the components of the pharmaceutical composition and the weight percentages thereof are as follows: 25% to 40% of the active ingredient, 56% to 68% of pregelatinized starch, 1% to 3% of colloidal silicon dioxide, 2% to 3% of magnesium stearate, and 0% to 4% of sodium starch glycolate and/or croscarmellose sodium, and the sum of the weight percentage of each component is 100%.

Preferably, the components of the pharmaceutical composition and the weight percentages thereof are as follows: 25% to 40% of the active ingredient, 56% to 68% of pregelatinized starch, 3% to 5% of colloidal silicon dioxide, and 2% to 3% of magnesium stearate, and the sum of the weight percentage of each component is 100%.

Preferably, the components of the pharmaceutical composition and the weight percentages thereof are as follows: 25% to 40% of the active ingredient, 56% to 68% of pregelatinized starch, 3% to 4% of colloidal silicon dioxide, and 2% to 3% of magnesium stearate, and the sum of the weight percentage of each component is 100%.

In other embodiments of the present application, the components of the pharmaceutical composition and the weight percentages thereof are as follows: 25% to 50% of the active ingredient, 50% to 75% of microcrystalline cellulose, 0% to 5% of colloidal silicon dioxide, 0% to 3% of magnesium stearate, and 0% to 5% of sodium starch glycolate and/or croscarmellose sodium, and the sum of the weight percentage of each component is 100%.

Preferably, the components of the pharmaceutical composition and the weight percentages thereof are as follows: 25% to 40% of the active ingredient, 55% to 75% of microcrystalline cellulose, 0% to 5% of colloidal silicon dioxide, 1% to 3% of magnesium stearate, and 0% to 5% of sodium starch glycolate and/or croscarmellose sodium, and the sum of the weight percentage of each component is 100%.

Preferably, the components of the pharmaceutical composition and the weight percentages thereof are as follows: 30% to 35% of the active ingredient, 60% to 70% of microcrystalline cellulose, 0% to 3% of colloidal silicon dioxide, 1% to 2% of magnesium stearate, and 0% to 4% of sodium starch glycolate and/or croscarmellose sodium, and the sum of the weight percentage of each component is 100%.

Preferably, the components of the pharmaceutical composition and the weight percentages thereof are as follows: 30% to 35% of the active ingredient, 60% to 70% of microcrystalline cellulose, 0% to 3% of colloidal silicon dioxide, and 1% to 2% of magnesium stearate, and the sum of the weight percentage of each component is 100%.

In other embodiments of the present application, the components of the pharmaceutical composition and the weight percentages thereof are as follows: 5% to 50% of the active ingredient, 5% to 55% of pregelatinized starch, 5% to 70% of mannitol, 0% to 5% of colloidal silicon dioxide, 1% to 3% of magnesium stearate, and 0% to 5% of sodium starch glycolate and/or croscarmellose sodium, and the sum of the weight percentage of each component is 100%.

Preferably, the components of the pharmaceutical composition and the weight percentages thereof are as follows: 20% to 50% of the active ingredient, 5% to 55% of pregelatinized starch, 5% to 70% of mannitol, 1% to 5% of colloidal silicon dioxide, 1% to 3% of magnesium stearate, and 0% to 5% of sodium starch glycolate and/or croscarmellose sodium, and the sum of the weight percentage of each component is 100%.

Preferably, the components of the pharmaceutical composition and the weight percentages thereof are as follows: 25% to 40% of the active ingredient, 10% to 50% of pregelatinized starch, 5% to 63% of mannitol, 1% to 4% of colloidal silicon dioxide, 1% to 3% of magnesium stearate, and 0% to 5% of sodium starch glycolate and/or croscarmellose sodium, and the sum of the weight percentage of each component is 100%.

Preferably, the components of the pharmaceutical composition and the weight percentages thereof are as follows: 25% to 40% of the active ingredient, 10% to 50% of pregelatinized starch, 5% to 63% of mannitol, 1% to 3% of colloidal silicon dioxide, 1% to 3% of magnesium stearate, and 0% to 5% of sodium starch glycolate and/or croscarmellose sodium, and the sum of the weight percentage of each component is 100%.

Preferably, the components of the pharmaceutical composition and the weight percentages thereof are as follows: 25% to 40% of the active ingredient, 10% to 50% of pregelatinized starch, 5% to 63% of mannitol, 3% to 4% of colloidal silicon dioxide, 2% to 3% of magnesium stearate, and 0% to 5% of sodium starch glycolate and/or croscarmellose sodium, and the sum of the weight percentage of each component is 100%.

Preferably, the components of the pharmaceutical composition and the weight percentages thereof are as follows: 25% to 40% of the active ingredient, 10% to 50% of pregelatinized starch, 5% to 63% of mannitol, 3% to 4% of colloidal silicon dioxide, and 2% to 3% of magnesium stearate, and the sum of the weight percentage of each component is 100%.

In some embodiments of the present application, the components of the pharmaceutical composition and the weight percentages thereof are as follows: 23% to 50% of the active ingredient, 10% to 55% of microcrystalline cellulose, 10% to 50% of lactose, 0% to 5% of colloidal silicon dioxide, 1% to 4% of magnesium stearate or sodium stearyl fumarate, and 0% to 5% of sodium starch glycolate and/or croscarmellose sodium, and the sum of the weight percentage of each component is 100%.

Preferably, the components of the pharmaceutical composition and the weight percentages thereof are as follows: 23% to 40% of the active ingredient, 10% to 50% of microcrystalline cellulose, 10% to 50% of lactose, 1% to 5% of colloidal silicon dioxide, 1% to 4% of magnesium stearate or sodium stearyl fumarate, and 0% to 5% of sodium starch glycolate and/or croscarmellose sodium, and the sum of the weight percentage of each component is 100%.

Preferably, the components of the pharmaceutical composition and the weight percentages thereof are as follows: 28% to 35% of the active ingredient, 15% to 45% of microcrystalline cellulose, 15% to 45% of lactose, 1% to 4% of colloidal silicon dioxide, 2% to 3% of magnesium stearate or sodium stearyl fumarate, and 1% to 4% of sodium starch glycolate and/or croscarmellose sodium, and the sum of the weight percentage of each component is 100%.

Preferably, the components of the pharmaceutical composition and the weight percentages thereof are as follows: 28% to 35% of the active ingredient, 15 % to 45% of microcrystalline cellulose, 15% to 45% of lactose, 1% to 3% of colloidal silicon dioxide, 2% to 3% of magnesium stearate or sodium stearyl fumarate, and 1% to 4% of sodium

starch glycolate and/or croscarmellose sodium, and the sum of the weight percentage of each component is 100%.

Preferably, the components of the pharmaceutical composition and the weight percentages thereof are as follows: 28% to 35% of the active ingredient, 15% to 45% of microcrystalline cellulose, 15% to 45% of lactose, 3% to 5% of colloidal silicon dioxide, 2% to 4% of magnesium stearate or sodium stearyl fumarate, and 1% to 4% of sodium starch glycolate and/or croscarmellose sodium, and the sum of the weight percentage of each component is 100%.

Preferably, the components of the pharmaceutical composition and the weight percentages thereof are as follows: 28% to 35% of the active ingredient, 15% to 45% of microcrystalline cellulose, 15% to 45% of lactose, 3% to 4% of colloidal silicon dioxide, 2% to 3% of magnesium stearate or sodium stearyl fumarate, and 1% to 3% of sodium starch glycolate and/or croscarmellose sodium, and the sum of the weight percentage of each component is 100%.

In some embodiments of the present application, the components of the pharmaceutical composition and the weight percentages thereof are as follows: 10% to 90% of the active ingredient, 5% to 85% of anhydrous dibasic calcium phosphate, 1% to 5% of colloidal silicon dioxide, 1% to 4% of magnesium stearate, and 0% to 5% of sodium starch glycolate and/or croscarmellose sodium, and the sum of the weight percentage of each component is 100%.

Preferably, the components of the pharmaceutical composition and the weight percentages thereof are as follows: 10% to 80% of the active ingredient, 15% to 85% of anhydrous dibasic calcium phosphate, 1% to 4% of colloidal silicon dioxide 2% to 3% of magnesium stearate, and 0% to 4% of sodium starch glycolate and/or croscarmellose sodium, and the sum of the weight percentage of each component is 100%.

Preferably, the components of the pharmaceutical composition and the weight percentages thereof are as follows: 50% to 60% of the active ingredient, 35% to 40% of anhydrous dibasic calcium phosphate, 1% to 3% of colloidal silicon dioxide, 2% to 3% of magnesium stearate, and 0% to 4% of sodium starch glycolate and/or croscarmellose sodium, and the sum of the weight percentage of each component is 100%.

In some embodiments of the present application, the components of the pharmaceutical composition and the weight percentages thereof are as follows: 10% to 85% of the active ingredient, 5% to 23% of anhydrous dibasic calcium phosphate, 5% to 70% of pregelatinized starch, 1% to 4% of colloidal silicon dioxide, 1% to 4% of magnesium stearate, and 0% to 4% of sodium starch glycolate and/or croscarmellose sodium, and the sum of the weight percentage of each component is 100%.

Preferably, the components of the pharmaceutical composition and the weight percentages thereof are as follows: 15% to 80% of the active ingredient, 7% to 18% of anhydrous dibasic calcium phosphate, 7% to 63% of pregelatinized starch, 1% to 3% of colloidal silicon dioxide, 2% to 3% of magnesium stearate, and 0% to 4% of sodium starch glycolate and/or croscarmellose sodium, and the sum of the weight percentage of each component is 100%.

In some embodiments of the present application, the components of the pharmaceutical composition and the weight percentages thereof are as follows: 10% to 82% of the active ingredient, 10% to 85% of calcium carbonate, 1% to 4% of colloidal silicon dioxide, 1% to 4% of magnesium stearate, 0 to 5% of copovidone VA64 and 0% to 4% of sodium starch glycolate and/or croscarmellose sodium, and the sum of the weight percentage of each component is 100%.

Preferably, the components of the pharmaceutical composition and the weight percentages thereof are as follows: 12% to 80% of the active ingredient, 15% to 78% of calcium carbonate, 1% to 3% of colloidal silicon dioxide, 2% to 3% of magnesium stearate, 0% to 3% of copovidone VA64, and 0.5% to 4% of sodium starch glycolate and/or croscarmellose sodium, and the sum of the weight percentage of each component is 100%.

In some embodiments of the present application, the components of the pharmaceutical composition and the weight percentages thereof are as follows: 10% to 83% of the active ingredient, 5% to 50% of calcium carbonate, 5% to 68% of pregelatinized starch, 1% to 4 % of colloidal silicon dioxide, 1% to 4% of magnesium stearate, 0% to 3% of copovidone VA64, and 0% to 2% of sodium starch glycolate and/or croscarmellose sodium, and the sum of the weight percentage of each component is 100%.

Preferably, the components of the pharmaceutical composition and the weight percentages thereof are as follows: 12% to 80% of the active ingredient, 7% to 45% of calcium carbonate, 7% to 65% of pregelatinized starch, 1% to 3% of colloidal silicon dioxide, 1.5% to 3% of magnesium stearate, 0% to 2% of copovidone VA64, and 0% to 1% of sodium starch glycolate and/or croscarmellose sodium, and the sum of the weight percentage of each component is 100%.

Preferably, the components of the pharmaceutical composition and the weight percentages thereof are as follows: 12% to 80% of the active ingredient, 7% to 45% of calcium carbonate, 7% to 65% of pregelatinized starch, 2% to 3% of colloidal silicon dioxide, 2% to 3% of magnesium stearate, 0% to 2% of copovidone VA64 and 0% to 1% of sodium starch glycolate and/or croscarmellose sodium, and the sum of the weight percentage of each component is 100%.

In some embodiments of the present application, the pharmaceutical composition is prepared into an oral solid formulation; preferably, the oral solid formulation is selected from the group consisting of a capsule, a tablet, a powder, and a fine granule; further preferably a capsule, a tablet, or a fine granule; further preferably a capsule or a tablet; still further preferably a capsule.

In some embodiments of the present application, the tablet is a film-coated tablet or an uncoated tablet.

In the fifth aspect, the present application provides a pharmaceutical composition, comprising a compound represented by Formula II, Formula A or Formula B as an active ingredient, and an excipient, and the excipient comprises

a filler and a binder and optionally further comprises a flavoring agent.

In some embodiments of the present application, the excipient comprises a filler, a binder, and a flavoring agent.

In some embodiments of the present application, the flavoring agent comprises a fragrance, and optionally further comprises a sweetening agent.

In some embodiments of the present application, the filler is one or more selected from the group consisting of sugar alcohols; preferably, the sugar alcohol-based filler is one or more selected from the group consisting of lactose, mannitol, and sorbitol; preferably, the filler is mannitol.

In some embodiments of the present application, the binder is one or more selected from the group consisting of copovidone, Confectioner's sugar, syrup, polyvinyl pyrrolidone, cellulose derivative, mucilage, and dextrin; preferably, the cellulose derivative is hydroxypropyl methyl cellulose and/or carboxymethyl cellulose sodium; further preferably copovidone, Confectioner's sugar, polyvinyl pyrrolidone, hydroxypropyl methyl cellulose and/or carboxymethyl cellulose sodium; further preferably copovidone and/or polyvinyl pyrrolidone.

In some embodiments of the present application, the sweetening agent is selected from the group consisting of sorbose, xylose, xylitol, glycerol, disodium glycyrrhizinate, mannose, galactose, maltose, lactose, fructose, sucrol, saccharin sodium, stevioside, glucose, sucrose, sucralose, aspartame, and neotame; preferably xylitol, sucralose, aspartame, and neotame.

In some embodiments of the present application, the fragrance is one or more selected from the group consisting of oils and essences; preferably one or more selected from the group consisting of fennel oil, rose oil, peppermint oil, orange-peel oil, lemon oil, rose essence, lemon essence, vanilla essence, vanillin, banana essence, pineapple essence, and apple essence; further preferably one or more selected from the group consisting of lemon essence, pineapple essence, and apple essence.

In some embodiments of the present application, the components of the pharmaceutical composition and the weight percentages thereof are as follows:

the active ingredient having a weight percentage of 0.50% to 30%, or 0.60% to 25%, or 0.70% to 20%, or 0.80% to 15%, or 1% to 10%, or 1.50% to 8%, or 2% to 6%, or 2.50% to 5%, or 3% to 4.50%;
a filler having a weight percentage of 50% to 99.90%, or 60% to 99.50%, or 70% to 99%, or 80% to 99%, or 85% to 99%;
a binder having a weight percentage of 0% to 10%, or 0% to 6%, or 0% to 5%, or 0% to 4%, or 0% to 3%, or 0% to 2%;
a flavoring agent having a weight percentage of 0% to 10%, or 0% to 6%, or 0% to 5%, or 0% to 4%, or 0% to 3%, or 0% to 2%, or 0% to 1%;
an additional excipient having a weight percentage of 0% to 25%, or 0% to 20%, or 0% to 15%, or 0% to 10%, or 0% to 5%, or 0.10% to 3%, or 0.10% to 2%, or 0.10% to 1%; and the sum of the weight percentage of each component above is 100%.

In some embodiments of the present application, the filler is mannitol; the binder is copovidone, preferably copovidone VA64 or copovidone VA64Fine; the flavoring agent is a sweetening agent and/or a fragrance; the sweetening agent is selected from the group consisting of xylitol, sucralose, aspartame and/or neotame; the fragrance is lemon essence; and the components of the pharmaceutical composition and the weight percentages thereof are as follows:

0.50% to 30% of the active ingredient, 50% to 99.90% of filler, 0% to 10% of binder, 0% to 10% of flavoring agent, and 0% to 25% of additional excipient; or
0.60% to 25% of the active ingredient, 60% to 99.50% of filler, 0% to 8% of binder, 0% to 6% of flavoring agent, and 0% to 15% of additional excipient; or
0.70% to 20% of the active ingredient, 70% to 99% of filler, 2% to 8% of binder, 0% to 3% of flavoring agent, and 0% to 10% of additional excipient; or
1% to 10% of the active ingredient, 85% to 99% of filler, 3% to 8% of binder, 0% to 1% of flavoring agent, and 0% to 2% of additional excipient;
1% to 5% of the active ingredient, 85% to 95% of filler, 4% to 8% of binder, 0% to 1% of flavoring agent, and 0% to 2% of additional excipient; and
the sum of the weight percentage of each component above is 100%.

Preferably, in the pharmaceutical composition, the filler is mannitol; the binder is copovidone, preferably copovidone VA64; and the flavoring agent is lemon essence.

In some embodiments of the present application, the pharmaceutical composition according to each aspect described above may further comprise an additional excipient except for the excipients of aforesaid types. Specific examples thereof may be exemplified by a disintegrant, a bacteriostat, a thickener, a dispersing agent, an antioxidant, a stabilizer, and the like. In the embodiments of the present application, the weight percentage of the additional excipient in the pharmaceutical composition is 0% to 25%, preferably 0% to 20%, 0% to 15%, 0% to 10% or 0% to 5%.

In some embodiments of the present application, the pharmaceutical composition is prepared into an oral solid formulation; preferably, the oral solid formulation is selected from the group consisting of a granule, a powder, and a fine granule; further preferably a granule.

In the sixth aspect, the present disclosure provides a pharmaceutical composition, comprising a compound represented by Formula II or Formula A as an active ingredient and an excipient, and the excipient comprises a solvent, and optionally further comprises one or more selected from the group consisting of a flavoring agent, a bacteriostat, and a pH modifier.

In some embodiments of the present application, the excipient comprises a solvent and a flavoring agent, and optionally further comprises one or more selected from the group consisting of a bacteriostat and a pH modifier.

In some embodiments of the present application, the excipient comprises a solvent, a flavoring agent, and a pH modifier, and optionally further comprises a bacteriostat.

In some embodiments of the present application, the flavoring agent comprises a sweetening agent, and optionally further comprises a fragrance.

In some embodiments of the present application, the sweetening agent described above is one or more selected from the group consisting of sorbose, xylose, xylitol, glycerol, disodium glycyrrhizinate, mannose, galactose, maltose, lactose, fructose, sucrol, saccharin sodium, stevioside, glucose, sucrose, sucralose, aspartame and/or neotame; preferably one or more selected from the group consisting of xylitol, sucralose, aspartame and/or neotame.

In some embodiments of the present application, the fragrance described above is one or more selected from the group consisting of oils and essences; preferably one or more selected from the group consisting of fennel oil, rose oil, peppermint oil, orange-peel oil, lemon oil, rose essence, lemon essence, vanilla essence, vanillin, banana essence, pineapple essence, and apple essence; further preferably one or more selected from the group consisting of lemon essence, pineapple essence, and apple essence.

In some embodiments of the present application, the solvent described above is selected from the group consisting of water, ethanol, and vegetable oil; preferably water; further preferably purified water.

In some embodiments of the present application, the bacteriostat described above is one or more selected from the group consisting of sodium benzoate, potassium sorbate, urotropine, ethanol, benzyl alcohol, and parabens; preferably one or more selected from the group consisting of sodium benzoate, potassium sorbate, urotropine, ethanol, benzyl alcohol, methyl parahydroxybenzoate, ethyl parahydroxybenzoate, and propyl parahydroxybenzoate; further preferably one or more selected from the group consisting of sodium benzoate, potassium sorbate, methyl parahydroxybenzoate, ethyl parahydroxybenzoate, and propyl parahydroxybenzoate.

In some embodiments of the present application, the pH modifier described above is one or more selected from the group consisting of sodium hydroxide, sodium bicarbonate, and sodium carbonate.

In some embodiments of the present application, the components of the pharmaceutical composition and the weight percentages thereof are as follows:

the active ingredient having a weight percentage of 0.10% to 40%, or 0.10% to 35%, or 0.10% to 30%, or 0.50% to 25%, or 0.50% to 20%, or 1% to 10%;
a solvent having a weight percentage of 90% to 99.99%, or 95% to 99.99%, or 96% to 99.99%, or 97% to 99.99%;
a sweetening agent having a weight percentage of 0% to 5%, or 0.01% to 4%, or 0.05% to 3%, or 0.10% to 2%, or 0.10% to 1%, or 0.10% to 0.50%;
a fragrance having a weight percentage of 0% to 5%, or 0.01% to 4%, or 0.05% to 3%, or 0.05% to 2%, or 0.05% to 1.50%, or 0.05% to 1%;
a bacteriostat having a weight percentage of 0% to 6%, or 0% to 5%, or 0% to 4%, or 0% to 3%, or 0% to 2.50%, or 0% to 2%, or 0% to 1.50%, or 0% to 1%, or 0% to 0.50%;
an additional excipient having a weight percentage of 0% to 25%, or 0% to 20%, or 0% to 15%, or 0% to 10%, or 0% to 5%; and
a pH modifier;
wherein the amount of the pH modifier is subject to the actual amount required to reach pH 4 to 6, and the sum of the weight percentage of each component above is 100%.

In some embodiments of the present application, the solvent is water; the fragrance is lemon essence; the sweetening agent is neotame; and the pH modifier is sodium hydroxide.

In some embodiments of the present application, the components of the pharmaceutical composition and the weight percentages thereof are as follows:

0.10% to 10% of the active ingredient, 90% to 99.99% of solvent, 0% to 5% of sweetening agent, 0% to 5% of fragrance, 0% to 6% of bacteriostat, and 0% to 25% of additional excipient; or
0.10% to 5% of the active ingredient, 95% to 99.99% of solvent, 0.05% to 3% of sweetening agent, 0.05% to 3% of

fragrance, 0% to 4% of bacteriostat, and 0% to 25% of additional excipient; or
0.50% to 4% of the active ingredient, 96% to 99.99% of solvent, 0.10% to 2% of sweetening agent, 0.05% to 2% of fragrance, 0% to 2.50% of bacteriostat, and 0% to 25% of additional excipient; or
1% to 3% of the active ingredient, 97% to 99.99% of solvent, 0.10% to 1% of sweetening agent, 0% to 1% of fragrance, 0% to 1% of bacteriostat, and 0% to 25% of additional excipient; and
a pH modifier;
wherein the amount of the pH modifier is subject to the actual amount required to reach pH 4 to 6, and the sum of the weight percentage of each component above is 100%.

In some embodiments of the present application, the pharmaceutical composition according to each aspect described above may further comprise an additional excipient except for the excipients of aforesaid types. Specific examples thereof may be exemplified by a thickener, a dispersing agent, a surfactant, an antioxidant, a stabilizer, and the like. In the embodiments of the present application, the weight percentage of the additional excipient in the pharmaceutical composition is 0% to 25%, preferably 0% to 20%, 0% to 15%, 0% to 10% or 0% to 5%.
In some embodiments of the present application, the pharmaceutical composition is prepared into an oral solution.
In some embodiments of the present application, the pharmaceutical composition may be prepared into a single-dose form or a multi-dose form.
In some embodiments of the present application, the single-dose form of the oral solution is 1 to 30 mL, preferably 1 to 20 mL, further preferably 1 to 15 mL, further preferably 1 to 10 mL, further preferably 1 mL, 2 mL, 3 mL, 4 mL, 5 mL, 6 mL, 7 mL, 8 mL, 9 mL or 10 mL.
In some embodiments of the present application, the multi-dose form of the oral solution is 2 to 500 mL, preferably 2 to 250 mL, further preferably 5 to 250 mL, further preferably 10 to 200 mL, further preferably 15 to 150 mL, further preferably 20 to 100 mL, further preferably 20 to 50 mL, further preferably 30 to 50 mL.
In some embodiments of the present application, the pharmaceutical composition described above (in the first to sixth aspects) contains 0.001 to 1000 mg of the active ingredient, preferably contains the active ingredient in a dose range of 0.1 to 800 mg, further preferably 1 to 500 mg, further preferably 10 to 300 mg, further preferably 10 to 200 mg, still further preferably 10 to 100 mg. For example, the pharmaceutical composition in a single-dose form contains 5 mg, 10 mg, 20 mg, 25 mg, 30 mg, 50 mg, 60 mg, 100 mg, 150 mg, 200 mg, 250 mg, 300 mg, 500 mg or the like of the active ingredient, wherein the weight of the active ingredient is in terms of a hydrochloride form (e.g., in terms of $C_{24}H_{29}N_9{\cdot}3HCl$ in some embodiments of the present application).
In some embodiments of the present application, the oral formulation, oral solid formulation or oral solution described above (in the first to sixth aspects) may contain 0.001 to 1000 mg of an active ingredient, preferably contains the active ingredient in a dose range of 0.1 to 800 mg, further preferably 1 to 500 mg, further preferably 10 to 300 mg, further preferably 10 to 200 mg, still further preferably 10 to 100 mg in each dosage unit. For example, the oral formulation in a single-dose form contains 5 mg, 10 mg, 20 mg, 25 mg, 30 mg, 50 mg, 60 mg, 100 mg, 150 mg, 200 mg, 250 mg, 300 mg, 500 mg or the like of the active ingredient, wherein the weight of the active ingredient is in terms of a hydrochloride form (e.g., in terms of $C_{24}H_{29}N_9{\cdot}3HCl$ in some embodiments of the present application).
In the seventh aspect, the present application provides a method for preparing the pharmaceutical composition described above, the method comprising: (1) weighing: weighing an active ingredient and an excipient according to prescribed doses; and (2) mixing: mixing the active ingredient and the excipient evenly in a mixing vessel.
In some embodiments of the present application, the pharmaceutical composition is prepared into a capsule, the preparation method comprising: (1) weighing an active ingredient and an excipient according to prescribed doses; (2) adding the active ingredient and the excipient to a mixing vessel for mixing to produce a mixed powder; and (3) filling a capsule with the above mixed powder.
In some embodiments of the present application, the excipient of the pharmaceutical composition comprises an external lubricant; and the pharmaceutical composition is prepared into a capsule, the preparation method comprising: (1) weighing an active ingredient and an excipient according to prescribed doses; (2) adding the active ingredient and the excipient other than the external lubricant to a mixing vessel for mixing to produce a mixed powder; (3) granulating the above mixed powder; (4) mixing the granules obtained in step (3) with the external lubricant; and (5) filling a capsule with the mixture obtained in step (4).
In some embodiments of the present application, the pharmaceutical composition is prepared into a tablet, the preparation method comprising: (1) weighing an active ingredient and an excipient according to prescribed doses; (2) adding the active ingredient and the excipient to a mixing vessel for mixing to produce a mixed powder; (3) granulating the above mixed powder; (4) mixing the granules obtained in step (3) with an external lubricant; and (5) tableting; and optionally further comprising a coating step.
In some embodiments of the present application, the pharmaceutical composition is prepared into a granule, the preparation method comprising: (1) weighing an active ingredient and an excipient according to prescribed doses;

(2) adding the excipient and the active ingredient to a mixing vessel for mixing to produce a premix; (3) adding water to prepare the premix into a soft material; (4) granulating and drying; and (5) granule sizing.

In some embodiments of the present application, the pharmaceutical composition is prepared into an oral liquid formulation, the preparation method comprising: (1) weighing an active ingredient and an excipient according to prescribed doses; (2) adding the excipient and the active ingredient to a mixing vessel and stirring evenly; (3) sterilizing; and (4) filling.

In some embodiments of the present application, there is provided a method for preparing the solid oral formulation described above, comprising: packing the pharmaceutical composition obtained by the above preparation method into a packaging bag according to the packaging dose to prepare a powder or a fine granule. The "mixing vessel" described above shall be construed in a broad sense to include not only flexible mixers (including, but not limited to, flexible bags such as clean plastic bags and ziplock bags), but also rigid mixers (including, but not limited to, mixers, hopper mixers and the like). A person skilled in the art may select a suitable mixer for corresponding premixing and total mixing operation depending on the batch size and weight.

The "granulating" step described above shall be construed in a broad sense to include not only the granulation using a mortar (optionally further assisted with the sieving operation for sizing or assisted with the sieving operation to enable the prepared granules to meet the requirements), but also the granulating operation using a mechanical equipment such as a dry granulator. A person skilled in the art may select a suitable tool or mechanical equipment for corresponding granulating operations depending on the batch size and weight.

In the eighth aspect, the present application provides use of the pharmaceutical composition described above or the solid oral formulation described above for inhibiting the activity of one or more kinases selected from the group consisting of FLT3, EGFR, Abl, Fyn, Hck, Lck, Lyn, Ret, Yes, VEGFR2, ALK, BTK, c-KIT, c-SRC, FGFR1, KDR, MET, and PDGFRα.

In the ninth aspect, the present application provides use of the pharmaceutical composition described above or the oral formulation described above in the preparation of a medicament.

In some embodiments of the present application, the medicament is used for treating or preventing a protein kinase-mediated disease, and the protein kinase is one or more kinases selected from the group consisting of FLT3, EGFR, Abl, Fyn, Hck, Lck, Lyn, Ret, Yes, VEGFR2, ALK, BTK, c-KIT, c-SRC, FGFR1, KDR, MET, and PDGFRα.

In some embodiments of the present application, the protein kinase-mediated disease is a tumor disease, preferably a hematological tumor or a solid tumor, further preferably leukemia or lung cancer, more preferably acute myeloid leukemia such as FLT3-mutation-positive acute myeloid leukemia (further, for example, FLT3-ITD mutated or FLT3-TKD mutated acute myeloid leukemia), chronic myeloid leukemia (such as Ph-positive chronic myeloid leukemia), or non-small cell lung cancer (such as non-small cell lung cancer with EGFR activating mutation).

In some embodiments of the present application, the medicament is used for treating or preventing acute myeloid leukemia; preferably, the acute myeloid leukemia is relapsed and/or refractory acute myeloid leukemia, or the acute myeloid leukemia is selected from the group consisting of FLT3-ITD mutated and/or TKD mutated acute myeloid leukemia, relapsed and/or refractory acute myeloid leukemia with treatment failure with a Type II FLT3 inhibitor (such as Sorafenib), and DEK-CAN positive acute myeloid leukemia with FLT3-ITD mutation; more preferably, the acute myeloid leukemia is acute myeloid leukemia with FLT3-ITD high mutation; and/or the number of the unfavorable prognostic factors of the acute myeloid leukemia is 0 to 2; and/or the FAB classification of the acute myeloid leukemia is type M2, M4, or M5, preferably type M5. Furthermore, the aforesaid use in the preparation of a medicament for treating or preventing acute myeloid leukemia is described in detail in the Patent Application No. PCT/CN2020/127449, and the disclosure of this Patent Application No. PCT/CN2020/127449 is incorporated herein as if it is disclosed in the present application.

In the tenth aspect, the present application provides use of the pharmaceutical composition described above or the oral formulation described above in the preparation of a medicament as a protein kinase inhibitor, and the protein kinase is one or more kinases selected from the group consisting of FLT3, EGFR, Abl, Fyn, Hck, Lck, Lyn, Ret, Yes, VEGFR2, ALK, BTK, c-KIT, c-SRC, FGFR1, KDR, MET, and PDGFRα.

In some embodiments of the present application, the medicament as a protein kinase inhibitor is an anti-tumor medicament, and the tumor is preferably a solid tumor or a hematological tumor, further preferably leukemia or lung cancer, more preferably acute myeloid leukemia such as FLT3-mutation-positive acute myeloid leukemia (further, for example, FLT3-ITD mutated or FLT3-TKD mutated acute myeloid leukemia), chronic myeloid leukemia (such as Ph-positive chronic myeloid leukemia), or non-small cell lung cancer (such as non-small cell lung cancer with EGFR activating mutation).

In some embodiments of the present application, the medicament as a protein kinase inhibitor is an anti-tumor medicament, and the tumor is selected from the group consisting of non-small cell lung cancer, acute myeloid leukemia, chronic granulocytic leukemia, chronic myeloid leukemia, squamous cell carcinoma, breast cancer, colorectal cancer, liver cancer, gastric cancer, and malignant melanoma; further preferably, the disorder is selected from the group consisting of human non-small cell lung cancer, human acute myeloid leukemia, human chronic granulocytic leukemia, human

chronic myeloid leukemia, human squamous cell carcinoma, human breast cancer, human colorectal cancer, human liver cancer, human gastric cancer, and human malignant melanoma.

In the eleventh aspect, the present application provides use of the pharmaceutical composition described above or the oral formulation described above in the preparation of a medicament for treating or preventing a disorder; preferably, the disorder is a disorder caused by one or more than one kinase selected from the group consisting of FLT3, EGFR, Abl, Fyn, Hck, Lck, Lyn, Ret, Yes, VEGFR2, ALK, BTK, c-KIT, c-SRC, FGFR1, KDR, MET, and PDGFR$\alpha$; more preferably, the disorder is selected from the group consisting of non-small cell lung cancer, acute myeloid leukemia, chronic granulocytic leukemia, chronic myeloid leukemia, squamous cell carcinoma, breast cancer, colorectal cancer, liver cancer, gastric cancer, and malignant melanoma; further preferably, the disorder is selected from the group consisting of human non-small cell lung cancer, human acute myeloid leukemia, human chronic granulocytic leukemia, human chronic myeloid leukemia, human squamous cell carcinoma, human breast cancer, human colorectal cancer, human liver cancer, human gastric cancer, and human malignant melanoma.

In the twelfth aspect, the present application provides the above-mentioned pharmaceutical composition or the above-mentioned oral formulation for use in the treatment of a protein kinase-associated disease, wherein the protein kinase is one or more kinases selected from the group consisting of FLT3, EGFR, Abl, Fyn, Hck, Lck, Lyn, Ret, Yes, VEGFR2, ALK, BTK, c-KIT, c-SRC, FGFR1, KDR, MET, and PDGFR$\alpha$.

In some embodiments of the present application, the protein kinase-associated disease is selected from the group consisting of non-small cell lung cancer, acute myeloid leukemia, chronic granulocytic leukemia, chronic myeloid leukemia, squamous cell carcinoma, breast cancer, colorectal cancer, liver cancer, gastric cancer, and malignant melanoma; further preferably, the disorder is selected from the group consisting of human non-small cell lung cancer, human acute myeloid leukemia, human chronic granulocytic leukemia, human chronic myeloid leukemia, human squamous cell carcinoma, human breast cancer, human colorectal cancer, human liver cancer, human gastric cancer, and human malignant melanoma.

In the thirteenth aspect, the present application provides a method for treating a protein kinase-mediated disease, comprising administering the pharmaceutical composition described above or the oral formulation described above to a subject in need thereof, and the protein kinase is one or more kinases selected from the group consisting of FLT3, EGFR, Abl, Fyn, Hck, Lck, Lyn, Ret, Yes, VEGFR2, ALK, BTK, c-KIT, c-SRC, FGFR1, KDR, MET, and PDGFR$\alpha$.

In some embodiments of the present application, the protein kinase-associated disease is selected from the group consisting of non-small cell lung cancer, acute myeloid leukemia, chronic granulocytic leukemia, chronic myeloid leukemia, squamous cell carcinoma, breast cancer, colorectal cancer, liver cancer, gastric cancer, and malignant melanoma; further preferably, the disorder is selected from the group consisting of human non-small cell lung cancer, human acute myeloid leukemia, human chronic granulocytic leukemia, human chronic myeloid leukemia, human squamous cell carcinoma, human breast cancer, human colorectal cancer, human liver cancer, human gastric cancer, and human malignant melanoma.

When the medicament, oral formulation, oral solid formulation or oral solution according to all aspects described above exerts a therapeutic effect, the therapeutically effective amount of the active ingredient is 0.001 to 1000 mg, preferably 1 to 800 mg, or 1 to 500 mg, or 20 to 400 mg, or 100 to 350 mg, most preferably 150 to 310 mg, and the therapeutically effective amount is calculated in terms of a hydrochloride form (*e.g.*, in terms of $C_{24}H_{29}N_9 \cdot 3HCl$ in some embodiments of the present application). Administration may be conducted in a single dose or in divided doses.

Unless otherwise indicated, all percentages are given in weight % of the total weight of the pharmaceutical composition and the oral formulation thereof.

The "oral formulation" according to the present application refers to a pharmaceutical formulation for oral administration.

The "oral solid formulation" according to the present application refers to a pharmaceutical formulation in a solid state for oral administration.

The "oral solution formulation" or "oral solution" according to the present application refers to a pharmaceutical formulation in a liquid state for oral administration.

In the embodiments of the present application, the subject of administration may be a human being or a non-human mammal, more preferably a human being.

In the embodiments of the present application, the terms "comprise", "include" and "contain" generally express an open meaning, which, however, also includes a close-ended case defined by "consisting of ..." within its scope. For example, "A pharmaceutical composition, comprising: a compound represented by Formula II, Formula A or Formula B as an active ingredient and an excipient" also includes the case of "A pharmaceutical composition consisting of a compound represented by Formula II, Formula A or Formula B as an active ingredient and an excipient".

Within the scope of the present application, various options for any one of the features may be combined with various options for other features to constitute many different embodiments. The present application is intended to include all possible embodiments composed of various options for all technical features.

Within the scope of the present application, it is possible to appropriately adjust the amount of the compound

represented by Formula I, Formula II, Formula A or Formula B or a pharmaceutically acceptable salt thereof in the prescription, or adjust its ratio (mass ratio) to the excipient, so as to obtain pharmaceutical compositions or oral formulations with different strengths or different drug weights, which also fall within the scope of the present application.

After meticulous researches and experiments, one or more of the following advantageous technical effects are achieved in the present application.

(1) The preparation method for the pharmaceutical composition is simple and convenient with smooth formulating process and without demanding requirements for both the instruments and equipments used and the operating process, and is more suitable for industrial production;
(2) after screening, some of the pharmaceutical compositions according to the present application have been found to be suitable for direct mixing or dry granulation, which may simplify the preparation process of the solid formulations;
(3) compared with other prescriptions, the oral formulations made from the pharmaceutical compositions described above satisfy the formulating criteria and requirements, for example, the oral solid formulations (such as capsules and tablets) have good dissolution, content uniformity, and stability; and (4) the pharmaceutical compositions and/or oral formulations, in particular oral solid formulations, also have excellent stability, and in the Stress testing, accelerated test and/or long-term test, the pharmaceutical compositions and/or oral formulations (particularly oral solid formulations, such as capsules and tablets, more particularly capsules) to be tested do not show significant changes in terms of the maximum single impurity, total impurity, content, crystal form, etc., and are therefore suitable for long-term storage.

## BRIEF DESCRIPTION OF THE DRAWINGS

Figure 1: an XRPD pattern of the compound represented by Formula B obtained in Preparation Example 2.
Figure 2: a Differential Scanning Calorimetry - Thermogravimetry (DSC-TGA) diagram of the compound represented by Formula B obtained in Preparation Example 2.

## DETAILED DESCRIPTION

### Determination methods:

### 1. Content (determination of the free base content of compound B)

Detection instrument: high performance liquid chromatography/Waters e2695-2489

Analysis method:

Octadecylsilane-bonded silica gel is used as a filler (the applicable pH range should be greater than 10.0), and 20 mmol/L of disodium hydrogen phosphate solution (the pH value is adjusted to 10.0 with sodium hydroxide)-acetonitrile (65:35) is used as a mobile phase; the detection wavelength is 287 nm, and the column temperature is 30°C. The number of theoretical plates should be not less than 3000.

Determination method: About 20 mg of a sample is taken, precisely weighed, and put in a 100-mL volumetric flask. A diluent (50% methanol/water) is added to dissolve and dilute the sample to the scale, and the mixture is shaken well. 10 μL of the resulting solution is precisely measured and injected into a liquid chromatograph, and the chromatogram is recorded. Additionally, an appropriate amount of the reference substance is taken and determined by the same method. The content is obtained by calculating the peak area according to the external standard method.

### 2. Moisture (determination of the moisture content of compound B)

Detection instrument: Karl Fischer moisture titrator/915 KF Ti-Touch

Test method: After the instrument is balanced, an appropriate amount (about 200 mg) of a test sample is taken, precisely weighed, and added to a titration cup with absolute methanol as a solvent. The test sample is tested directly using a moisture titration solution. Each test sample is tested twice and the test results are averaged.

**3. Solubility**

Detection instrument: ultraviolet spectrophotometer/Evolution 300

Test method:

Solutions with pH 1.2, pH 4.5, and pH 6.8 and water are used as solvents, and the preparation methods of the solvents are specifically as follows.

(1) pH 1.2 hydrochloric acid solution: 1000 ml of water is added to 7.65 ml of hydrochloric acid, and the mixture is shaken uniformly to obtain the target solution.
(2) pH 4.5 phosphate buffer solution: 6.8 g of potassium dihydrogen phosphate is taken and diluted with water to 1000 ml, and the mixture is shaken uniformly to obtain the target solution.
(3) pH 6.8 phosphate buffer solution: 6.8 g of potassium dihydrogen phosphate and 0.896 g of sodium hydroxide are taken and diluted with water to 1000 ml, and the mixture is shaken uniformly to obtain the target solution.
(4) Water: purified water.

Sample preparation:

Test tubes with stopper are taken, and 10 ml of dissolution media at various pH values are precisely added to the test tubes, respectively. Excessive active pharmaceutical ingredients are added until supersaturated solutions are formed, and the addition amounts are recorded. The solutions are shaken uniformly, sealed with stoppers, and shaken for 24 hours in a shaker. 2 ml of solutions are taken out at different time points respectively, and centrifuged. The supernatants are taken, filtered, and the subsequent filtrates are taken for later use.

The above-mentioned saturated solutions in different solvents are taken, and diluted to certain volumes by adding solvents. The absorbance is measured at a wavelength of 287 nm.

Preparation of the solution of the reference substance: an appropriate amount of the reference substance of the compound represented by Formula 1 is taken and precisely weighed. A solvent is added to dissolve and dilute the reference substance to produce a solution containing about 10 μg of the compound represented by Formula 1 per 1 ml. The absorbance is measured at a wavelength of 287 nm to calculate the solubility.

**4. Hygroscopicity**

Detection instrument: XPE105DR

Test method:

(1) A dry glass weighing bottle with a stopper is taken, placed in a suitable constant-temperature desiccator at 25°C±1°C (with a saturated solution of ammonium chloride or ammonium sulfate placed in the lower part) or an artificial climate box (with the set temperature of 25°C±1°C and the relative humidity of 80%±2%) on the day before the test, and precisely weighed and recorded as the weight (m1).
(2) An appropriate amount of the test sample is taken and spread in the above-mentioned weighing bottle. The thickness of the test sample is generally about 1 mm, and the bottle is precisely weighed and recorded as the weight (m2).
(3) The stopper is removed to open the weighing bottle, and the opened weighing bottle and the stopper are placed under the above conditions with constant temperature and humidity for 24 hours.
(4) The opened weighing bottle is stoppered, precisely weighed and recorded as the weight (m3).

$$\text{Percentage of weight gain} = (m3-m2)/(m2-m1) \times 100\%.$$

(5) Description of hygroscopicity characteristics and definition for the weight gain due to hygroscopicity:

Deliquescence: sufficient amount of water is absorbed to form a liquid.
Highly hygroscopic: the weight gain due to hygroscopicity is not less than 15%.
Hygroscopic: the weight gain due to hygroscopicity is less than 15% but not less than 2%.

Slightly hygroscopic: the weight gain due to hygroscopicity is less than 2% but not less than 0.2%.

Not or nearly not hygroscopic: the weight gain due to hygroscopicity is less than 0.2%.

**5. X-Ray Powder Diffraction (XRPD)**

Detection instrument: PANalytical Empyrean powder X-ray diffractometer

Test conditions:

type of light tube: Cu target, metal-ceramic X-ray tube;
X-ray wavelength: CuKα, $K\alpha_1$(Å): 1.540598, $K\alpha_2$(Å): 1.544426, $K\alpha_2/K\alpha_1$ intensity ratio: 0.5;
voltage and current: 45 kV, 40 mA;
scanning range: 3-40°2θ;
total scanning time: about 5 min.

**6. Differential Scanning Calorimetry - Thermogravimetry (DSC-TGA)**

Detection instrument: NETZSCH STA 449F3

Test conditions:

temperature range: 20°C to 350°C;
heating rate: 10.0 (K/min);
sample holder/thermocouple: DSC/TG Cp S/S
crucible: DSC/TG pan $Al_2O_3$
atmosphere: $N_2$, 20.0 ml/min/$N_2$, 50.0 ml/min
calibration/measurement range: 020/5000 μV

**7. Nuclear Magnetic Resonance spectroscopy (NMRS)**

Detection instrument: AVIII BRUKER 600 superconducting nuclear magnetic resonance spectrometer
Contents and test solvent: $^1$H-NMR, $H_2D$ as the test solvent.

**8. Single crystal**

Single crystal diffraction data are collected at a temperature of 120.00(10)K using a Rigaka XtaLAB Synergy-R (Micro-Max007HF Cu mode, CuKαλ=1.54184 Å, Hypix 6000 HE detector) single crystal diffractometer. The micrograph of the single crystal sample is taken by a Shanghai CEWEI PXS9-T stereoscopic microscope.

**9. Dissolution**

Detection method: Dissolution and Release Assay - Approach 2 (sinker) (Chinese Pharmacopoeia 2020 Edition, Volume IV: General Principles 0931)

Six tablets of this product are taken and detected in accordance with Approach 2 of the Dissolution and Release Assay. 900 mL of the pH 1.2 hydrochloric acid solution is used as a dissolution medium. The rotation speed is 50 rpm. The operations are conducted according to the above method. After 30 min, 10 mL of the solution is taken and filtered. The subsequent filtrate is taken, and diluted with the pH 1.2 hydrochloric acid solution to a solution equivalent to the solution having an API content of 10 μg/mL as a test sample solution. Additionally, about 25 mg of the reference substance of compound B is taken, precisely weighed, and put in a 100-mL volumetric flask. The pH 1.2 hydrochloric acid solution is added to dissolve and dilute the reference substance of compound B to the scale. The resultant is shaken uniformly and used as a stock solution of the reference substance. The stock solution of the reference substance is measured

precisely and diluted to a solution (10 μg/mL) as a reference substance solution.

Determination method: Appropriate amounts of the reference substance solution and the test sample solution are measured, respectively. The absorbance is measured at a wavelength of 287 nm by ultraviolet-visible spectrophotometry. The dissolution of each tablet of the product is calculated by absorbance according to the external standard method.

**10. API-excipient compatibility test**

**Test Conditions and Time of API-Excinient Compatibility Test**

| Dosage Form | Test Conditions | Test Time |
|---|---|---|
| Capsules | High temperature 60°C;<br>High humidity 75% (25°C);<br>High humidity 92.5% (25°C);<br>Illumination (45001x±5001x) | 0 day, 7 days, 14 days, 30 days |
| Tablets, granules | High temperature 60°C;<br>High humidity 92.5% (25°C);<br>Illumination (45001x±5001x) | 0 day, 7 days, 14 days, 28 days |

**11. Stress testing**

**Test Conditions and Time of Stress testing**

| Dosage Form | Test Conditions | Test Time |
|---|---|---|
| Capsules | High temperature 60°C;<br>High humidity 75% (25°C);<br>High humidity 92.5% (25°C);<br>Illumination (45001x±5001x) | 0 day, 7 days, 14 days, 30 days |
| Tablets, granules | High temperature 60°C;<br>High humidity 92.5% (25°C);<br>Illumination (45001x±5001x) | 0 day, 5 days, 10 days |

**12. Accelerated and long-term stability tests**

**Test Conditions and Time of Stability Test**

| Dosage Form | Test Conditions | Test Time |
|---|---|---|
| Capsules | Accelerated test 40°C±2°C, relative humidity 75%±5% | 0 day, 1 month, 3 months, 6 months |
| | Long-term test 25°C±2°C, relative humidity 60 %±5% | 0 day, 3 months, 6 months, 9 months, 12 months, 18 months, 24 months, 36 months, 48 months |
| Tablets, granules | Accelerated test 40°C±2°C, relative humidity 75%±5% | 0 day, 1 month, 3 months, 6 months |

The present application is described in detail below by means of some examples, but the present application

is not limited thereto. Modifications that are obvious to a person skilled in the art are intended to fall within the scope of the claims of the present application.

Where the specific conditions are not indicated in the examples, conventional conditions or the conditions recommended by the manufacturers shall be followed. Reagents or instruments used, where the manufacturers are not specified, are all conventional products that are commercially available. Unless otherwise defined, all technical and scientific terms used herein have the same meanings as those familiar to a person skilled in the art.

Compound I of the present application may be prepared by a plurality of synthetic methods well known to a person skilled in the art, including the specific embodiments listed below, embodiments resulting from their combination with other chemical synthesis methods, and equivalent alternatives obvious to a person skilled in the art. Preferred embodiments include, but are not limited to, the preparation examples of the present application.

In order to obtain the compounds of the present application, it is sometimes necessary for a person skilled in the art to modify or select the synthetic steps or reaction processes on the basis of the existing embodiments.

**Preparation Example 1: Preparation of compound of Formula I**

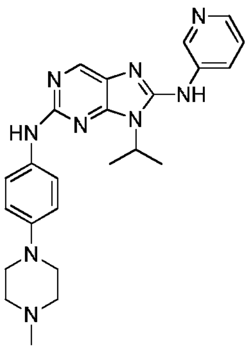

(I)

Referring to the method described in Example 90 of the Patent Literature WO 2011/147066, 100 g of the compound represented by Formula I was prepared.

**Preparation Example 2: Preparation of compound B**

The arylamino purine derivative (90 g, 0.203 mol) obtained in Preparation Example 1 as well as 800 mL of purified water and 400 mL of acetone were added into a reactor and heated to 40±5°C under stirring, and then concentrated hydrochloric acid (74 g, 0.731 mol) was slowly added into the reactor. After completing the addition of the concentrated hydrochloric acid, 2 L of acetone was further added. While the temperature was kept at 40±5°C, the reaction was continued for 1 h. Thereafter, the reaction mixture was cooled to 10±5°C under stirring, crystallized for 2 h, and subjected to suction filtration. The filter cake was washed with 300 mL of acetone to obtain 74.7 g of hydrochloride, which was yellow or pale yellow. $^1$H-NMR(600MHz, $D_2O$) δ: 1.556(d, 6H), 2.896(s, 3H), 3.058(t, 2H), 3.187(t, 2H), 3.586(d, 2H), 3.749(d, 2H), 4.701(s, 1H), 7.062(d, 2H), 7.377(d, 2H), 7.968(t, 1H), 8.086(s, 1H), 8.431(d, 1H), 8.636(d, 1H), 9.171(s, 1H). The resulting hydrochloride showed good crystallinity, and its XRPD characteristic pattern was shown in Figure 1. The main diffraction peak data were as follows:

| Peak Position 2θ Angle (°) | Relative Peak Intensity % | Peak Position 2θ Angle (°) | Relative Peak Intensity % |
|---|---|---|---|
| 7.300 | 20.96 | 20.027 | 26.40 |
| 8.504 | 40.4 | 21.140 | 22.06 |
| 9.052 | 14.65 | 21.913 | 14.4 |
| 11.814 | 34.65 | 23.701 | 25.54 |
| 12.579 | 13.44 | 25.162 | 62.26 |
| 14.300 | 15.86 | 26.137 | 15.54 |
| 18.136 | 18.09 | 27.165 | 100 |

(continued)

| Peak Position 2θ Angle (°) | Relative Peak Intensity % | Peak Position 2θ Angle (°) | Relative Peak Intensity % |
|---|---|---|---|
| 19.641 | 29.87 | | |

A sample was taken for the DSC and TGA tests, and the spectra were shown in Figure 2, respectively. The DSC spectrum showed that there was no obvious melting point, and the TGA spectrum showed that the sample started to lose water of crystallization at about 40°C and started to lose acid at about 140°C.

From the calculation of the free base content by HPLC and the determination of the water content (see the table below), it could be inferred that the base/acid/$H_2O$ ratio of this hydrochloride was 1:3:5.

| Name | Theoretical stoichiometric ratio (base/ acid/$H_2O$) | Theoretical water content (%) | Theoretical acid content (%) | Theoretical base content (%) | Measured water content (%) | Measured base content (%, HPLC) |
|---|---|---|---|---|---|---|
| Preparation Example 2 Hydrochloride | 1:3:5 | 14.0% | 17.0% | 69.0% | 13.9% | 69.0% |

The sample obtained in Preparation Example 2 was cultured to obtain a single crystal (see the method described in PCT/CN2021/073285). The X-ray diffraction characteristic results of the single crystal showed that this crystal was a triclinic system, had a space group $P\overline{1}$, and had the following unit cell parameter: {a=7.04142(7)Å, b=12.15291(7)Å, c=18.13188(10)Å, $\alpha$=93.2215(5)°, $\beta$=95.3039(6)°, $\gamma$=91.9554(6)°, V=1541.32(2)$Å^3$}. The asymmetric unit of this crystal was composed of the cation of the compound represented by Formula 1, three chloride ions, and five water molecules. Moreover, after comparison, the XRPD pattern of this single crystal was found to be basically consistent with Figure 1.

**Example 1: Capsules of compound B**

Table 1 Prescription (100 capsules) of Example 1

| Name of Material | | Weight (g) | Weight Percentage (%) |
|---|---|---|---|
| API | Compound B | 5.815 | 36.34 |
| Filler | Pregelatinized starch | 9.385 | 58.66 |
| Glidant | Colloidal silicon dioxide | 0.480 | 3.00 |
| Lubricant | Magnesium stearate (internally added) | 0.160 | 1.00 |
| | Magnesium stearate (externally added) | 0.160 | 1.00 |
| Total | | 16.000 | 100.00 |

The dry granulation process was adopted with the specific method as follows. 1) Weighing or preparation of materials: the active ingredient (API) compound B and the corresponding filler, glidant, and lubricant (internally/externally added) were weighed according to the prescribed doses; 2) premixing 1: the active ingredient, filler, glidant, and lubricant (internally added) were added in sequence into a mixing vessel for premixing; 3) deagglomeration: the resulting mixed powder was subjected to deagglomeration; 4) premixing 2: the mixed powder obtained after the deagglomeration treatment was added into a mixing vessel, and premixed again; 5) dry granulation: the mixed powder that had been premixed again was tableted by a tablet press into a sheet-like substance, which was granulated after the completion of pressing; 6) total mixing: the granules obtained in step 5) were added into a mixing vessel, and a lubricant (externally added) was added for mixing; 7) capsule filling: capsule shells of a suitable model were selected for filling according to the filling capacity.

Table 2 Detection Results of Example 1

| Prescription | Phenomenon | Dissolution |
|---|---|---|
| | | 15 min |
| Example 1 | No sticking, tablet with uniform color and lustre, no delamination phenomenon in premix | 100.9% |

**Results:** Prior to granulation, the process of pressing into a large tablet was smooth without sticking, and the formulations were dissolved rapidly, which satisfied the formulation requirements. The prescription was qualified.

**Examples 2 to 5: Capsules of compound B**

With reference to the preparation method described in Example 1, the following pharmaceutical compositions could be obtained by adjusting the ratio of the glidant to the lubricant and adjusting the amounts of the API and pregelatinized starch (API:pregelatinized starch (w/w) = 1: 1.58) depending on the total amount. The specific prescriptions and detection results were listed in Table 3.

Table 3 Prescriptions and Detection Results of Examples 2 to 5

| Adjuvant | | Example 2 | Example 3 | Example 4 | Example 5 |
|---|---|---|---|---|---|
| Glidant | Colloidal silicon dioxide | 1% | 2% | 2.5% | 3% |
| Lubricant | Magnesium stearate (internally added) | 1.5% | 1.5% | 1.5% | 2% |
| Phenomenon when pressing | Sticking or not | No | No | No | No |
| | With or without | No | No | No | No |
| into a large tablet | even color distribution | | | | |
| State of mixed powder after premixing 2 | Agglomerating/delaminating or not | No | No | No | No |
| Notes: ① all of the weight percentages of the externally added magnesium stearate were 1%; ② the data in the table were weight percentages (the same applies to the contents below). | | | | | |

**Results:** Within the scope of the present application, none of the prescriptions obtained by adjusting the weight percentages of colloidal silicon dioxide and magnesium stearate (internally added) showed the phenomena such as sticking, uneven color distribution, agglomeration and/or delamination, and the process of pressing into a large tablet prior to granulation was smooth. That is, the tableting step met the requirements, and the formulations were dissolved rapidly, which satisfied the formulation requirements. All of the prescriptions were qualified.

**Examples 6 to 9: Capsules of compound B**

With reference to the preparation method described in Example 1, the following pharmaceutical compositions could be obtained by adjusting the amount of the filler. The specific prescriptions and detection results were listed in Table 4.

Table 4 Prescriptions and Detection Results of Examples 6 to 9

| Prescription | | Example 6 | Example 7 | Example 8 | Example 9 |
|---|---|---|---|---|---|
| API | Compound B | 33.34% | 27.00% | 29.00% | 31.00% |
| Filler | Pregelatinized starch | 61.66% | 68.00% | 66.00% | 64.00% |
| Glidant | Colloidal silicon dioxide | 3.00% | 3.00% | 3.00% | 3.00% |
| Lubricant | Magnesium stearate | 2.00% | 2.00% | 2.00% | 2.00% |
| Total | | 100% | 100% | 100% | 100% |
| Phenomenon | | Slight sticking or no sticking | | | |

(continued)

| Prescription | | Example 6 | Example 7 | Example 8 | Example 9 |
|---|---|---|---|---|---|
| Dissolution | 15 min | 99.0% | 102.0% | 101.2% | 101.9% |
| | 30 min | 99.0% | 102.6% | 101.4% | 101.9% |
| Notes: the weight percentages of magnesium stearate (internally added) and magnesium stearate (externally added) were both 1%; for each of the prescriptions, 100 capsules were prepared with a total weight of 16 g. | | | | | |

**Results:** On the basis of the prescription of Example 1, adjusting the amount of pregelatinized starch within the scope of the present application exerted no obvious influence on the phenomena related to tableting and the dissolution results. That is, the resulting prescriptions allowed for a smooth process of pressing into a large tablet prior to granulation with slight sticking or no sticking, and none of the prescriptions showed the phenomena such as uneven color distribution, agglomeration and/or delamination. That is, the tableting step met the requirements, and the formulations were dissolved rapidly, which satisfied the formulation requirements. All of the prescriptions were qualified.

**Example 10: Capsules of compound B**

With reference to the preparation method described in Example 1, the following pharmaceutical composition could be obtained by adding a disintegrant in step 6). The specific prescription and detection results were listed in Table 5.

Table 5 Prescription (100 capsules) and Detection Results of Example 10

| Name of Material | | Weight (g) | Weight Percentage (%) |
|---|---|---|---|
| API | Compound B | 5.815 | 36.34 |
| Filler | Pregelatinized starch | 9.225 | 57.66 |
| Glidant | Colloidal silicon dioxide | 0.480 | 3.00 |
| Lubricant | Magnesium stearate (internally added) | 0.160 | 1.00 |
| | Magnesium stearate (externally added) | 0.160 | 1.00 |
| Disintegrant | Sodium starch glycolate | 0.160 | 1.00 |
| Total | | 16.000 | 100.00 |
| Phenomenon | | Slight sticking | |
| Dissolution (15 min) | | 99.0% | |

**Results:** The prescription obtained by adding a disintegrant on the basis of Example 1 allowed for a smooth process of pressing into a large tablet prior to granulation with slight sticking or no sticking and without the phenomena such as uneven color distribution, agglomeration and/or delamination. That is, the tableting step met the requirements, and the formulations were dissolved rapidly, which satisfied the formulation requirements. The prescription was qualified. Namely, the effects were equivalent to those achieved in the absence of a disintegrant. Therefore, whether or not to add a disintegrant had little influence on the effects of the prescription with a single filler (pregelatinized starch).

**Examples 11 to 14: Capsules of compound B**

With reference to the prescription and preparation method described in Example 1, the following pharmaceutical compositions could be obtained using the pregelatinized starch and mannitol as a combined filler. The specific prescriptions and detection results were listed in Table 6.

Table 6 Prescriptions and Detection Results of Examples 11 to 14

| Prescription | | Example 11 | Example 12 | Example 13 | Example 14 |
|---|---|---|---|---|---|
| API | Compound B | 36.34% | 36.34% | 36.34% | 36.34% |
| Filler | Pregelatinized starch | 10.00% | 20.00% | 40.00% | 50.00% |

(continued)

| Prescription | | Example 11 | Example 12 | Example 13 | Example 14 |
|---|---|---|---|---|---|
| Filler | Mannitol | 48.66% | 38.66% | 18.66% | 8.66% |
| Glidant | Colloidal silicon dioxide | 3.00% | 3.00% | 3.00% | 3.00% |
| Lubricant | Magnesium stearate | 2.00% | 2.00% | 2.00% | 2.00% |
| Total | | 100% | 100% | 100% | 100% |
| Phenomenon | | Slight sticking or no sticking | | | |
| Dissolution | 15 min | 100.8% | 100.7% | 95.2% | 102.1% |
| | 30 min | 100.9% | 101.2% | 96.6% | 102.3% |
| Notes: the weight percentages of magnesium stearate (internally added) and magnesium stearate (externally added) were both 1%; for each of the prescriptions, 100 capsules were prepared with a total weight of 16 g. | | | | | |

**Results:** Using a combined filler composed of pregelatinized starch and mannitol and adjusting the weight ratio of these two components within the scope of the present application exerted no obvious influence on the phenomena related to tableting and the dissolution results. That is, the resulting prescriptions allowed for a smooth process of pressing into a large tablet prior to granulation with slight sticking or no sticking, and none of the prescriptions showed the phenomena such as uneven color distribution, agglomeration and/or delamination. That is, the tableting step met the requirements and satisfied the formulation requirements. All of the prescriptions were qualified. Moreover, the formulations could be dissolved rapidly in the absence of a disintegrant.

**Example 15: Capsules of compound B**

With reference to the preparation method described in Example 1, the following pharmaceutical composition could be obtained using microcrystalline cellulose and lactose as a combined filler. The specific prescription and detection results were listed in Table 7 and Table 8, respectively.

Table 7 Prescription (100 capsules) of Example 15

| Name of Material | | Weight (g) | Weight Percentage (%) |
|---|---|---|---|
| API | Compound B | 5.816 | 32.31 |
| Filler | Microcrystalline cellulose | 7.162 | 39.79 |
| Filler | Lactose | 3.582 | 19.90 |
| Disintegrant | Croscarmellose sodium | 0.540 | 3.0 |
| Glidant | Colloidal silicon dioxide | 0.540 | 3.0 |
| Lubricant | Magnesium stearate | 0.360 | 2.0 |
| Total | | 18.000 | 100.0 |
| Note: the weight percentages of magnesium stearate (internally added) and magnesium stearate (externally added) were both 1%. | | | |

Table 8 Detection Results of Example 15

| Prescription | Phenomenon | Dissolution | |
|---|---|---|---|
| Example 15 | No sticking, tablet with uniform color and lustre, no delamination phenomenon in premix | 15 min | 30 min |
| | | 92% | 95% |

**Results:** With the adoption of a combined filler of microcrystalline cellulose and lactose, the process of pressing into a large tablet prior to granulation was smooth without sticking and the phenomena such as uneven color distribution, agglomeration and/or delamination. That is, the tableting step met the requirements, and the formulations were dissolved

rapidly, which satisfied the formulation requirements. All of the prescriptions were qualified.

**Examples 16 and 17: Capsules of compound B**

With reference to the preparation method and filling capacity described in Example 15, microcrystalline cellulose was used as the single filler to prepare pharmaceutical compositions according to the prescription compositions and weight percentages listed in Table 9. The specific prescriptions and experimental results were listed in Table 9 below.

Table 9 Prescriptions and Detection Results of Examples 16 and 17

| Prescription | | | Example 16 | Example 17 |
|---|---|---|---|---|
| API | Compound B | | 32.31% | 32.31% |
| Filler | Microcrystalline cellulose | | 63.69% | 66.69% |
| Glidant | Colloidal silicon dioxide | | 3.00% | / |
| Lubricant | Magnesium stearate | | 1.00% | 1.00% |
| Total | | | 100% | 100% |
| Phenomenon | | | Slight sticking | Slight sticking |
| Dissolution | | 15 min | 83% | 82% |
| | | 30 min | 93% | 92% |
| Note: for each of the prescriptions, 100 capsules were prepared with a total weight of 18 g. | | | | |

**Results:** When microcrystalline cellulose was used as the single filler, the resulting prescription (Example 16) met the requirements in the granulation process in the case of not adding a disintegrant; moreover, even without the addition of a glidant (colloidal silicon dioxide), the resulting prescription (Example 17) still met the requirements in the granulation process. That is, the resulting prescriptions allowed for a smooth process of pressing into a large tablet prior to granulation with slight sticking or no sticking, and none of the prescriptions showed the phenomena such as uneven color distribution, agglomeration and/or delamination.

For the prescriptions of Example 16 and Example 17, the dissolution results showed that although the formulations were dissolved a little bit slowly at 15 min, the dissolution at 30 min satisfied the requirements. Formulations could be selected according to the medication requirements.

**Examples 18 and 19: Capsules of compound B**

With reference to the prescription composition and preparation method described in Example 15, the following pharmaceutical compositions (100 capsules) could be obtained by adjusting the weight ratio of the combined filler. The specific prescriptions and detection results were listed in Table 10.

Table 10 Prescriptions and Detection Results of Examples 18 and 19

| Prescription | | Example 18 | Example 19 |
|---|---|---|---|
| API | Compound B | 32.31% | 32.31% |
| Filler | Lactose | 29.84% | 39.79% |
| Filler | Microcrystalline cellulose | 29.85% | 19.90% |
| Disintegrant | Croscarmellose sodium | 3.00% | 3.00% |
| Glidant | Colloidal silicon dioxide | 3.00% | 3.00% |
| Lubricant | Magnesium stearate | 2.00% | 2.00% |
| Total | | 100% | 100% |
| Phenomenon | | No sticking | No sticking |

(continued)

| Prescription | | Example 18 | Example 19 |
|---|---|---|---|
| Dissolution | 15 min | 93% | 76% |
| | 30 min | 96% | 96% |
| Note: the weight percentages of magnesium stearate (internally added) and magnesium stearate (externally added) were both 1%; for each of the prescriptions, 100 capsules were prepared with a total weight of 18 g. | | | |

**Results:** The resulting prescriptions obtained by adjusting the ratio of the two fillers in the combined filler within the scope of the present application allowed for a smooth process of pressing into a large tablet prior to granulation without sticking and without the phenomena such as uneven color distribution, agglomeration and/or delamination. That is, the tableting step met the requirements and satisfied the formulation requirements. In case of the prescription using lactose/microcrystalline cellulose at a weight ratio of 1:1 (Example 18), the formulation was dissolved rapidly within 15 min; in case of the prescription using lactose/microcrystalline cellulose at a weight ratio of 2:1 (Example 19), the formulation was dissolved a little bit slowly within 15 min, but the dissolution within 30 min satisfied the requirements.

**Examples 20 and 21: Capsules of compound B**

With reference to the prescription composition and preparation method described in Example 15, the following pharmaceutical compositions (100 capsules) could be obtained by replacing the type of the disintegrant and adjusting the ratio of two fillers. The specific prescriptions and detection results were listed in Table 11.

Table 11 Prescriptions and Detection Results of Examples 20 and 21

| Prescription | | Example 20 | Example 21 |
|---|---|---|---|
| API | Compound B | 32.31% | 32.31% |
| Filler | Lactose | 19.90% | 39.79% |
| Filler | Microcrystalline cellulose | 39.79% | 19.90% |
| Disintegrant | Sodium starch glycolate | 3.00% | 3.00% |
| Glidant | Colloidal silicon dioxide | 3.00% | 3.00% |
| Lubricant | Magnesium stearate | 2.00% | 2.00% |
| Total | | 100% | 100% |
| Phenomenon | | No sticking | No sticking |
| Dissolution | 15 min | 93% | 92% |
| | 30 min | 95% | 95% |
| Note: the weight percentages of magnesium stearate (internally added) and magnesium stearate (externally added) were both 1%; for each of the prescriptions, 100 capsules were prepared with a total weight of 18 g. | | | |

**Results:** When sodium starch glycolate was used as a disintegrant, the resulting prescriptions allowed for a smooth process of pressing into a large tablet prior to granulation without sticking, and none of the prescriptions showed the phenomena such as uneven color distribution, agglomeration and/or delamination. That is, the tableting step met the requirements, and all of the resulting prescriptions exhibited good effect in terms of dissolution and satisfied the formulation requirements. All of the prescriptions were qualified. Therefore, in case of satisfying the API-excipient compatibility, the disintegrant might be selected and replaced according to the medication requirements.

**Examples 22 and 23: Capsules of compound B**

With reference to the preparation method described in Example 15 and the prescription composition of Example 20, the following pharmaceutical compositions (100 capsules) could be obtained by using sodium starch glycolate as a disintegrant and adjusting its amount. The specific prescription compositions and detection results were listed in Table 12.

Table 12 Prescriptions and Detection Results of Examples 22 and 23

| Prescription | | Example 22 | Example 23 |
|---|---|---|---|
| API | Compound B | 32.31% | 32.31% |
| Filler | Lactose | 19.90% | 19.90% |
| Filler | Microcrystalline cellulose | 40.79% | 41.79% |
| Disintegrant | Sodium starch glycolate | 2.00% | 1.00% |
| Glidant | Colloidal silicon dioxide | 3.00% | 3.00% |
| Lubricant | Magnesium stearate | 2.00% | 2.00% |
| Total | | 100% | 100% |
| Phenomenon | | No sticking | No sticking |
| Dissolution | 15 min | 93% | 93% |
| | 30 min | 96% | 97% |
| Note: the weight percentages of magnesium stearate (internally added) and magnesium stearate (externally added) were both 1%; for each of the prescriptions, 100 capsules were prepared with a total weight of 18 g. | | | |

**Results:** The resulting prescriptions obtained by adjusting the amount of the disintegrant allowed for a smooth process of pressing into a large tablet prior to granulation without sticking, and none of the prescriptions showed the phenomena such as uneven color distribution, agglomeration and/or delamination. That is, the tableting step met the requirements, and all of the prescriptions exhibited good effect in terms of dissolution and satisfied the formulation requirements. All of the prescriptions were qualified.

**Example 24: Capsules of compound B**

With reference to the preparation method described in Example 15 and the prescription composition of Example 20, the following composition (100 capsules) could be obtained by replacing the type of the lubricant. The specific prescription and detection results were listed in Table 13.

Table 13 Prescription and Detection Results of Example 24

| Prescription | | Example 24 |
|---|---|---|
| API | Compound B | 32.31% |
| Filler | Lactose | 19.90% |
| Filler | Microcrystalline cellulose | 39.79% |
| Disintegrant | Sodium starch glycolate | 3.00% |
| Glidant | Colloidal silicon dioxide | 3.00% |
| Lubricant | Sodium stearyl fumarate | 2.00% |
| Filling capacity/mg/capsule | | 180 |
| Phenomenon | | No sticking |
| Dissolution | 15 min | 92% |
| | 30 min | 94% |
| **Note:** the weight percentages of sodium stearyl fumarate (internally added) and magnesium stearate (externally added) were both 1%. | | |

**Results:** Compared with Example 20, the change in lubricant had no significant influence on the granulation process (without sticking and without the phenomena such as uneven color distribution, agglomeration and/or delamination) and the dissolution results, and magnesium stearate was more preferred in terms of price.

**Example 25: Capsules of compound B**

With reference to the prescription and preparation method described in Example 1, the following pharmaceutical composition (100 capsules) could be obtained by replacing the filler. The specific prescription and detection results were listed in Table 14.

Table 14 Prescription and Detection Results of Example 25

| Prescription | | Example 25 |
|---|---|---|
| API | Compound B | 58.15% |
| Filler | Anhydrous dibasic calcium phosphate | 37.85% |
| Glidant | Colloidal silicon dioxide | 2.00% |
| Lubricant | Magnesium stearate | 2.00% |
| Filling capacity/mg/capsule | | 100 |
| Phenomenon | | No sticking |
| Dissolution | 15min | 98.6% |
| | 30min | 99.8% |
| Note: the weight percentages of magnesium stearate (internally added) and magnesium stearate (externally added) were both 1%; 100 capsules were prepared according to the prescription with a total weight of 10 g. | | |

**Results:** Compared with Example 1, the replacement of the filler with a calcium salt (such as anhydrous dibasic calcium phosphate) had no influence on the granulation process (without sticking and without the phenomena such as uneven color distribution, agglomeration and/or delamination) and the dissolution results, and the prescription was qualified.

**Examples 26 to 29: Capsules of compound B**

With reference to the preparation method described in Example 1 and the prescription composition of Example 25, the following pharmaceutical compositions could be obtained by adjusting the proportion of each component or further adding a disintegrant. The specific prescriptions and detection results were listed in the table.

Table 15 Prescriptions and Detection Results of Examples 26 to 29

| Prescription | | Example 26 | Example 27 | Example 28 | Example 29 |
|---|---|---|---|---|---|
| API | Compound B | 77.5% | 58.15% | 14.5% | 14.5% |
| Filler | Anhydrous dibasic calcium phosphate | 18.5% | 38.85% | 81.5% | 79.5% |
| Disintegrant | Sodium starch glycolate | / | / | / | 2.00% |
| Glidant | Colloidal silicon dioxide | 2.00% | 1.00% | 2.00% | 2.00% |
| Lubricant | Magnesium stearate | 2.00% | 2.00% | 2.00% | 2.00% |
| Filling capacity/mg/capsule | | 75 | 100 | 400 | 400 |
| Phenomenon | | No sticking | Slight sticking | No sticking | No sticking |
| Dissolution | 15 min | / | / | 64.4% | 79.9% |
| | 30 min | / | / | 80.5% | 95.0% |
| Note: the weight percentages of magnesium stearate (internally added) and magnesium stearate (externally added) were both 1%; 100 capsules were prepared for each of the prescriptions and the total weight was calculated according to the filling capacity of each capsule; "/" indicated "not tested". | | | | | |

**Results:** Compared with Example 25, the adjustments to the proportion of each component or further addition of a disintegrant had no influence on the granulation process (with slight sticking or no sticking and without the phenomena

such as uneven color distribution, agglomeration and/or delamination). Although the dissolution at 15 min was relatively low, the dissolution at 30 min could reach 80% or more. Formulations could be selected as actually required. All of the prescriptions were qualified.

**Examples 30 to 32: Capsules of compound B**

With reference to the preparation method described in Example 1 or Example 15 and the prescription composition of Example 25, the following pharmaceutical compositions could be obtained by replacing a single filler with a combination of two fillers. The specific prescriptions and detection results were listed in Table 16.

Table 16 Prescriptions and Detection Results of Examples 30 to 32

| Prescription | | Example 30 | Example 31 | Example 32 |
|---|---|---|---|---|
| API | Compound B | 77.6% | 29.08% | 19.38% |
| Filler | Anhydrous dibasic calcium phosphate | 9.2% | 10.00% | 15.00% |
| Filler | Pregelatinized starch | 9.2% | 55.92% | 59.12% |
| Disintegrant | Sodium starch glycolate | / | 1.00% | 2.00% |
| Glidant | Colloidal silicon dioxide | 2.00% | 2.00% | 3.00% |
| Lubricant | Magnesium stearate | 2.00% | 2.00% | 1.50% |
| Filling capacity/mg/capsule | | 75 | 200 | 300 |
| Phenomenon | | No sticking | No sticking | No sticking |
| Dissolution | 15 min | 100.4% | 99.6% | 98.9% |
| | 30 min | 101.0% | 99.7% | 99.1 % |
| Note: in Examples 30 and 31, the weight percentages of magnesium stearate (internally added) and magnesium stearate (externally added) were both 1%; in Example 32, the weight percentages of magnesium stearate (internally added) and magnesium stearate (externally added) were 0.5% and 1%, respectively; 100 capsules were prepared for each of the prescriptions and the total weight was calculated according to the filling capacity of each capsule. | | | | |

**Results:** Compared with Example 25, use of a combination of two fillers (e.g., a combination of anhydrous dibasic calcium phosphate and pregelatinized starch) or further adjustments to the proportion of each component had no influence on both the granulation process (without sticking and without the phenomena such as uneven color distribution, agglomeration and/or delamination) and the dissolution results. All of the prescriptions were qualified.

**Examples 33 and 34: Capsules of compound B**

With reference to the prescription and preparation method described in Example 1, Example 15 or Example 30, the following pharmaceutical compositions could be obtained by replacing the filler, or further adding a disintegrant and/or replacing the disintegrant, or further adding a binder. The specific prescriptions and detection results were listed in Table 17.

Table 17 Prescriptions and Detection Results of Examples 33 and 34

| Prescription | | Example 33 | Example 34 |
|---|---|---|---|
| API | Compound B | 77.50% | 14.50% |
| Filler | Calcium carbonate | 18.00% | 74.50% |
| Disintegrant | Croscarmellose sodium | 0.50% | / |
| Disintegrant | Sodium starch glycolate | / | 4.00% |
| Binder | Copovidone VA64 | / | 3.00% |
| Glidant | Colloidal silicon dioxide | 2.00% | 2.00% |
| Lubricant | Magnesium stearate | 2.00% | 2.00% |

(continued)

| Prescription | | Example 33 | Example 34 |
|---|---|---|---|
| Filling capacity/mg/capsule | | 75 | 100 |
| Phenomenon | | No sticking | No sticking |
| Dissolution | 15 min | 91.0% | 98.4% |
| | 30 min | 101.6% | 98.5% |
| Note: the weight percentages of magnesium stearate (internally added) and magnesium stearate (externally added) were both 1%; 100 capsules were prepared for each of the prescriptions and the total weight was calculated according to the filling capacity of each capsule. | | | |

**Results:** The above adjustments to the composition and proportion of the prescription had no influence on both the granulation process (without sticking and without the phenomena such as uneven color distribution, agglomeration and/or delamination) and the dissolution results. All of the prescriptions were qualified.

**Examples 35 to 38: Capsules of compound B**

With reference to the prescription and preparation method described in Example 1, Example 15 or Example 30, the following pharmaceutical compositions could be obtained by adding a second filler, or further adding a disintegrant and/or a binder. The specific prescriptions and detection results were listed in Table 18.

Table 18 Prescriptions and Detection Results of Examples 35 to 38

| Prescription | | Example 35 | Example 36 | Example 37 | Example 38 |
|---|---|---|---|---|---|
| API | Compound B | 77.6% | 14.5% | 29.08% | 19.38% |
| Filler | Calcium carbonate | 9.2% | 39.5% | 20.00% | 15.00% |
| Filler | Pregelatinized starch | 9.2% | 39.5% | 45.92% | 59.62% |
| Disintegrant | Croscarmellose sodium | / | 0.5% | 1.00% | 1.00% |
| Binder | Copovidone VA64 | / | 2% | / | / |
| Glidant | Colloidal silicon dioxide | 2% | 2% | 2.00% | 3.00% |
| Lubricant | Magnesium stearate | 2% | 2% | 2.00% | 2.00% |
| Filling capacity/mg/capsule | | 75 | 400 | 200 | 300 |
| Phenomenon | | No sticking | No sticking | No sticking | No sticking |
| Dissolution | 15 min | 99.3% | 100.7% | 99.6% | 100.1% |
| | 30 min | 99.5% | 102.4% | 99.5% | 100.1% |
| Note: the weight percentages of magnesium stearate (internally added) and magnesium stearate (externally added) were both 1%; 100 capsules were prepared for each of the prescriptions and the total weight was calculated according to the filling capacity of each capsule. | | | | | |

**Results:** The above adjustments to the composition and proportion of the prescription had no influence on both the granulation process (without sticking and without the phenomena such as uneven color distribution, agglomeration and/or delamination) and the dissolution results. All of the prescriptions were qualified.

**Example 39: Capsules of compound B**

According to the above qualified prescriptions of the pharmaceutical compositions, formulations containing active ingredients with different strengths could be prepared. Exemplary prescriptions (100 capsules) included:

Table 19 Prescription (Single Filler) of Example 39

| Prescription | 39-1 | | 39-2 | | 39-3 | |
|---|---|---|---|---|---|---|
| | Weight (g) | Weight Percentage | Weight (g) | Weight Percentage | Weight (g) | Weight Percentage |
| Active ingredient | 2.908 | 29.08% | 5.815 | 36.34% | 11.630 | 36.34% |
| Pregelatinized starch | 6.592 | 65.92% | 9.385 | 58.66% | 18.770 | 58.66% |
| Colloidal silicon dioxide | 0.300 | 3.00% | 0.480 | 3.00% | 0.960 | 3.00% |
| Magnesium stearate (internally added) | 0.100 | 1.00% | 0.160 | 1.00% | 0.320 | 1.00% |
| Magnesium stearate (externally added) | 0.100 | 1.00% | 0.160 | 1.00% | 0.320 | 1.00% |
| Total: | 10.0 | 100.0% | 16.0 | 100.0% | 32.0 | 100.0% |
| Vacant gelatin capsule | 3# | | 3# | | 1# | |
| Note: with reference to the prescription and preparation method described in Example 1, the proportion of the filler and the vacant gelatin capsule could be adjusted appropriately according to the desired strength and filling capacity; the prescription of Example 39-2 was the same as that of Example 1. | | | | | | |

Table 20 Prescription Combined Filler of Example 39

| Prescription | 39-4 | | 39-5 | | 39-6 | |
|---|---|---|---|---|---|---|
| | Weight (g) | Weight Percentage | Weight (g) | Weight Percentage | Weight (g) | Weight Percentage |
| Active ingredient | 2.908 | 29.08% | 5.815 | 36.34% | 11.630 | 36.34% |
| Mannitol | 5.468 | 54.68% | 7.785 | 48.66% | 15.570 | 48.66% |
| Pregelatinized starch | 1.124 | 11.24% | 1.600 | 10.00% | 3.200 | 10.00% |
| Colloidal silicon dioxide | 0.300 | 3.00% | 0.480 | 3.00% | 0.960 | 3.00% |
| Magnesium stearate (internally added) | 0.100 | 1.00% | 0.160 | 1.00% | 0.320 | 1.00% |
| Magnesium stearate (externally added) | 0.100 | 1.00% | 0.160 | 1.00% | 0.320 | 1.00% |
| Total: | 10.0 | 100.0% | 16.0 | 100.0% | 32.0 | 100.0% |
| Vacant gelatin capsule | 3# | | 3# | | 1# | |
| Note: with reference to the prescription of Example 11 and the preparation method described in Example 1, the ratio of the fillers and the vacant gelatin capsule could be adjusted appropriately according to the desired strength and filling capacity; the prescription of Example 39-5 was the same as that of Example 11. | | | | | | |

Table 21 Prescription (Combined Filler of Example 39

| Prescription | 39-7 | | 39-8 | | 39-9 | |
|---|---|---|---|---|---|---|
| | Weight (g) | Weight Percentage | Weight (g) | Weight Percentage | Weight (g) | Weight Percentage |
| Active ingredient | 11.30 | 8.7% | 33.9 | 26.1% | 67.8 | 26.1% |
| Mannitol | 79.70 | 61.3% | 68.3 | 52.5% | 136.6 | 52.5% |
| Pregelatinized starch | 32.50 | 25% | 20.0 | 15.4% | 40.0 | 15.4% |
| Colloidal silicon dioxide | 3.25 | 2.5% | 3.9 | 3.0% | 7.8 | 3.0% |
| Magnesium stearate (internally added) | / | / | 1.95 | 1.5% | 3.9 | 1.5% |
| Magnesium stearate (externally added) | 3.25 | 2.5% | 1.95 | 1.5% | 3.9 | 1.5% |
| Total: | 130.0 | 100% | 130.0 | 100% | 260.0 | 100% |
| Vacant gelatin capsule | 3# | | 3# | | 1# | |
| Note: Examples 39-8 and 39-9 were carried out by referring to the preparation method described in Example 1, and the ratio of the fillers and the vacant gelatin capsule could be adjusted appropriately according to the desired strength and filling capacity. | | | | | | |

In Example 39-7, the formulation was prepared by a direct mixing process. The specific method was as follows: 1) material preparation: the active ingredient (API) compound B and various excipients were weighed according to the prescribed doses; 2) premixing 1: colloidal silicon dioxide and the active ingredient were sieved and mixed, and placed in a mixing vessel; 3) premixing 2: pregelatinized starch and mannitol were added to the above mixed powder, and the resulting mixture was shaken and mixed evenly; 4) total mixing: magnesium stearate was added to the mixed powder obtained in step 3) described above, and the resulting mixture was shaken and mixed evenly; and 5) capsule filling: capsule shells of a suitable model were selected for filling according to the filling capacity.

Table 22 Prescription (Combined Filler of Example 39

| Prescription | 39-10 | | 39-11 | | 39-12 | |
|---|---|---|---|---|---|---|
| | Weight (g) | Weight Percentage | Weight (g) | Weight Percentage | Weight (g) | Weight Percentage |
| Active ingredient | 2.908 | 29.08% | 5.814 | 32.31% | 11.628 | 32.31% |
| Microcrystalline cellulose | 4.195 | 41.95% | 7.164 | 39.79% | 14.328 | 39.79% |
| Lactose | 2.097 | 20.97% | 3.582 | 19.90% | 7.164 | 19.90% |
| Sodium starch glycolate | 0.300 | 3.00% | 0.54 | 3.00% | 1.080 | 3.00% |
| Colloidal silicon dioxide | 0.300 | 3.00% | 0.54 | 3.00% | 1.080 | 3.00% |
| Magnesium stearate | 0.200 | 2.00% | 0.36 | 2.00% | 0.720 | 2.00% |
| Total: | 10.0 | 100.0% | 18.0 | 100.0% | 36.0 | 100% |

(continued)

| Prescription | 39-10 | | 39-11 | | 39-12 | |
|---|---|---|---|---|---|---|
| | Weight (g) | Weight Percentage | Weight (g) | Weight Percentage | Weight (g) | Weight Percentage |
| Vacant gelatin capsule | 3# | | 3# | | 1# | |
| Note: with reference to the prescription and preparation method described in Example 15, the ratio of the fillers and the vacant gelatin capsule could be adjusted appropriately according to the desired strength and filling capacity. | | | | | | |

Table 23 Prescription (Single Filler of Example 39

| Prescription | 39-13 | | 39-14 | | 39-15 | |
|---|---|---|---|---|---|---|
| | Weight (g) | Weight Percentage | Weight (g) | Weight Percentage | Weight (g) | Weight Percentage |
| Active ingredient | 1.163 | 11.63% | 17.444 | 87.22% | 17.444 | 69.78% |
| Anhydrous dibasic calcium phosphate | 8.337 | 83.37% | 1.556 | 7.78% | 6.306 | 25.22% |
| Colloidal silicon dioxide | 0.300 | 3.00% | 0.600 | 3.00% | 0.75 | 3.00% |
| Magnesium stearate (internally added) | 0.100 | 1.00% | 0.200 | 1.00% | 0.25 | 1.00% |
| Magnesium stearate (externally added) | 0.100 | 1.00% | 0.200 | 1.00% | 0.25 | 1.00% |
| Total: | 10.0 | 100.0% | 20.0 | 100.0% | 25.00 | 100.0% |
| Vacant gelatin capsule | 3# | | 3# | | 1# | |
| Note: with reference to the prescription and preparation method described in Example 1, the proportion of the filler and the vacant gelatin capsule could be adjusted appropriately according to the desired strength and filling capacity. | | | | | | |

**Example 40: Tablets of compound B**

Table 24 Prescription (100 tablets) of Example 40

| Name of Material | | Weight (g) | Weight Percentage (%) |
|---|---|---|---|
| API | Compound B | 5.815 | 36.34 |
| Filler | Pregelatinized starch | 9.385 | 58.66 |
| Glidant | Colloidal silicon dioxide | 0.480 | 3.00 |
| Lubricant | Magnesium stearate (internally added) | 0.160 | 1.00 |
| Lubricant | Magnesium stearate (externally added) | 0.160 | 1.00 |
| Total weight of tablet core | | 16.000 | 100.00 |
| Coating powder | Colorcon gastric-soluble coating powder | 0.24 | 1.50% |
| Water | | 1.76 | / |

(continued)

| Name of Material | Weight (g) | Weight Percentage (%) |
|---|---|---|
| Total weight of tablet | | / |
| Note: 100 tablets were prepared for each of the prescriptions; the weight percentage of the coating powder was the weight of coating powder /total weight of tablet core; the model of the coating powder was Opadry 85G68918, and the specific composition was polyvinyl alcohol, titanium dioxide, talc, polyethylene glycol, and soyabean lecithin. | | |

The dry granulation process was adopted with the specific method as follows. 1) Material preparation: the active ingredient (API) compound A and the corresponding filler, glidant, and lubricant were weighed according to the prescribed doses; 2) premixing 1: the active ingredient, filler, glidant, and lubricant (internally added) were added in sequence into a mixing vessel for premixing; 3) deagglomeration: the resulting mixed powder was subjected to deagglomeration; 4) premixing 2: the mixed powder obtained after the deagglomeration treatment was added into a mixing vessel, and premixed again; 5) granulation; 6) total mixing: the granules obtained in step 5) were mixed with the lubricant (externally added); 7) tableting: the mixture was tableted by a tablet press; and 8) coating: the tablets prepared in step 5) were coated.

Table 25 Detection results of Example 40

| Prescription | Hardness | Content | Friability | Dissolution |
|---|---|---|---|---|
| | | | | 30 min |
| Example 40 | 8.8-11.31 kg | 100.1% | 0.35% | 100.9% |

**Results:** The prepared tablets had uniform content and no obvious abrasion on the edge, had hardness conformed to the requirements, and were dissolved rapidly. The prescription was qualified.

**Example 41: Granules of compound B**

Table 26 Prescription (100 sacks) of Example 41

| Name of Material | | Weight (g) | Weight Percentage (%) |
|---|---|---|---|
| API | Compound B | 17.445 g | 3.489 |
| Filler | Mannitol | 452.455 g | 90.491 |
| Binder | Copovidone VA64 | 30 g | 6.00 |
| fragrance | Lemon powder essence | 0.1 g | 0.02 |
| Total | | 500 g | 100.00 |

The specific method was as follows. 1) Material preparation: the active ingredient (API) compound B and the corresponding excipients were weighed according to the prescribed doses; 2) premixing 1: the active ingredient and various excipients were added in sequence into a mixing vessel and premixed; 3) soft material preparation: an appropriate amount of water was added to prepare the premix into suitable soft material; 4) granulation: the soft material was granulated; 5) drying: the granules were dried at 45°C; and 6) granule sizing.

Table 27 Detection results of Example 41

| Prescription | Appearance | Content | Solubility | Wight loss after Drying | Particle size |
|---|---|---|---|---|---|
| Example 41 | Yellow granules | 99.8% | Dissolved completely within 5 min, resulting in clear solution | Eligible | Eligible |

**Results:** The prepared granules had uniform content and good solubility performance, and the solution was clear. The prescription was qualified.

**Example 42: Oral solution of compound B**

Table 28 Prescription (100 mL) of Example 42

| Name of Material | | Weight (g) | Proportion % |
|---|---|---|---|
| API | Compound B | 1.7445 | 1.7445 |
| Sweetening agent | Neotame | 0.3 | 0.3 |
| Solvent | Purified water | added until the total weight reached 100 g | 98.2525 |
| pH modifier | NaOH | Appropriate amount | N/A |

The specific method was as follows. 1) Weighing: the active ingredient (API) compound B and the corresponding sweetening agent were weighed according to the prescribed doses; 2) solution preparation: the active ingredient and the sweetening agent were put in a beaker, then the solvent was added while stirring, and after the mixture was stirred evenly, sodium hydroxide was used to adjust the solution to pH 4 to pH 5; 3) filtering: the prepared solution was filtered and sterilized; 4) filling: the filling of the filtered solution was conducted according to the desired dose; and 5) capping.

Table 29 Detection Results of Example 42

| Prescription | Appearance | Content | pH |
|---|---|---|---|
| Example 42 | Clear solution | 99.7% | 4.55 |

**Results:** The prepared oral solution formulation had uniform content and the solution was clear. The prescription was qualified.

**Test Example 1: Solubility Test**

The salt obtained in Preparation Example 2 and the sample obtained in Preparation Example 1 were taken and subjected to the solubility test in the following media. The results were listed in the table below.

| **Preparation Example** | **Salt** | **Solubility (25°C, mg/mL)** | | | |
|---|---|---|---|---|---|
| | | **Aqueous medium** | **Aqueous medium pH=1.2** | **Aqueous medium pH=4.5** | **Aqueous medium pH=6.8** |
| Preparation Example 2 | Hydrochloride | 40.41 | 33.89 | 47.27 | 50.62 |
| Sample of Preparation Example 1 | | 0.05 | 12.60 | 0.57 | 0.04 |

**Test Example 2: Hygroscopicity Test**

The salt obtained in Preparation Example 2 and the sample obtained in Preparation Example 1 were taken and subjected to the hydroscopicity test at a temperature of 25±1°C and a relative humidity of 80%±2%, respectively. The results were listed in the table below.

| **Preparation Example** | **Salt** | **Result of Hygroscopicity Test (DVS, 80%RH)** | |
|---|---|---|---|
| | | **Weight gain due to hygroscopicity** | **Hygroscopicity** |
| Preparation Example 2 | Hydrochloride | 0.7% | Slight hygroscopicity |
| Sample of Preparation Example 1 | | 0.45% | Slight hygroscopicity |

**Test Example 3: Accelerated Stability Test**

The salt obtained in Preparation Example 2 was taken and placed at a temperature of 25±2°C under 60%±5%RH in an open environment for 10 days and at temperature of 40±2°C under 75%±5%RH in an open environment for 10 days respectively to perform the accelerated tests. The results were as follows.

| Preparation Example | Salt | Placed at 25±2°Cunder 60%±5%RH in an open environment for 10 days | | Placed at 40±2°C under 75%±5%RH in an open environment for 10 days | |
|---|---|---|---|---|---|
| | | Appearance | Crystal form | Appearance | Crystal form |
| Preparation Example 2 | Hydrochloride | Pale yellow or yellow solid | Same before and after placement | Pale yellow or yellow solid | Same before and after placement |

**Test Example 4: Long-term Stability Test**

An appropriate amount of the sample of the salt obtained in Preparation Example 2 was taken. A medicinal low-density polyethylene sack was used as an inner packing and a polyester/aluminum/polyethylene composite bag for pharmaceutical packaging was used as an outer packing. Sampling was carried out at the end of the $3^{rd}$, $6^{th}$, $9^{th}$, $12^{th}$, and $18^{th}$ month respectively after being stored at a temperature of 25±2°C and a relative humidity of 60%±5%. The appearances were compared, and then other investigation indices were tested. The results were compared with those tested in the $0^{th}$ month. The test results were shown in the table below.

| Time | Characteristics | Moisture (%) | Acidity | Related Substances (%) | Content (%) |
|---|---|---|---|---|---|
| 0 Month | Yellow crystalline powder | 13.9 | 3.1 | 0.33 | 100.9 |
| 3 Months | Yellow crystalline powder | 13.9 | 3.3 | 0.34 | 100.4 |
| 6 Months | Yellow crystalline powder | 14.3 | 3.3 | 0.34 | 100.8 |
| 9 Months | Yellow crystalline powder | 14.3 | 3.3 | 0.31 | 101.7 |
| 12 Months | Yellow crystalline powder | 13.9 | 3.3 | 0.34 | 99.6 |
| 18 Months | Yellow crystalline powder | 14.2 | 3.3 | 0.26 | 100.2 |

**Test Example 5: Bioactivity Test**

The samples of the salt obtained in Preparation Example 2 were tested according to the evaluation of the inhibitory activity of kinase described under Biological Assessment of the Patent Application No. WO 2011/147066. The test results suggested that the samples were capable of inhibiting the activities of kinase FLT3, EGFR, Abl, Fyn, Hck, Lck, Lyn, Ret, Yes, VEGFR2, ALK, BTK, c-KIT, c-SRC, FGFR1, KDR, MET, and PDGFRα. The test results of some kinases were listed in the table below.

| Kinase | $IC_{50}$ (nM) | Kinase | $IC_{50}$ (nM) |
|---|---|---|---|
| FLT3(h) | 26 | Lyn(h) | 7 |
| FLT3-ITD(h) | 3-10 | Ret(h) | 10 |
| EGFR(h) | 42 | Yes | 4 |
| Abl(h) | 25 | c-SRC(h) | 176 |
| Fyn(h) | 34 | FGFR1(h) | 247 |
| Hck(h) | 93 | KDR(h) | 323 |
| Lck(h) | 37 | | |

According to the in-vivo anti-tumor test described under Biological Assessment of the Patent Application No. WO 2011/147066, the samples of the salt obtained in Preparation Example 2 were tested (specifically for FLT3-ITD acute myeloid leukemia, non-small cell lung cancer with EGFR activating mutation, and Ph-positive chronic myeloid

leukemia, respectively). The test results suggested that, in the experiment conducted in MV4-11 (FLT3-ITD mutation) subcutaneous tumor model (the model was developed by referring to Example 4 of WO 2011/147066), the sample (orally administered once daily for 21 days) could completely inhibit the tumor growth at the administration dose of 5 mg/kg, and could cause complete tumor regression at administration doses of 10 mg/kg and 20 mg/kg. In the non-small cell lung cancer model (the model was developed by referring to Example 3 of WO 2011/147066), the sample could dose-dependently inhibit the growth of human non-small cell lung cancer HCC827, and caused tumor shrinkage (compared with the initial tumor) in all of the three dose groups of 7.5 mg/kg, 15 mg/kg, and 30 mg/kg (orally administered once daily for 30 days), among which in the 30 mg/kg group, the sample could cause almost complete tumor regression. In the experiment conducted in K562 (BCR-Abl gene rearrangement) subcutaneous tumor model (the model was developed in a similar way to that of the MV4-11 subcutaneous tumor model), the sample (orally administered once daily for 18 days) could effectively inhibit the tumor growth at the administration dose of 70 mg/kg, and the tumor inhibition rate reached 71.3%.

The corresponding studies on the hydrochloride and other salt forms of compound I had been described in the Patent Application No. PCT/CN2021/073285, and the content of this application was incorporated herein by reference in its entirety.

**Experimental Example 1: API-Excipient Compatibility Test**

The active ingredient (compound of Formula B) was mixed evenly with various excipients mentioned in the present application respectively, and put in 10-ml injection vials. The injection vials were stored for 14 days and 28 days or 30 days under the conditions of an illumination of 4500 lux±500 lux/peripheral humidity, a high temperature of 60°C±2°C/peripheral humidity, a high humidity of 90%±5%RH, and a temperature of 25°C±2°C respectively to detect the impurity content, so as to test the compatibility of the active ingredient with various excipients.

The results showed that after 14 days and 28 days or 30 days of accelerated compatibility test, the active ingredient exhibited good compatibility with various excipients mentioned in the present application without significant increase in the content of the maximum single impurity and total impurity, and the color of the mixed samples did not change significantly except for a slight discoloration of the calcium carbonate sample.

**Experimental Example 2: Accelerated Test**

The capsule samples of Examples 39-1, 39-2, 39-7, 39-8, and 39-9 and the tablet samples of Example 40 were taken, packaged according to the exemplary packaging conditions (packaged with solid medicinal composite hard sheets molded from aluminum foil and polyamide/aluminum/polyvinyl chloride by cold stamping), placed in a constant temperature and humidity chamber (40°C±2°C, relative humidity: 75%RH±5%RH), and subjected to the accelerated test for 6 months. Sampling was carried out at the end of the $0^{th}$, $1^{st}$, $2^{nd}$, $3^{rd}$, and $6^{th}$ month to investigate the major items. The experimental results of representative examples (Examples 39-2, 39-7, and 39-8, and Example 40) were listed in Table 30 to Table 33 (scaling up or proportional adjustments were made in other examples, not all data were presented and the results were similar).

Table 30 (Al-Al Packaging) Accelerated Test Results of Example 39-2 Batch

| Investigation Item | | 0 Day | 1 Month | 2 Months | 3 Months | 6 Months |
|---|---|---|---|---|---|---|
| Characteristic | | Capsules with the content of yellow powder or granules | Capsules with the content of yellow powder or granules | Capsules with the content of yellow powder or granules | Capsules with the content of yellow powder or granules | Capsules with the content of yellow powder or granules |
| Dissolution (%) at 30 min | | 102 | 102 | 101 | 101 | 100 |
| Related Substance (%) | Maximum single impurity | 0.009 | 0.01 | 0.02 | 0.02 | 0.02 |
| | Total impurity content | 0.02 | 0.02 | 0.02 | 0.02 | 0.04 |
| Microbial limit | | Eligible | - | - | - | Eligible |

(continued)

| Investigation Item | 0 Day | 1 Month | 2 Months | 3 Months | 6 Months |
|---|---|---|---|---|---|
| Content (%) | 101.5 | 100.1 | 99.4 | 100.1 | 99.5 |
| Crystal form | Unchanged | - | - | Unchanged | Unchanged |

Table 31 (Al-Al Packaging) Accelerated Test Results of Example 39-7 Batch

| Investigation Item | | 0 Day | 1 Month | 2 Months | 3 Months | 6 Months |
|---|---|---|---|---|---|---|
| Characteristic | | Capsules with the content of pale yellow powder | Capsules with the content of pale yellow powder | Capsules with the content of pale yellow powder | Capsules with the content of pale yellow powder | Capsules with the content of pale yellow powder |
| Dissolution (%) at 30 min | | 100 | 101 | 100 | 101 | 98 |
| Related Substance (%) | Maximum single impurity | 0.05 | 0.04 | 0.05 | 0.09 | 0.08 |
| | Total impurity content | 0.13 | 0.13 | 0.12 | 0.16 | 0.15 |
| Microbial limit | | Eligible | - | - | - | Eligible |
| Content (%) | | 100.1 | 100.5 | 101.8 | 99.7 | 99.1 |
| Crystal form | | Unchanged | - | - | Unchanged | Unchanged |

Table 32 (Al-Al Packaging) Accelerated Test Results of Example 39-8 Batch

| Investigation Item | | 0 Day | 1 Month | 2 Months | 3 Months | 6 Months |
|---|---|---|---|---|---|---|
| Characteristic | | Capsules with the content of yellow powder or granules | Capsules with the content of yellow powder or granules | Capsules with the content of yellow powder or granules | Capsules with the content of yellow powder or granules | Capsules with the content of yellow powder or granules |
| Dissolution (%) at 30 min | | 102 | 100 | 101 | 101 | 100 |
| Related Substance (%) | Maximum single impurity | 0.07 | 0.08 | 0.07 | 0.06 | 0.07 |
| | Total impurity content | 0.22 | 0.16 | 0.19 | 0.17 | 0.18 |
| Microbial limit | | Eligible | - | - | - | Eligible |
| Content (%) | | 100.2 | 99.8 | 100.8 | 100.6 | 101.9 |
| Crystal form | | Unchanged | - | - | Unchanged | Unchanged |

Table 33 (Al-Al Packaging) Accelerated Test Results of Example 40 Batch

| Investigation Item | | 0 Day | 3 Months | 6 Months |
|---|---|---|---|---|
| Characteristic | | Tablets | Tablets | Tablets |
| Dissolution (%) at 30 min | | 101.4 | 100.7 | 101.1 |
| Related Substance (%) | Maximum single impurity | 0.05 | 0.05 | 0.06 |
| | Total impurity content | 0.07 | 0.12 | 0.06 |
| Content (%) | | 100.1 | 101.8 | 101.0 |
| Crystal form | | Unchanged | Unchanged | Unchanged |

**Results:** The accelerated test results suggested that after the pharmaceutical compositions of the three examples packed in the packages were stored for 6 months under the conditions of a temperature of 40°C±2°C and a relative humidity of 75%RH±5%RH, the related substances did not increase or slightly increased, and the content remained basically unchanged; other investigation items at various time points were all eligible, and the crystal form of the active ingredient did not change. The capsule samples of Examples 39-1 and 39-9 also remained stable in the accelerated test, and showed no obvious change in terms of drug characteristic, related substance, drug content, dissolution, microbial limit, etc. The tablet samples of Example 40 also remained stable in the accelerated test, and showed no obvious change in terms of drug characteristic, related substance, drug content, microbial limit, etc.

**Experimental Example 3: Long-Term Test**

The capsule samples of Examples 39-1, 39-2, 39-7, 39-8, and 39-9 were taken, packaged according to the exemplary packaging conditions (packaged with solid medicinal composite hard sheets molded from aluminum foil and polyamide/aluminum/polyvinyl chloride by cold stamping), placed in a constant temperature and humidity chamber at a temperature of 30°C±2°C and a relative humidity of 65%RH±5%RH, and subjected to the accelerated test for 30 months. Sampling was carried out at the end of the $3^{rd}$, $6^{th}$, $9^{th}$, $12^{th}$, $18^{th}$, $24^{th}$, and $30^{th}$ month. The experimental results of the representative examples (Examples 39-7 and 39-8) were listed in Table 34 and Table 35 (scaling up or proportional adjustments were made in other examples, not all data were presented and the results were similar).

Table 34 (Al-Al Packaging) Long-Term Test Results of Example 39-7 Batch

| Investigation Item | | 0 Day | 3 Months | 6 Months | 9 Months | 12 Months | 18 Months | 24 Months | 30 Months |
|---|---|---|---|---|---|---|---|---|---|
| Characteristic | | Capsules with the content of pale yellow powder | Same as that on Day 0 | Same as that on Day 0 | Same as that on Day 0 | Same as that on Day 0 | Same as that on Day 0 | Same as that on Day 0 | Same as that on Day 0 |
| Dissolution (%) | | 100 | 100 | 98 | 100 | 100 | 99 | 100 | 100 |
| Related substance (%) | Maximum single impurity | 0.05 | 0.04 | 0.04 | 0.05 | 0.07 | 0.09 | 0.06 | 0.21 |
| | Total impurity content | 0.13 | 0.11 | 0.13 | 0.15 | 0.14 | 0.18 | 0.25 | 0.35 |
| Microbial limit | | Eligible | - | Eligible | - | Eligible | Eligible | Eligible | Eligible |
| Content (%) | | 100.1 | 100.3 | 100.3 | 100.6 | 99.4 | 99.8 | 100 | 99.2 |

Table 35 (Al-Al Packaging) Long-Term Test Results of Example 39-8 Batch

| Investigation Item | | 0 Day | 3 Months | 6 Months | 9 Months | 12 Months | 18 Months | 24 Months | 30 Months |
|---|---|---|---|---|---|---|---|---|---|
| Characteristic | | Capsules with the content of yellow powder or granules | Same as that on Day 0 | Same as that on Day 0 | Same as that on Day 0 | Same as that on Day 0 | Same as that on Day 0 | Same as that on Day 0 | Same as that on Day 0 |
| Dissolution (%) | | 102 | 101 | 99 | 101 | 98 | 101 | 99 | 98 |
| Related substance (%) | Maximum single impurity | 0.07 | 0.06 | 0.07 | 0.09 | 0.09 | 0.13 | 0.08 | 0.11 |
| | Total impurity content | 0.22 | 0.16 | 0.17 | 0.25 | 0.17 | 0.26 | 0.25 | 0.22 |
| Microbial limit | | Eligible | - | Eligible | - | Eligible | Eligible | Eligible | Eligible |
| Content (%) | | 100.2 | 100.9 | 102.6 | 103.6 | 99.6 | 99.6 | 100.7 | 100.2 |

**Results:** The long-term test results suggested that after the pharmaceutical compositions of the two examples packed in the packages were stored for 30 months under the conditions of a temperature of 30°C±2°C and a relative humidity of 65%RH±5%RH, the related substances did not increase or slightly increased, and the content remained basically unchanged; other investigation items at various time points were all eligible. The capsule samples of Examples 39-1, 39-2, and 39-9 also remained stable in the long-term test, and showed no obvious change in terms of drug characteristic, related substance, drug content, dissolution, microbial limit, etc.

**Claims**

1. A pharmaceutical composition, comprising a compound represented by Formula II as an active ingredient, and an excipient,

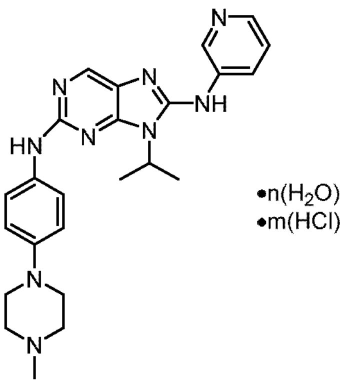

(II),

wherein m is 1 to 5; and n is 0 to 10.

2. The pharmaceutical composition according to claim 1, wherein the compound is a compound represented by Formula A:

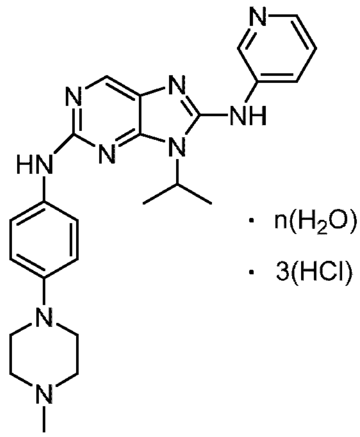

(A),

wherein n is 0 to 10.

3. The pharmaceutical composition according to claim 2, wherein the compound is a compound represented by Formula B:

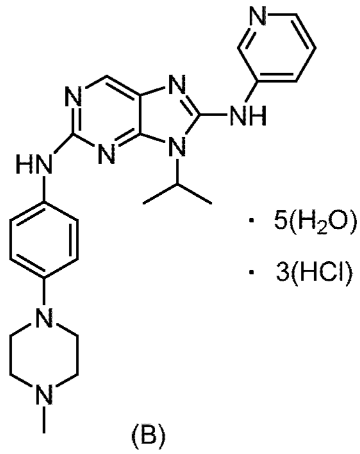

(B) ;

preferably, the compound represented by Formula B has characteristic peaks at 2θ angles of 8.5±0.2°, 11.8±0.2°, 19.6±0.2°, 25.2±0.2°, and 27.2±0.2° in an X-ray powder diffraction pattern obtained by using Cu-Kα radiation.

4. The pharmaceutical composition according to any one of claims 1 to 3, wherein components in the pharmaceutical composition and weight percentages thereof are as follows:

the active ingredient having a weight percentage of 0.5% to 90%, or 1% to 90%, or 0.5% to 85%, or 0.5% to 80%, or 1% to 80%, or 5% to 80%, or 5% to 75%, or 5% to 70%, or 5% to 65%, or 5% to 60%, or 5% to 55%, or 5% to 50%, or 5% to 45%, or 5% to 40%, or 10% to 70%, or 10% to 65%, or 10% to 60%, or 15% to 60%, or 20% to 60%, or 25% to 60%, or 25% to 50%, or 25% to 45%, or 25% to 40%; and
the remainder being an excipient;
preferably, components in the pharmaceutical composition and weight percentages thereof are as follows:
the active ingredient having a weight percentage of 5% to 90%, or 10% to 90%, or 10% to 80%, or 15% to 80%, or 20% to 70%, or 25% to 60%, or 25% to 50%, or 25% to 45%, or 25% to 40%; and the remainder being an excipient.

5. The pharmaceutical composition according to any one of claims 1 to 3, wherein the excipient comprises one or more selected from the group consisting of a filler, a binder, a disintegrant, a flavoring agent, a lubricating adjuvant, a bacteriostat, an antioxidant, a pH modifier, a surfactant, a perfume, a stabilizer, a thickener, a dispersing agent, a colouring agent, a solvent, and a coating material.

6. The pharmaceutical composition according to any one of claims 1 to 3, wherein the excipient comprises a filler, and optionally further comprises a disintegrant and a lubricating adjuvant; or the excipient comprises a filler and a lubricating adjuvant, and optionally further comprises a disintegrant and/or a binder.

7. The pharmaceutical composition according to claim 6, wherein the filler is one or more selected from the group consisting of starch, Confectioner's sugar, magnesium hydroxide, pregelatinized starch, lactose, microcrystalline cellulose, sugar alcohols, and inorganic calcium salts; preferably, the filler is one or more selected from the group consisting of starch, Confectioner's sugar, magnesium hydroxide, pregelatinized starch, lactose, microcrystalline cellulose, mannitol, sorbitol, xylitol, calcium phosphate, dibasic calcium phosphate, calcium sulfate, and calcium carbonate; further preferably, the filler is one of pregelatinized starch, anhydrous dibasic calcium phosphate, calcium carbonate, microcrystalline cellulose, and mannitol, or is a combination of pregelatinized starch and mannitol, or a combination of microcrystalline cellulose and lactose, or a combination of pregelatinized starch and anhydrous dibasic calcium phosphate, or a combination of pregelatinized starch and calcium carbonate, or a combination of pregelatinized starch and lactose; still further preferably, the filler is a combination of pregelatinized starch and mannitol, a combination of microcrystalline cellulose and lactose, a combination of pregelatinized starch and anhydrous dibasic calcium phosphate, or a combination of pregelatinized starch and calcium carbonate, wherein a weight ratio between two fillers is 1:10 to 10:1, or 1:7 to 7:1, or 1:6 to 6:1, or 1:5 to 5:1, or 1:4 to 4:1, or 1:3 to 3:1, or 1:2 to 2:1, or 1:1.

8. The pharmaceutical composition according to claim 6, wherein the pharmaceutical composition comprises a lubricating adjuvant, and the lubricating adjuvant is a lubricant or a combination of a lubricant and a glidant; preferably, the lubricant is one or more selected from the group consisting of stearic acid, magnesium stearate, calcium stearate, palmitic acid, glyceryl palmitostearate, sodium benzoate, sodium lauryl sulfate, hydrogenated vegetable oil, talc, silicon dioxide, zinc stearate, sodium stearyl fumarate, magnesium stearyl fumarate, magnesium lauryl sulfate, sodium dodecyl sulfate, magnesium dodecyl sulfate, and polyethylene glycol;
preferably, the glidant is one or more selected from the group consisting of colloidal silicon dioxide and aluminum hydroxide.

9. The pharmaceutical composition according to claim 6, wherein the pharmaceutical composition comprises no disintegrant; or the pharmaceutical composition comprises a disintegrant, and the disintegrant is one or more selected from the group consisting of dry starch, carboxymethyl cellulose, microcrystalline cellulose, powdered cellulose, methyl cellulose, polacrilin potassium, sodium alginate, sodium starch glycolate, polyvinylpyrrolidone, maltodextrin, magnesium aluminum silicate, corn starch, pregelatinized starch, crospovidone, low-substituted hydroxypropyl cellulose, carboxymethyl cellulose calcium, effervescent disintegrant, sodium starch glycolate, and croscarmellose sodium.

10. The pharmaceutical composition according to claim 6, wherein the pharmaceutical composition comprises no binder; or the pharmaceutical composition comprises a binder, and the binder is one or more selected from the group consisting of starch slurry, copovidone, Confectioner's sugar, syrup, polyvinylpyrrolidone, methyl cellulose, hydroxypropyl methyl cellulose, carboxymethyl cellulose sodium, mucilage, polyethylene glycol 4000, hydroxypropyl cellulose, ethyl cellulose, and dextrin.

11. The pharmaceutical composition according to claim 6, wherein the filler is one of pregelatinized starch, microcrystalline cellulose, and anhydrous dibasic calcium phosphate, or is a combination of pregelatinized starch and mannitol, or a combination of microcrystalline cellulose and lactose, or a combination of pregelatinized starch and anhydrous dibasic calcium phosphate, or a combination of pregelatinized starch and calcium carbonate; the lubricating adjuvant is a lubricant and/or a glidant, wherein the lubricant is magnesium stearate, and the glidant is colloidal silicon dioxide; the disintegrant is selected from the group consisting of sodium starch glycolate and croscarmellose sodium; and the binder is copovidone.

12. The pharmaceutical composition according to claim 6, wherein components of the pharmaceutical composition and weight percentages thereof are as follows:

the active ingredient having a weight percentage of 0.5% to 90%, or 1% to 90%, or 0.5% to 85%, or 5% to 80%, or 5% to 75%, or 5% to 70%, or 10% to 70%, or 5% to 65%, or 5% to 60%, or 5% to 55%, or 5% to 50%, or 5% to 45%, or 5% to 40%, or 10% to 65%, or 10% to 60%, or 15% to 60%, or 20% to 60%, or 25% to 60%, or 25% to 50%, or 25% to 45%, or 25% to 40%;
a filler having a weight percentage of 5% to 99%, or 10% to 95%, or 15% to 95%, or 15% to 90%, or 15% to

85%, or 15% to 80%, or 20% to 80%, or 25% to 90%, or 25% to 75%, or 30% to 70%, or 35% to 90%, or 35% to 70%, or 40% to 90%, or 40% to 70%, or 45% to 70%, or 50% to 90%, or 50% to 70%, or 55% to 70%;
a disintegrant having a weight percentage of 0% to 20%, or 0% to 15%, or 0% to 10%, or 0% to 8%, or 0% to 6%, or 0% to 5%, or 0% to 4%, or 0% to 3%, or 0.5% to 3%, or 1% to 3%, or 1.5% to 3%, or 2% to 3%;
a binder having a weight percentage of 0% to 10%, or 0% to 6%, or 0% to 5%, or 0% to 4%, or 0% to 3%, or 0% to 2%;
a lubricating adjuvant having a weight percentage of 0% to 18%, or 0.1% to 18%, or 0% to 15%, or 0.1% to 15%, or 0% to 10%, or 0.1% to 10%, or 0% to 8%, or 0% to 7%, or 0% to 6%, or 1% to 6%, or 0.5% to 6%, or 0% to 5%, or 1% to 5%, or 1.5% to 4.5%, or 2% to 4%, or 2% to 3.5%, or 2% to 3%, or 2.5% to 5%, or 3.5% to 5%, or 4% to 5%;
an additional excipient having a weight percentage of 0% to 25%, or 0% to 20%, or 0% to 15%, or 0% to 10%, or 0% to 5%; and
the sum of the weight percentage of each component above is 100%;
preferably, components of the pharmaceutical composition and weight percentages thereof are as follows:

the active ingredient having a weight percentage of 5% to 90%, or 10% to 90%, or 10% to 80%, or 15% to 80%, or 20% to 70%, or 25% to 60%, or 25% to 50%, or 25% to 45%, or 25% to 40%;
a filler having a weight percentage of 5% to 90%, or 15% to 90%, or 15% to 85%, or 25% to 85%, or 25% to 80%, or 35% to 75%, or 35% to 70%, or 50% to 70%, or 55% to 70%;
a disintegrant having a weight percentage of 0% to 5%, or 0% to 4%, or 0.5% to 4%;
a binder having a weight percentage of 0% to 5%, or 0% to 4%, or 0% to 3%;
a lubricating adjuvant having a weight percentage of 2% to 7%, or 2% to 6%, or 2.5% to 6%, or 3% to 5%, or 3% to 6%, or 3.5 to 6%, or 4% to 6%, or 4% to 5%, or 5% to 6%;
an additional excipient having a weight percentage of 0% to 10%, or 0% to 5%; and
the sum of the weight percentage of each component above is 100%;
further preferably, the lubricating adjuvant is a lubricant; or the lubricating adjuvant is a combination of a lubricant and a glidant, and a weight ratio of the glidant to the lubricant is 1:6 to 6:1; or 1:5 to 5:1; or 1:4 to 4:1; or 1:3 to 3:1; or 1:2 to 2:1; or 1:1, 1:2, 1:1.5, 2:1, 2:3, 3:1, 3:2, 4:3 or 5:3.

**13.** The pharmaceutical composition according to claim 6, wherein components of the pharmaceutical composition and weight percentages thereof are as follows:

the active ingredient having a weight percentage of 0.5% to 90%, or 1% to 90%, or 0.5% to 85%, or 5% to 80%, or 5% to 75%, or 5% to 70%, or 10% to 70%, or 5% to 65%, or 5% to 60%, or 5% to 55%, or 5% to 50%, or 5% to 45%, or 5% to 40%, or 10% to 65%, or 10% to 60%, or 15% to 60%, or 20% to 60%, or 25% to 60%, or 25% to 50%, or 25% to 45%, or 25% to 40%;
a filler having a weight percentage of 5% to 99%, or 10% to 95%, or 15% to 95%, or 15% to 90%, or 15% to 85%, or 15% to 80%, or 20% to 80%, or 25% to 90%, or 25% to 75%, or 30% to 70%, or 35% to 90%, or 35% to 70%, or 40% to 90%, or 40% to 70%, or 45% to 70%, or 50% to 90%, or 50% to 70%, or 55% to 70%;
a disintegrant having a weight percentage of 0% to 20%, or 0% to 15%, or 0% to 10%, or 0% to 8%, or 0% to 6%, or 0% to 5%, or 0% to 4%, or 0% to 3%, or 0.5% to .00%, or 1% to 3%, or 1.5% to 3%, or 2% to 3%;
a binder having a weight percentage of 0% to 10%, or 0% to 6%, or 0% to 5%, or 0% to 4%, or 0% to 3%, or 0% to 2%;
a lubricant having a weight percentage of 0% to 10%, or 0% to 8%, or 0.1% to 8%, or 0% to 6%, or 0% to 5%, or 0.1% to 5%, or 0% to 4%, or 0.1% to 4%, or 0% to 3%, or 1% to 5%, or 2% to 4%, or 2% to 3%, or 1% to 4%, or 1% to 3.5%, or 0.5% to 3%, or 1% to 3%, or 1% to 2.5%, or 1% to 2%, or 2% to 2.5%;
a glidant having a weight percentage of 0% to 10%, or 0.5% to 10%, or 0% to 8%, or 0.5% to 8 %, or 0% to 6%, or 0% to 5%, or 0.5% to 5%, or 1% to 5%, or 0% to 4%, or 1% to 4%, or 0% to 3.5%, or 0% to 3%, or 1% to 3%, or 1% to 2.5%, or 1% to 2%, or 1.5% to 3%, or 2% to 3%, or 3%;
an additional excipient having a weight percentage of 0% to 25%, or 0% to 20%, or 0% to 15%, or 0% to 10%, or 0% to 5%; and
the sum of the weight percentage of each component above is 100%;
preferably, components of the pharmaceutical composition and weight percentages thereof are as follows:

the active ingredient having a weight percentage of 5% to 90%, or 10% to 90%, or 10% to 80%, or 15% to 80%, or 20% to 70%, or 25% to 60%, or 25% to 50%, or 25% to 45%, or 25% to 40%;
a filler having a weight percentage of 5% to 90%, or 15% to 90%, or 15% to 85%, or 25% to 85%, or 25% to 80%, or 35% to 75%, or 35% to 70%, or 50% to 70%, or 55% to 70%;

a disintegrant having a weight percentage of 0% to 5%, or 0% to 4%, or 0.5% to 4%;
a binder having a weight percentage of 0% to 5%, or 0% to 4%, or 0% to 3%;
a lubricant having a weight percentage of 1% to 5%, or 1% to 4%, or 2% to 4%, or 2% to 3%;
a glidant having a weight percentage of 0% to 5%, or 0% to 4%, or 1% to 4%, or 1% to 3%, or 0% to 3%, or 2% to 4%, or 3% to 4%, or 2% to 3%;
an additional excipient having a weight percentage of 0% to 10%, or 0% to 5%; and
the sum of the weight percentage of each component above is 100%.

**14.** The pharmaceutical composition according to claim 6, wherein components of the pharmaceutical composition and weight percentages thereof are as follows:

0.5% to 90% of the active ingredient, 5% to 99% of filler, 0% to 20% of disintegrant, 0% to 10% of binder, 0.1% to 15% of lubricating adjuvant, and 0% to 25% of additional excipient; or
5% to 80% of the active ingredient, 10% to 95% of filler, 0% to 15% of disintegrant, 0% to 6% of binder, 0.50% to 10% of lubricating adjuvant, and 0% to 15% of additional excipient; or
5% to 80% of the active ingredient, 15% to 85% of filler, 0% to 5% of disintegrant, 0% to 4% of binder, 0% to 7% of lubricating adjuvant, and 0% to 10% of additional excipient; or
5% to 80% of the active ingredient, 15% to 80% of filler, 0% to 4% of disintegrant, 0% to 3% of binder, 1% to 6% of lubricating adjuvant, and 0% to 5% of additional excipient; and
the sum of the weight percentage of each component above is 100%;
preferably, components of the pharmaceutical composition and weight percentages thereof are as follows:

5% to 90% of the active ingredient, 5% to 90% of filler, 0% to 5% of disintegrant, 0% to 5% of binder, 1% to 7% of lubricating adjuvant, and 0% to 5% of additional excipient; or
10% to 90% of the active ingredient, 15% to 90% of filler, 0% to 5% of disintegrant, 0% to 4% of binder, 1% to 6% of lubricating adjuvant, and 0% to 5% of additional excipient; and
the sum of the weight percentage of each component above is 100%.

**15.** The pharmaceutical composition according to claim 6, wherein components of the pharmaceutical composition and weight percentages thereof are as follows:

0.50% to 90% of the active ingredient, 5% to 99% of filler, 0% to 20% of disintegrant, 0% to 10% of binder, 0% to 10% of lubricant, 0% to 10% of glidant, and 0% to 25% of additional excipient; or
5% to 80% of the active ingredient, 10% to 95% of filler, 0% to 10% of disintegrant, 0% to 6% of binder, 0% to 5% of lubricant, 0% to 5% of glidant, and 0% to 25% of additional excipient; or
5% to 80% of the active ingredient, 15% to 85% of filler, 0% to 6% of disintegrant, 0% to 5% of binder, 1% to 4% of lubricant, 0% to 4% of glidant, and 0% to 15% of additional excipient; or
5% to 80% of the active ingredient, 15% to 80% of filler, 0% to 5% of disintegrant, 0% to 4% of binder, 1% to 3.50% of lubricant, 0% to 3.50% of glidant, and 0% to 10% of additional excipient; or
5% to 80% of the active ingredient, 15% to 80% of filler, 0% to 4% of disintegrant, 0% to 3% of binder, 1% to 3% of lubricant, 0% to 3% of glidant, and 0% to 5% of additional excipient; and
the sum of the weight percentage of each component above is 100%;
preferably, components of the pharmaceutical composition and weight percentages thereof are as follows:

5% to 90% of the active ingredient, 5% to 90% of filler, 0% to 5% of disintegrant, 0% to 5% of binder, 1% to 5% of lubricant, 0% to 5% of glidant, and 0% to 5% of additional excipient; or
5% to 90% of the active ingredient, 5% to 90% of filler, 0% to 5% of disintegrant, 0% to 5% of binder, 1% to 4% of lubricant, 0% to 4% of glidant, and 0% to 5% of additional excipient; or
10% to 90% of the active ingredient, 15% to 90% of filler, 0% to 5% of disintegrant, 0% to 4% of binder, 1% to 3% of lubricant, 0% to 3% of glidant, and 0% to 5% of additional excipient; and
the sum of the weight percentage of each component above is 100%.

**16.** The pharmaceutical composition according to any one of claims 12 to 15, wherein the filler is one of pregelatinized starch, microcrystalline cellulose, anhydrous dibasic calcium phosphate, and calcium carbonate, or is a combination of pregelatinized starch and mannitol, or a combination of microcrystalline cellulose and lactose, or a combination of pregelatinized starch and anhydrous dibasic calcium phosphate, or a combination of pregelatinized starch and calcium carbonate; the lubricating adjuvant is a lubricant or a combination of a lubricant and a glidant; the lubricant is one or more selected from the group consisting of magnesium stearate and sodium stearyl fumarate; the glidant

is colloidal silicon dioxide; the disintegrant is selected from the group consisting of sodium starch glycolate and croscarmellose sodium; and the binder is copovidone.

**17.** The pharmaceutical composition according to claim 6, wherein the pharmaceutical composition is prepared into an oral solid formulation; preferably, the oral solid formulation is one or more selected from the group consisting of a capsule, a tablet, a powder, and a fine granule.

**18.** The pharmaceutical composition according to claim 6, comprising 0.001 to 1000 mg of the active ingredient.

**19.** Use of the pharmaceutical composition according to any one of claims 1 to 18 for inhibiting the activity of one or more kinases selected from the group consisting of FLT3, EGFR, Abl, Fyn, Hck, Lck, Lyn, Ret, Yes, VEGFR2, ALK, BTK, c-KIT, c-SRC, FGFR1, KDR, MET, and PDGFRα.

**20.** Use of the pharmaceutical composition according to any one of claims 1 to 18 in the preparation of a medicament, wherein
preferably, the medicament is used for treating a protein kinase-mediated disease, and the protein kinase is one or more kinases selected from the group consisting of FLT3, EGFR, Abl, Fyn, Hck, Lck, Lyn, Ret, Yes, VEGFR2, ALK, BTK, c-KIT, c-SRC, FGFR1, KDR, MET, and PDGFRα; further preferably, the protein kinase-mediated disease is a tumor disease, and the tumor disease is preferably a solid tumor or a hematological tumor, further preferably leukemia or lung cancer, more preferably acute myeloid leukemia or non-small cell lung cancer, still further preferably FLT3-mutation-positive acute myeloid leukemia or non-small cell lung cancer with EGFR activating mutation.

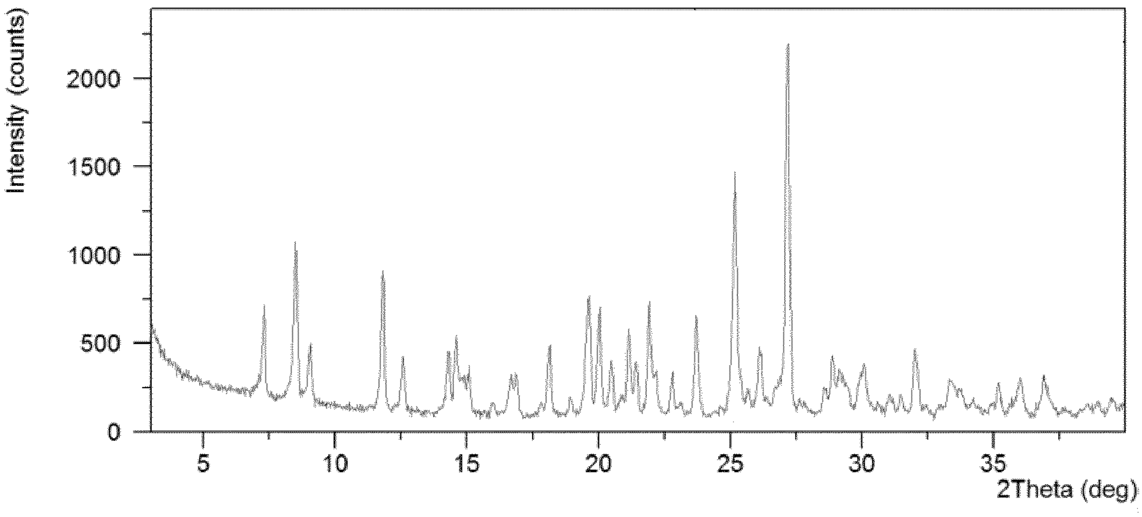
Intensity (counts)
2000
1500
1000
500
0
5
10
15
20
25
30
35
2Theta (deg)

FIG. 1

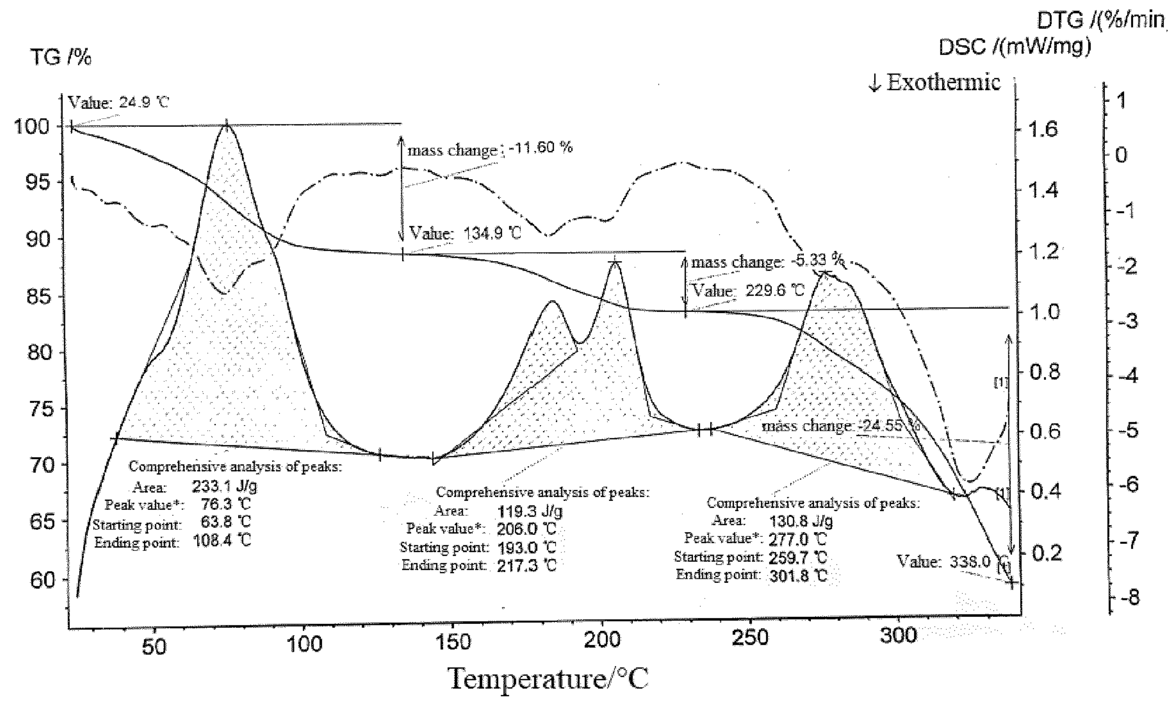
TG /%
DTG /(%/min)
DSC /(mW/mg)
↓ Exothermic
Value: 24.9 ℃
mass change: -11.60 %
Value: 134.9 ℃
mass change: -5.33 %
Value: 229.6 ℃
mass change: -24.55 %
Value: 338.0 ℃
Comprehensive analysis of peaks:
Area: 233.1 J/g
Peak value*: 76.3 ℃
Starting point: 63.8 ℃
Ending point: 108.4 ℃
Comprehensive analysis of peaks:
Area: 119.3 J/g
Peak value*: 206.0 ℃
Starting point: 193.0 ℃
Ending point: 217.3 ℃
Comprehensive analysis of peaks:
Area: 130.8 J/g
Peak value*: 277.0 ℃
Starting point: 259.7 ℃
Ending point: 301.8 ℃
Temperature/°C

FIG 2

**INTERNATIONAL SEARCH REPORT**

International application No.
**PCT/CN2021/143219**

**A. CLASSIFICATION OF SUBJECT MATTER**

A61K 31/5377(2006.01)i; A61K 31/52(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS; CNTXT; SIPOABS; DWPI; WOTXT; USTXT; EPTXT; STNext; 百度学术, BAIDU SCHOLAR: SKLB1028, 1350544-93-2, 芳胺基嘌呤, 盐酸盐, 蛋白激酶抑制剂, 晶体, arylaminopurine, salt, protein kinase inhibitor, 石药集团中奇制药技术（石家庄）有限公司, 刘翠艳, 白晶, 文师龙, 刘娜, 谷聪, 纪德华

**C. DOCUMENTS CONSIDERED TO BE RELEVANT**

| Category* | Citation of document, with indication, where appropriate, of the relevant passages | Relevant to claim No. |
|---|---|---|
| PX | WO 2021089038 A1 (CSPC ZHONGQI PHARMACEUTICAL TECHNOLOGY (SHIJIAZHUANG) CO., LTD.) 14 May 2021 (2021-05-14)<br>claims 1-24 | 1-20 |
| PX | WO 2021147996 A1 (CSPC ZHONGQI PHARMACEUTICAL TECHNOLOGY (SHIJIAZHUANG) CO., LTD.) 29 July 2021 (2021-07-29)<br>claims 1-12 | 1-20 |
| X | WO 2011147066 A1 (SICHUAN UNIVERSITY) 01 December 2011 (2011-12-01)<br>description, pp. 37, 79-80, and 97-108 | 1-20 |
| X | CAO, Z. X. et al. "SKLB1028, a novel oral multikinase inhibitor of EGFR, FLT3 and Abl, displays exceptional activity in models of FLT3-driven AML and considerable potency in models of CML harboring Abl mutants."<br>*Leukemia.*, Vol. 26, 03 April 2012 (2012-04-03),<br>pp. 1892-1895 | 1-20 |
| A | CN 101142215 A (SIGNAL PHARMACEUTICALS LLC et al.) 12 March 2008 (2008-03-12)<br>claims 1-36 | 1-20 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 March 2022** | **29 March 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/143219**

**C. DOCUMENTS CONSIDERED TO BE RELEVANT**

| Category* | Citation of document, with indication, where appropriate, of the relevant passages | Relevant to claim No. |
|---|---|---|
| A | WO 2019113345 A1 (MOUSSES SPYRO) 13 June 2019 (2019-06-13) claims 1-29 | 1-20 |

Form PCT/ISA/210 (second sheet) (January 2015)

**INTERNATIONAL SEARCH REPORT**

International application No.
**PCT/CN2021/143219**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19**
because they relate to subject matter not required to be searched by this Authority, namely:

    [1] Claim 19 relates to a use of a pharmaceutical composition for inhibiting multiple kinase activities, and said claim does not comply with PCT Rule 39.1(iv). A search is conducted on the basis that claim 19 relates to a use of a pharmaceutical composition in the preparation of multiple kinase inhibitors.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/143219**

| Patent document cited in search report | Publication date (day/month/year) | Patent family member(s) | Publication date (day/month/year) |
|---|---|---|---|
| WO 2021089038 A1 | 14 May 2021 | None | |
| WO 2021147996 A1 | 29 July 2021 | None | |
| WO 2011147066 A1 | 01 December 2011 | CN 103003278 A | 27 March 2013 |
| | | CN 103003278 B | 30 March 2016 |
| | | CN 105832740 A | 10 August 2016 |
| | | US 2013203986 A1 | 08 August 2013 |
| | | US 9096601 B2 | 04 August 2015 |
| | | KR 20130109984 A | 08 October 2013 |
| | | KR 101839915 B1 | 04 May 2018 |
| | | EP 2578584 A1 | 10 April 2013 |
| | | EP 2578584 A4 | 30 October 2013 |
| | | EP 2578584 B1 | 12 August 2020 |
| | | CN 102260263 A | 30 November 2011 |
| | | ES 2814257 T3 | 26 March 2021 |
| | | JP 2013528164 A | 08 July 2013 |
| | | JP 5662564 B2 | 04 February 2015 |
| CN 101142215 A | 12 March 2008 | ZA 200706662 A | 29 April 2009 |
| | | ZA 200706662 B | 29 April 2009 |
| WO 2019113345 A1 | 13 June 2019 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202011642452
- CN 202110829596
- WO 2011147066 A1
- CN 2021073285 W
- CN 2020127449
- WO 2011147066 A